# EUROPEAN PATENT SPECIFICATION

(11) **EP 2 340 042 B1**
(45) Date of publication and mention of the grant of the patent: **23.07.2014**
(21) Application number: 09783138.2
(22) Date of filing: 17.09.2009
(51) Int. Cl.: A61K 45/06, A61K 31/4425, A61K 33/24, C12Q 1/34, A61P 35/00

(54) **METHODS AND COMPOSITIONS FOR THE TREATMENT OF CANCER**
VERFAHREN UND ZUSAMMENSETZUNGEN ZUR BEHANDLUNG VON KREBS
PROCÉDÉ ET COMPOSITIONS DE TRAITEMENT DU CANCER

(30) Priority: 17.09.2008 EP 08380270
(43) Date of publication of application: 06.07.2011
(73) Proprietor: Traslational Cancer Drugs Pharma, S.L., 47001 Valladolid (ES)
(72) Inventor: RAMÍREZ DE MOLINA, Ana, E-28224 Pozuelo de Alarcón - Madrid (ES); GARCÍA OROZ, Lourdes, E-28012 Madrid (ES); LACAL SANJUÁN, Juan Carlos, E-28250 Torrelodones - Madrid (ES)
(74) Representative: ABG Patentes, S.L.
(86) International application number: PCT/EP2009/062078
(87) International publication number: WO 2010/031825

(56) References cited:
- EP-A- 1 889 920
- MORI NORIKO ET AL: "Choline kinase down-regulation increases the effect of 5-fluorouracil in breast cancer cells" CANCER RESEARCH, vol. 67, no. 23, December 2007 (2007-12), pages 11284-11290, XP002517954 ISSN: 0008-5472
- RODRIGUEZ-GONZALEZ AGUSTIN ET AL: "Choline kinase inhibition induces the increase in ceramides resulting in a highly specific and selective cytotoxic antitumoral strategy as a potential mechanism of action" ONCOGENE, vol. 23, no. 50, 28 October 2004 (2004-10-28), pages 8247-8259, XP002517955 ISSN: 0950-9232
- JANARDHAN S ET AL: "Choline kinase: An important target for cancer" CURRENT MEDICINAL CHEMISTRY 200604 NL, vol. 13, no. 10, April 2006 (2006-04), pages 1169-1186, XP002517956 ISSN: 0929-8673
- LIU XIANG ET AL: "Acid ceramidase inhibition: a novel target for cancer therapy" FRONTIERS IN BIOSCIENCE, FRONTIERS IN BIOSCIENCE, ALBERTSON, NY, US, vol. 13, 1 January 2008 (2008-01-01), pages 2293-2298, XP009113101 ISSN: 1093-9946
- ZEIDAN Y H ET AL: "Molecular targeting of acid ceramidase: Implications to cancer therapy" CURRENT DRUG TARGETS, BENTHAM SCIENCE PUBLISHER, US, vol. 9, no. 8, 1 January 2008 (2008-01-01) , pages 653-661, XP009113322 ISSN: 1389-4501
- MORALES A ET AL: "Pharmacological inhibition or small interfering RNA targeting acid ceramidase sensitizes hepatoma cells to chemotherapy and reduces tumor growth in vivo" ONCOGENE 20070208 GB, vol. 26, no. 6, 8 February 2007 (2007-02-08), pages 905-916, XP002517957 ISSN: 0950-9232 1476-5594
- ELOJEIMY SAEED ET AL: "Role of acid ceramidase in resistance to FasL: therapeutic approaches based on acid ceramidase inhibitors and FasL gene therapy." MOLECULAR THERAPY : THE JOURNAL OF THE AMERICAN SOCIETY OF GENE THERAPY JUL 2007, vol. 15, no. 7, July 2007 (2007-07), pages 1259-1263, XP002517958 ISSN: 1525-0024
- SAAD A F ET AL: "The functional effects of acid ceramidase overexpression in prostate cancer progression and resistance to chemotherapy" CANCER BIOLOGY AND THERAPY 200709 US, vol. 6, no. 9, September 2007 (2007-09), pages 1455-1460, XP002517959 ISSN: 1538-4047 1555-8576
- K. Glunde: "RNA Interference-Mediated Choline Kinase Suppression in Breast Cancer Cells Induces Differentiation and Reduces Proliferation", Cancer research, vol. 65, no. 23, 1 December 2005 (2005-12-01), pages 11034-11043, XP055067870, ISSN: 0008-5472, DOI: 10.1158/0008-5472.CAN-05-1807
- RAMIREZ DE MOLINA A ET AL: "Choline kinase as a link connecting phospholipid metabolism and cell cycle regulation: Implications in cancer therapy", INTERNATIONAL JOURNAL OF BIOCHEMISTRY AND CELL BIOLOGY, PERGAMON, GB, vol. 40, no. 9, 1 January 2008 (2008-01-01), pages 1753-1763, XP022734731, ISSN: 1357-2725, DOI: 10.1016/J.BIOCEL.2008.01.013 [retrieved on 2008-01-19]

## Description

### FIELD OF THE INVENTION

The invention relates to the field of therapeutics and, more in particular, to the field of cancer therapeutics using compositions containing several therapeutic compounds showing improved activity with respect to the compounds used individually.

### BACKGROUND OF THE INVENTION

Choline kinase is the first enzyme of the Kennedy pathway or the phospatidylcholine (PC) synthesis pathway. It acts by phosphorylating choline to phosphorylcholine (PCho) using adenosine 5'-triphosphate (ATP) as a phosphate group donor. Ras genes form a family of the so-called oncogenes which have been widely studied since they are activated in 25-30% of all human tumors and in several of them in 90%. Ras proteins have an important role in the transmission of intracellular signals due to their involvement in the regulation of cell proliferation, terminal differentiation and senescence. The transformation mediated by different oncogenes, among which the ras oncogenes stand out, induces high choline kinase activity levels, resulting in an abnormal increase in the intracellular levels of its product, PCho. Complementary findings support the role of ChoK in the generation of human tumors. For instance, nuclear magnetic resonance (NMR) techniques have shown the presence of high PCho levels in several human tumor tissues including breast, prostate, brain and ovarian tumors with respect to normal tissues. It is common knowledge that ras is one of the most deeply studied oncogenes in human carcinogenesis and that ChoK inhibition has been shown to be a new and effective antitumor strategy in cells transformed by oncogenes. These first observations were later extrapolated in vivo in nude mice.

In view of this data, the design of compounds directly affecting choline kinase activity or the enzyme activated by phosphorylcholine in an individual or combined manner would allow the development of effective antitumor therapies.

In this sense, the research on ChoK inhibitors has identified Hemicholinium-3 (HC-3) as a relatively potent and selective blocking agent (Cuadrado A., et al., 1993, Oncogene 8: 2959-2968, Jiménez B., et al., 1995, J. Cell Biochem. 57:141-149; Hernández-Alcoceba, R. et al., 1997, Oncogene, 15:2289-2301). This choline homologue with a biphenyl structure has been used for designing new antitumor drugs. Nevertheless, due to the fact that HC-3 is a potent respiratory paralyzing agent, it is not a good candidate for its use in clinical practice. Some derivatives having improved inhibitory activity of the ChoK and reduced toxic effects have been synthesized based on the structure of HC-3 by introducing structural modifications in this compound.

Bisquaternized symmetric compounds derived from pyridinium have also been found to inhibit PCho production in whole cells (WO98/05644). However, these derivatives have high toxicity levels limiting their extended therapeutic application.

Resistance to chemotherapy ('drug resistance') is a fundamental problem that limits the effectiveness of many chemotherapies currently used in cancer treatment. Drug resistance can occur due to a variety of mechanisms, such as increased drug inactivation, decreased drug accumulation, drug efflux from cancer cells, enhanced repair of chemotherapy-induced damage, activation of pro-survival pathways and inactivation of cell death pathways (Hersey P. et al., 2008, Adv Exp Med Biol. 2008;615:105-26). Drug resistance can be inherent to the tumour cells before the initiation of an antitumor treatment. In addition, specific drug resistance mechanisms can be activated after exposure of tumour cells to a particular treatment. The identification of those molecular entities responsible for either intrinsic or acquired resistance, may provide valuable information on the potential molecular targets that may be useful to overcome this resistance. Therefore, designing strategies aimed at interfering with the molecular components that confer drug resistance for a defined tumour-specific treatment constitutes an important step forward to increase the efficiency of cancer treatments.

Mori et al (Cancer Res., 2007, 67:11284-11290) have described that the combined use of a ChoK inhibitor (choline kinase-specific siRNA) and 5-fluorouracil results in a most likely additive effect on the reduction of cell proliferation/viability of breast cancer cells. However, this treatment relies on the use of a siRNA which is not transported to the target cells in vivo in an efficient manner. In addition the siRNA designed to this purpose recognizes both ChoKα and ChoKβ species (Mori et al., Cancer Res., 2007, 67:11284-11290) but only ChoKα and not ChoKβ is a molecular target in oncology (International patent application WO2006108905 ), questioning the potential therapeutic use of this strategy.

Pharmaceutical compositions comprising a therapeutically effective amount of one or several agents inhibiting the expression and/or activity of the choline kinase alpha protein, together with any other active ingredient that does not inhibit the function of the choline kinase alpha protein have been described (EP1889920A).

Nevertheless, there is a great need to develop compounds that provide a high inhibitory activity of the ChoK enzyme for the purpose of allowing their use for the treatment of tumors, while at the same time they considerably reduce their toxicity against compounds of the state of the art.

### SUMMARY OF THE INVENTION

In a first aspect, the invention relates to a composition comprising, separately or together, a first component comprising one or more choline kinase inhibitors specific for the choline kinase alpha isoform and a second component selected from of one or more acid ceramidase inhibitors and one or more chemotherapeutic agents, wherein the chemotherapy agent is selected from the group consisting of a DNA-alkylating agent, an antimetabolite, a mitotic inhibitor, an anthracycline, a topoisomerase I inhibitor, a topoisomerase II inhibitor, cetuximab, gefitinib and imatinib.

In a second aspect, the invention relates to a pharmaceutical composition comprising a composition according to the invention with a pharmaceutically acceptable carrier or excipient and to the uses thereof in medicine and, in particular, for the treatment of cancer.

In another aspect, it is also disclosed the use of an inhibitor of acid ceramidase, a chemotherapeutic agent or a ligand for a death receptor to increase the sensitivity of a tumor cell to a choline kinase inhibitor.

In yet another aspect, it is also dislosed a method for the identification of cancer patients resistant to the therapy with ChoK inhibitors comprising determining the levels of acid ceramidase in a sample from said patient wherein the patient is identified as being resistant to ChoK inhibitors when the acid ceramidase levels in said sample are higher than a reference sample.

In another aspect, it is also disclosed a method for selecting a personalised therapy for a patient suffering from cancer comprising determining the levels of acid ceramidase in a sample from said patient wherein if the expression levels of acid ceramidase in said sample are higher than in the reference sample, the patient is candidate for being treated with a combination of a ChoK inhibitor and an acid ceramidase inhibitor.

In yet another aspect, it is also disclosed a method for the identification of compounds capable of increasing the therapeutic effect of a ChoK inhibitor for the treatment of cancer comprising the steps of
(i) contacting a tumor cell showing resistance to ChoK inhibitors with a candidate compound and
(ii) determining in said cell the levels of acid ceramidase
wherein if the levels of acid ceramidase in the cell after having being treated with a candidate compound are lower than before the treatment, then the candidate compound is considered to be able to increase the effect of ChoK inhibitors for the treatment of cancer.

### BRIEF DESCRIPTION OF THE FIGURES

**Figure 1****.** Response to MN58b treatment in primary cultures of tumours of patients with NSCLC.
**Figure 2****.** Validation of the results of the microarray analysis by RT-qPCR.
**Figure 3****.** Proposed model for a mechanism of resistance to ChoK inhibition in NSCLC.
**Figure 4****.** Levels of ASAH1 and ChoK in different cell lines derived from human lung cancer determined by RT-qPCR.
**Figure 5****.** ASAH1 gene expression determined by qRT-PCR and acid ceramidase protein expression determined by Western blot analysis in H460 ChoK inhibitors-resistant cells.
**Figure 6****.** Synergistic effect of the combined sequential therapy of cisplatin/ChoK inhibitors.

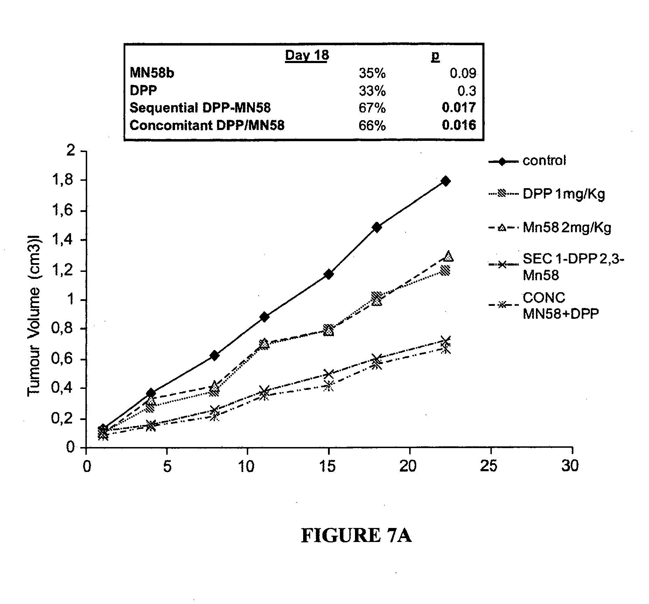

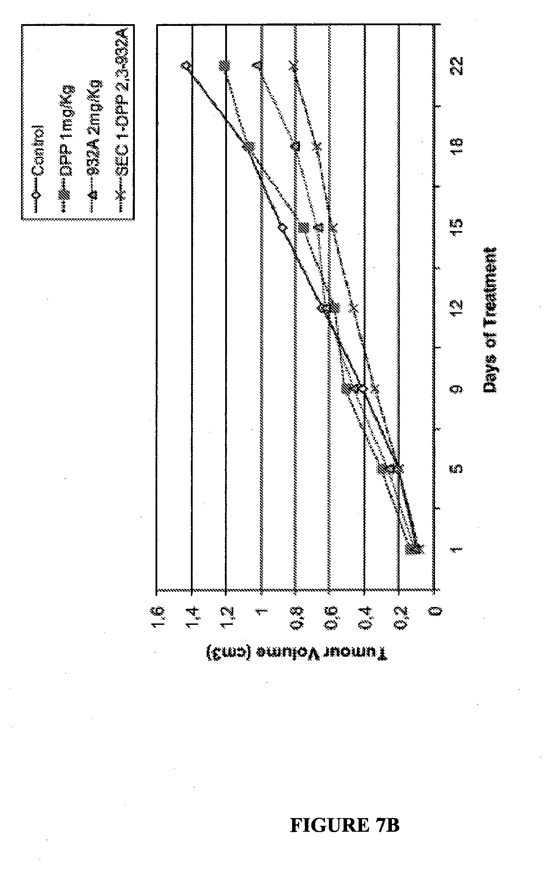

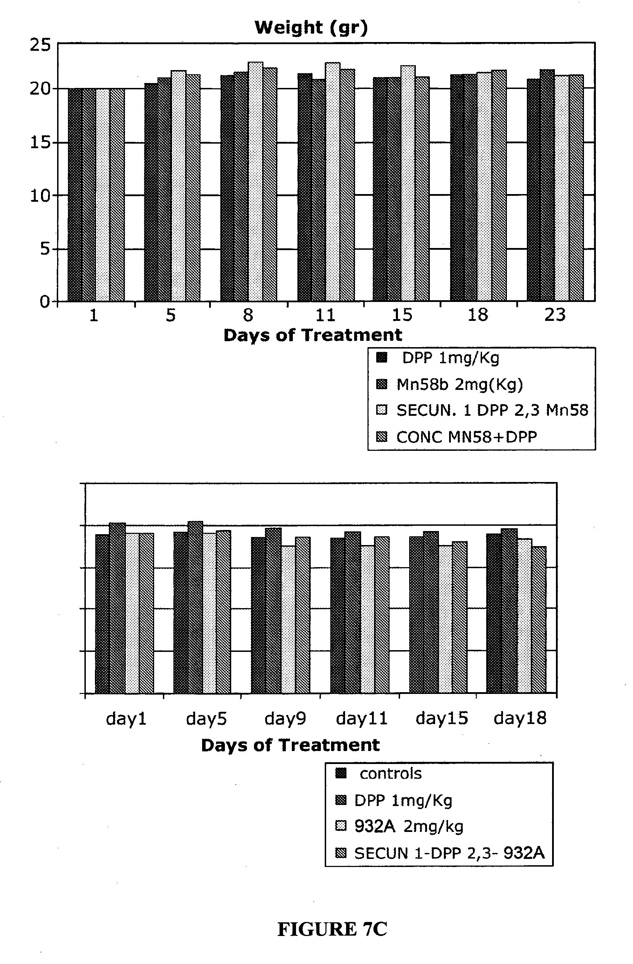

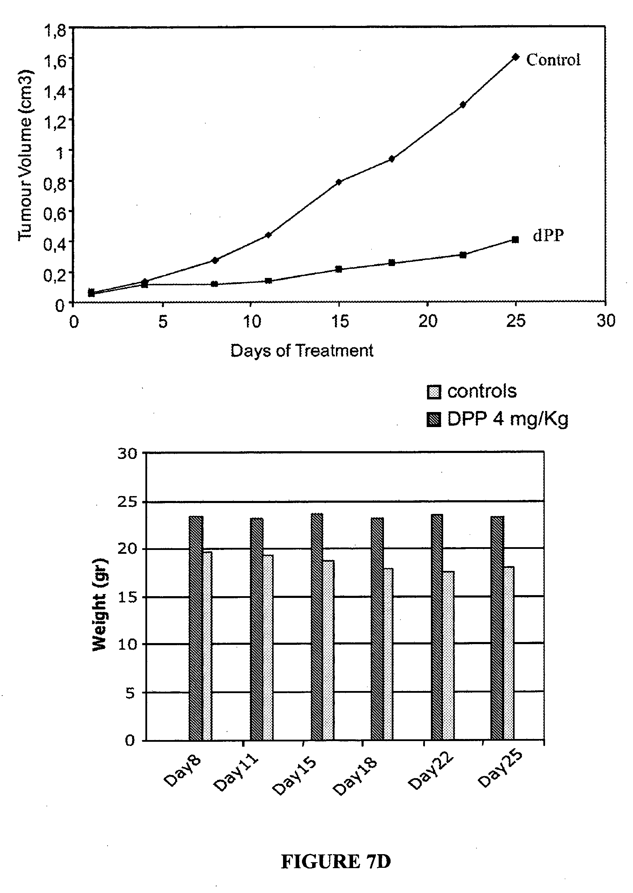

**Figure 7****.** Combined therapy of cisplatin/ChoK inhibitors in NSCLC xenografts. (A) MN58b/cispaltin (Statistical significance; MN58b p=0.09; Cisplatin p=0.3; Sequential p=0.017; Concomitant p=0.016), (B) Combined therapy in NSCLC xenografts (Statistical significance; RSM-932A (932A) p= 0.157; Cisplatin (DPP) p= 0.441; Sequential DPP and TCd-171 treatment (SEC) p= 0.03); (C) Weight monitoring as a measurement oftoxicity of the mice control and treated with ChoK inhibitors (2mg/Kg), Cisplatin (1mg/Kg) and the combined therapy group; (D) Similar antitumoral activity of cisplatin alone than that of the combined therapy results in a significant increase in toxicity.
**Figure 8****.** Sensitivity of tumor cells to choline kinase inhibitor RSM-932A (ChoKI) (A) or TRAIL (B) in the indicated cell lines.
**Figure 9****.** Cooperation of combined treatment of ChoKI and TRAIL.
**Figure 10****.** Synergistic effect of the combined therapy of MN58b and TRAIL in colon cancer cells.
**Figure 11****.** Synergistic effect of the combined therapy of MN58b and TRAIL in colon cancer cells determined by flow citometry analysis.
**Figure 12****.** ChoK inhibition increases the expression of DR5.
**Figure 13****.** Ceramides production after ChoKI/TRAIL combined therapy.
**Figure 14****.** Combined therapy ChokI/TRAIL in colon xenografts.
**Figure 15****.** Synergistic effect of the combined therapy of ChoK inhibitors and 5-FU in colon cancer cells.
**Figure 16****.** Synergistic effect of the combined therapy of ChoK inhibitors and 5-FU in colon cancer cells determined by flow citometry.
**Figure 17****.** Combined therapy ChokI/5-FU in colon xenografts.

### DETAILED DESCRIPTION OF THE INVENTION

### COMBINATIONS OF ChoK INHIBITORS AND ACID CERAMIDASE INHIBITORS (FIRST COMPOSITION OF THE INVENTION)

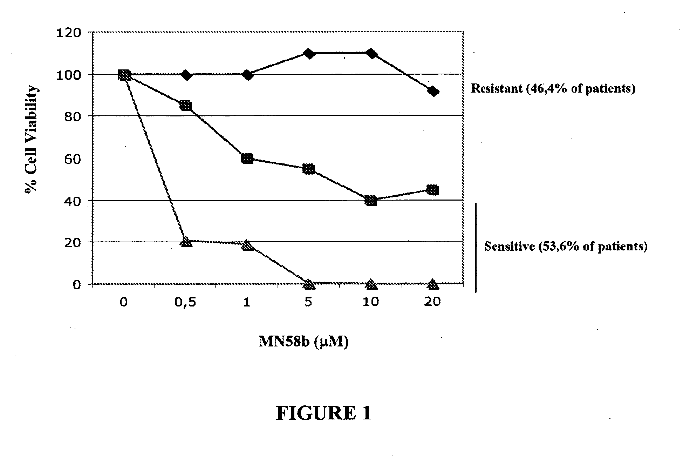

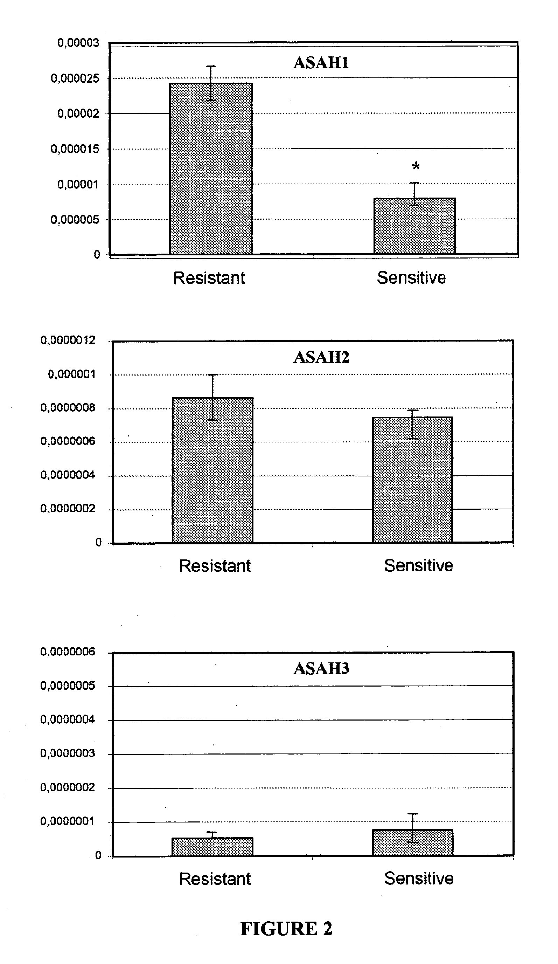

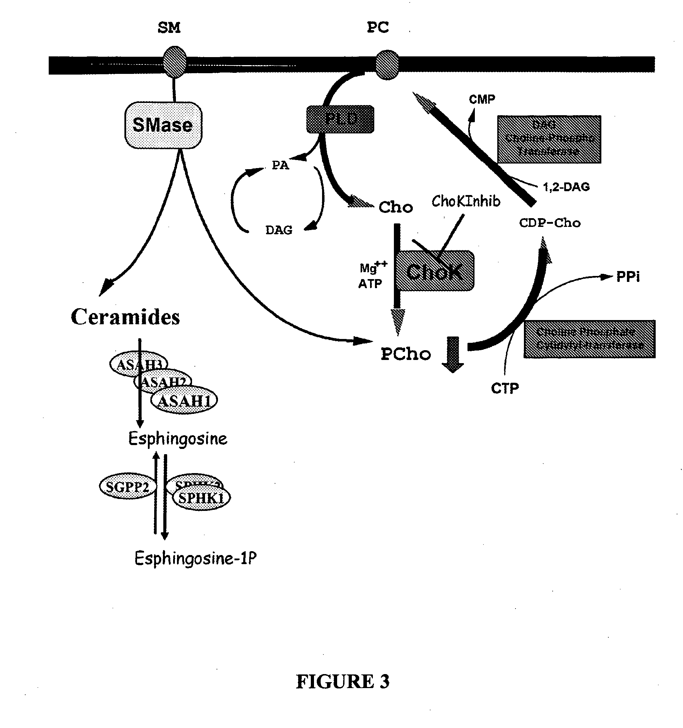

The authors of the present invention have identified the existence of primary human tumors resistant to choline kinase (ChoK) inhibitors (Figure 1). In particular, example 1 of the present invention describes that different primary cultures derived from human Non Small Cell Lung Cancer tissues show a differential sensitivity towards MN58b, a known ChoK inhibitor (Figure 1). Surprisingly, this resistance has been shown to be caused by an increase in the expression levels of acid ceramidase, as it has been shown in examples 2 and 3 of the present invention (Figure 2). The function of ChoK is to phosphorylate Cho to generate phophocholine (Pcho), a precursor of the major component of the plasma membrane, phosphatidylcholine (PC) (Lacal JC., IDrugs. 2001; 4:419-26). However, membranes and therefore PC are absolutely required for cell proliferation. Thus, without wishing to be bound by any theory, it is believed that tumoral cells respond to ChoK inhibition by the activation of an alternative pathway to generate PCho through the degradation of sphingomyeline (SM) (Figure 3). However, the cleavage of SM leads not only to the production of PCho but also of pro-apoptotic ceramides, resulting in that the tumoral cells engage in apoptotic cell death. Interestingly, it has been identified herein that those cells resistant to ChoK inhibition display increased levels of acid ceramidase, an enzyme that promotes the conversion of pro-apoptotic ceramides to pro-mitogenic sphingosine-1P (Figure 3). These results suggest that over-expression of acid ceramidase could inhibit the promotion of cell death mediated by ChoK inhibition by decreasing the intracellular levels of ceramides. Therefore, the proapoptotic signal triggered by ChoK inhibition in tumor cells is inactivated by overexpression of acid ceramidase. Furthermore, generated ceramides could be converted into the pro-mitogenic sphingosine-1P (Figure 3). This finding opens the possibility for improved therapies for cancer by increasing the sensitivity to ChoK inhibitors by the simultaneous administration of acid ceramidase inhibitors. In fact, example 4 of the present application shows that treatment of NSCLS-derived tumor cells with the acid ceramidase inhibitor NOE results in an increased sensitivity to ChoK inhibitors. Moreover, the combined use of the ChoK inhibitor MN58b or RSM-932A and the acid ceramidase inhibitor D-NMAPPD results in an improved antitumoral effect when compared with the use of the inhibitors in isolated manner or when compared with the combined used of a ChoK inhibitor and an inhibitor specific for alkaline ceramidase (see example 4). In this way, the combined administration of acid ceramidase inhibitors and ChoK inhibitors would allow the use of lower dosages of the later compounds, thus leading to less undesired side effects.

Thus, in a first aspect, the invention provides a composition (hereinafter the first composition of the invention) comprising, separately or together, a first component comprising one or more choline kinase inhibitors specific for the choline kinase alpha isoform and a second component comprising one or more acid ceramidase inhibitors.

The term "composition" refers to one or more compounds in various combinations according to alternative embodiments of this invention. Preferably, the composition comprises at least an acid ceramidase inhibitor and at least a ChoK inhibitor specific for the choline kinase alpha isoform.

Choline kinase, as used herein, refers to an enzyme which catalyses the phosphorylation of choline in the presence of ATP to produce phosphorylcholine (PCho) (EC 2.7.1.32). Exemplary choline kinases which can be inhibited according to the present invention include choline kinase alpha (as defined in UniProt under accession numbers P35790, 054804 and Q01134 for the human, mouse and rat proteins, respectively).

Acid ceramidase (N-acylsphingosine deacylase activity, EC 3.5.1.23), as used herein, is the lipid hydrolase responsible for the degradation of ceramide into sphingosine and free fatty acids within lysosomes. The cells contains at least three types of ceramidases which are classified, according to their pH optima for activity and location (Li CM. et al., Genomics, 1999, 62:223-31), as acid ceramidase (ASAH1, NM_177924.3 or Q13510), neutral ceramidase (ASAH2, NM_019893 or Q9NR71) and alkaline ceramidase (ASAH3, NM_133492, Q8TDN7 or Q5QJU3). A second acid ceramidase (known as acid ceramidase-like or ASAHL) polypeptide has also been described (UniProt Accession number is Q02083). The inhibitors for use in the present invention are those which inhibit at least acid ceramidase and/or the acid ceramidase-like protein, since none of the other two ceramidases show a significative increase in expression in ChoK inhibitor-resistant cells.

Acid ceramidase activity is aberrantly expressed in several human cancers. This enzyme may be useful as a new target in cancer, and could be involved in anti-oncogenic treatment resistance (Seelan RS., Genes Chromosomes Cancer. 2000, 29:137-46; Liu X., Front Biosci. 2008;13:2293-8 and Morales A., Oncogene. 2007, 26:905-16).

### Choline kinase inhibitors

Choline kinase inhibitors, as used herein, relates to any compound capable of causing a decrease in the ChoK activity, including those compounds which prevent expression of the ChoK gene, leading to reduced ChoK mRNA or protein levels as well as compounds that inhibit ChoK causing a decrease in the activity of the enzyme.

The choline kinase inhibitors are specific for choline kinase alpha.

Compounds leading to reduced ChoK mRNA levels can be identified using standard assays for determining mRNA expression levels such as RT-PCR, RNA protection analysis, Northern blot, in situ hybridization, microarray technology and the like.

Compounds leading to reduced ChoK protein levels can be identified using standard assays for determining protein expression levels such as Western-blot or Western transfer, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of protein biochips or microarrays which include specific antibodies or assays based on colloidal precipitation in formats such as dipsticks.

The determination of the inhibitory capacity on the biological activity of choline kinase is detected using standard assays to measure the activity of choline kinase such as the methods based on the detection of the phosphorylation of [¹⁴C] labelled choline by ATP in the presence of purified recombinant choline kinase or a fraction enriched in choline kinase followed by detection of the phosphorylated choline using standard analytical techniques (e.g. TLC) as described in EP1710236.

Compounds that are specific for choline kinase alpha (ChoKα) can be identified by either detecting a decrease in ChoKα mRNA levels using probes specific for ChoKα and which do not hybridise under stringent conditions with other ChoK isoforms (e.g, ChoKβ) or by detecting a decrease in ChoKα protein levels using antibodies specific for ChoKα and which do not bind other ChoK isoforrns (e.g, ChoKβ). Suitable reagents for the specific determination of ChoKα mRNA and ChoKα protein levels have been described in detail in WO2006108905.

Exemplary choline kinase inhibitors that can be used in the first composition of the present invention are described under I to XVIII in Table 1.

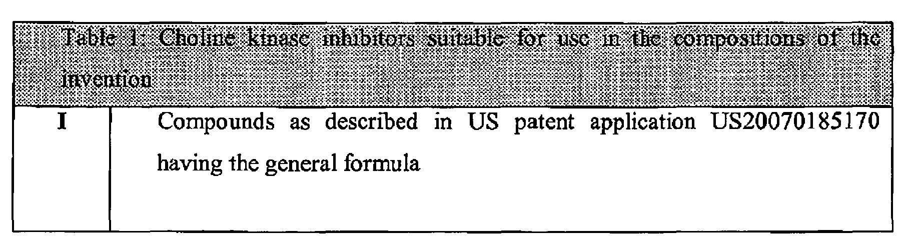

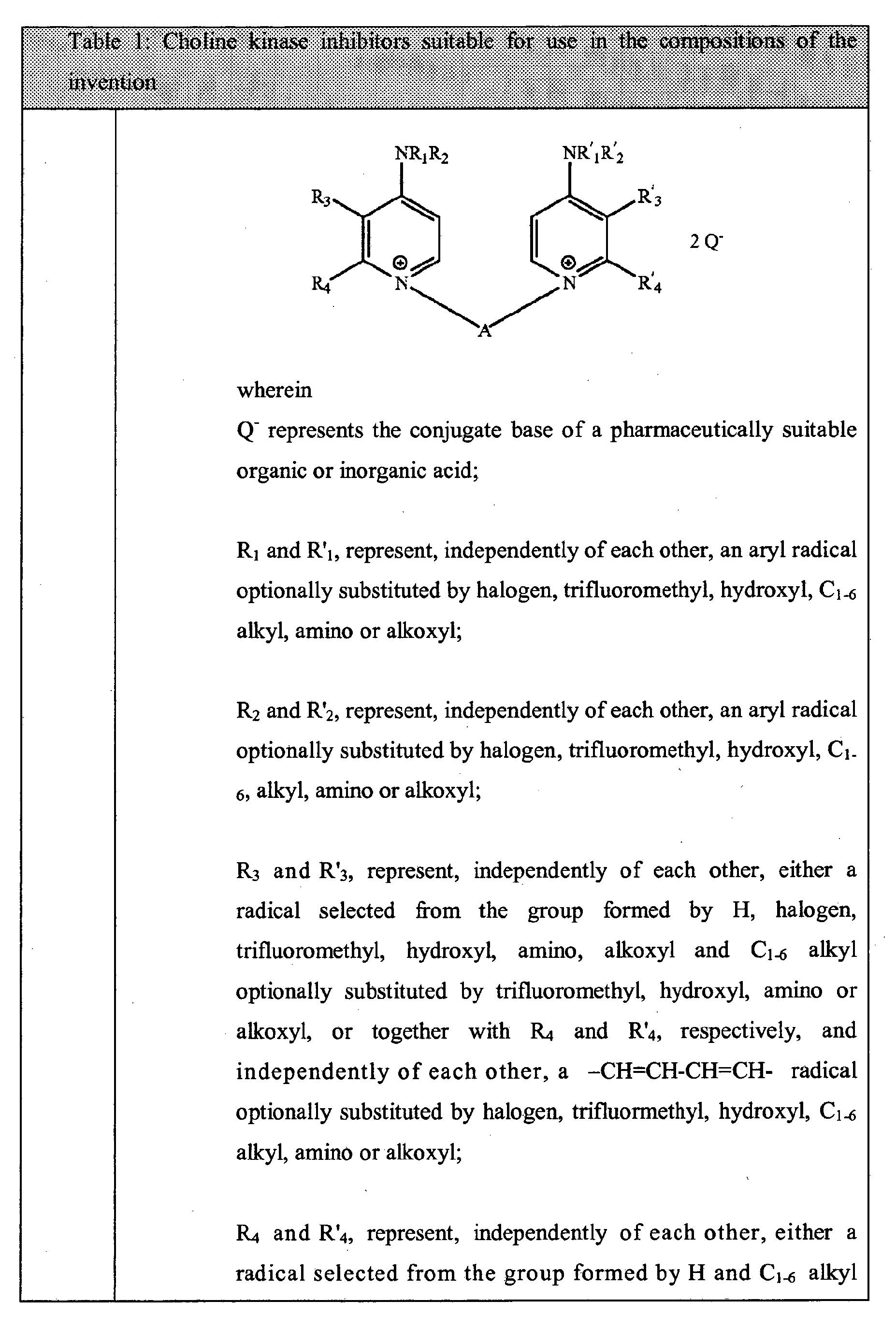

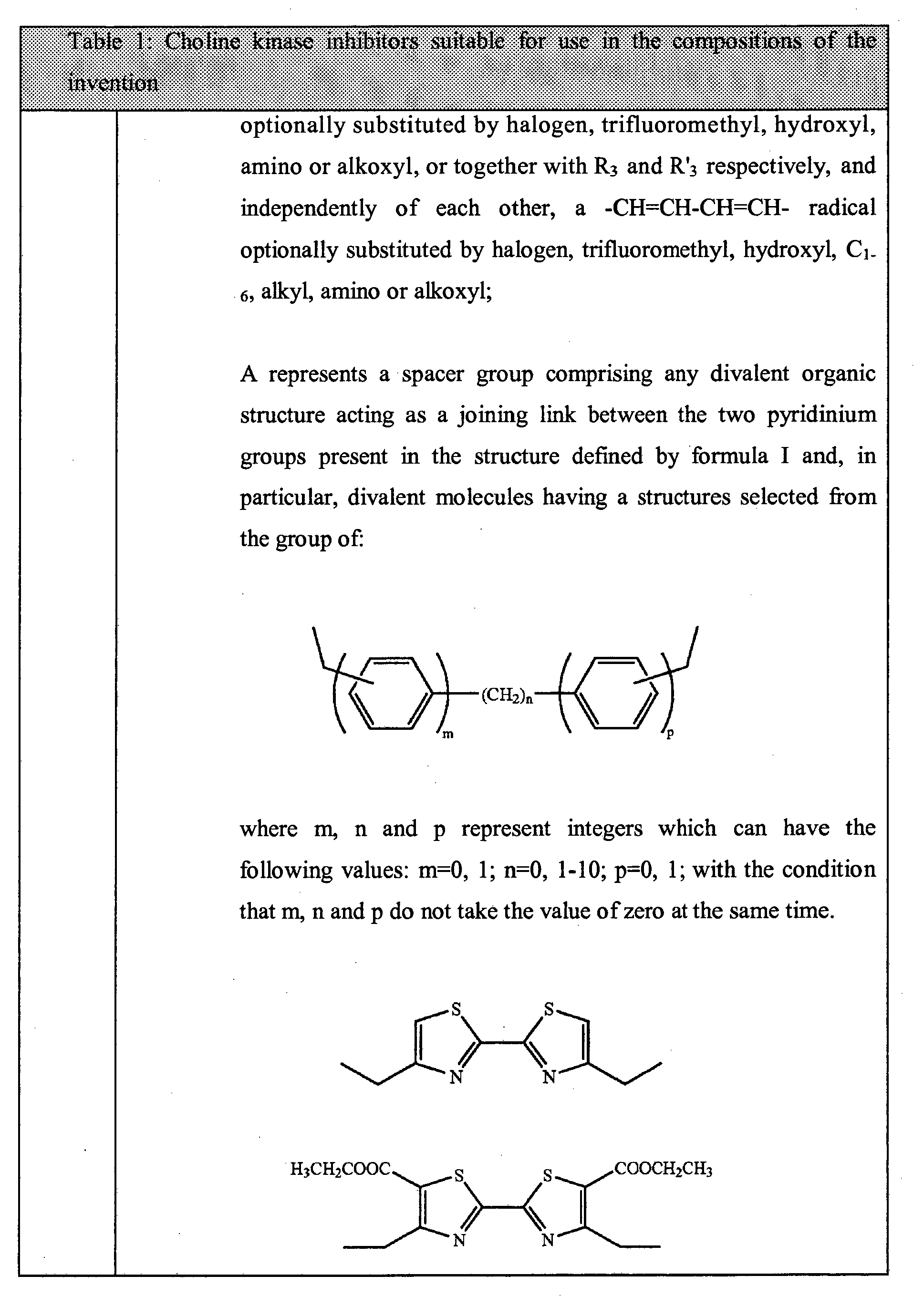

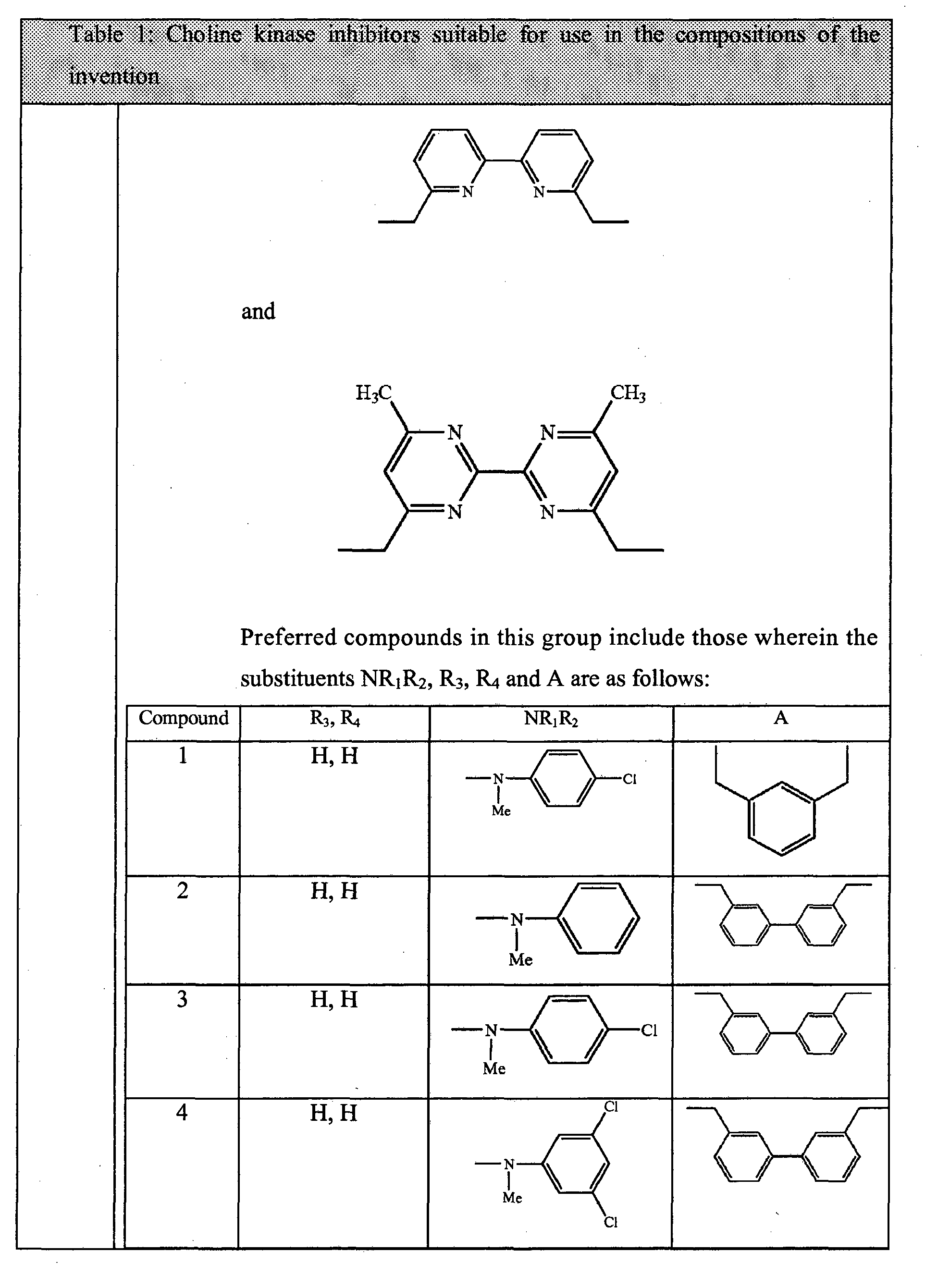

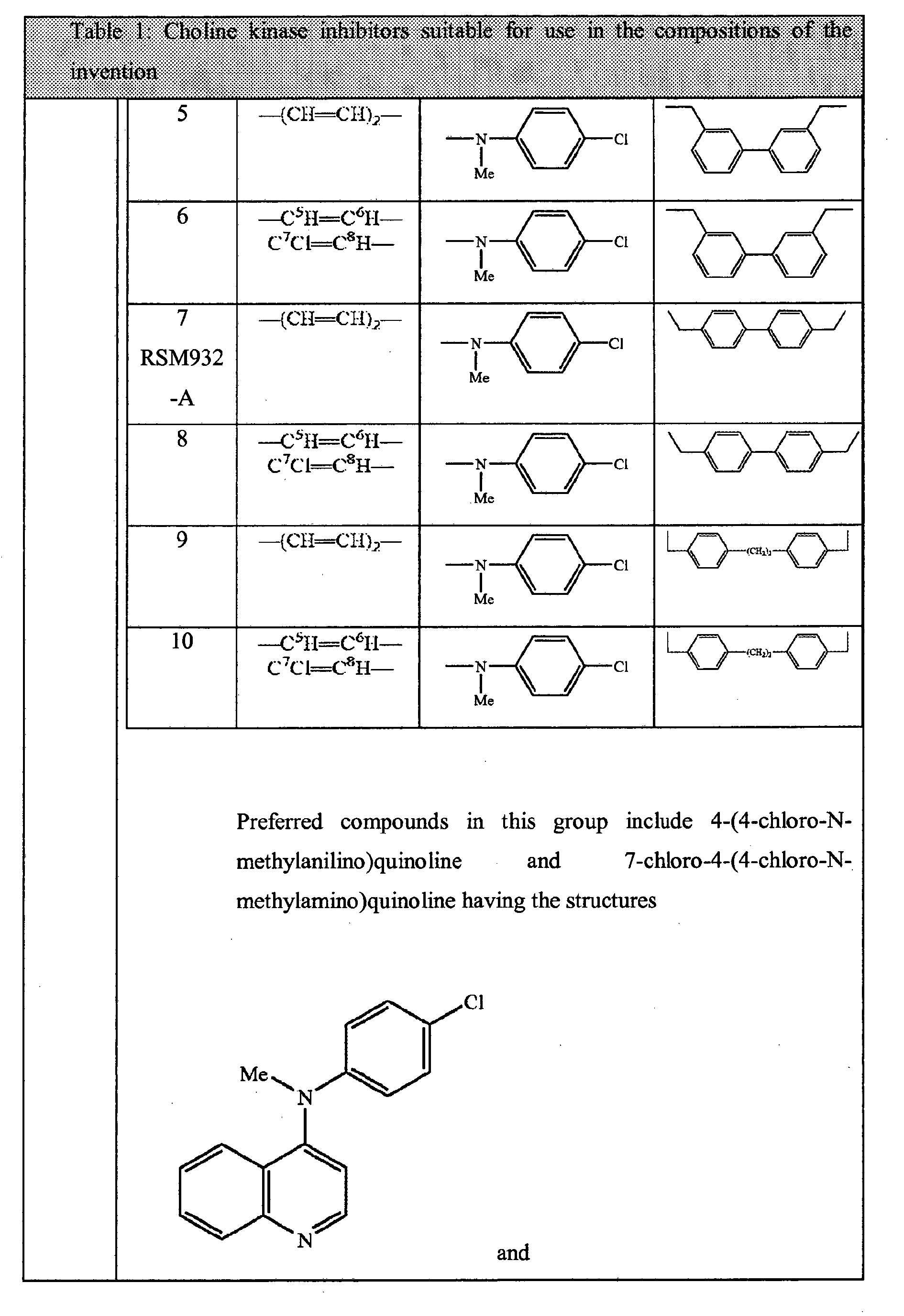

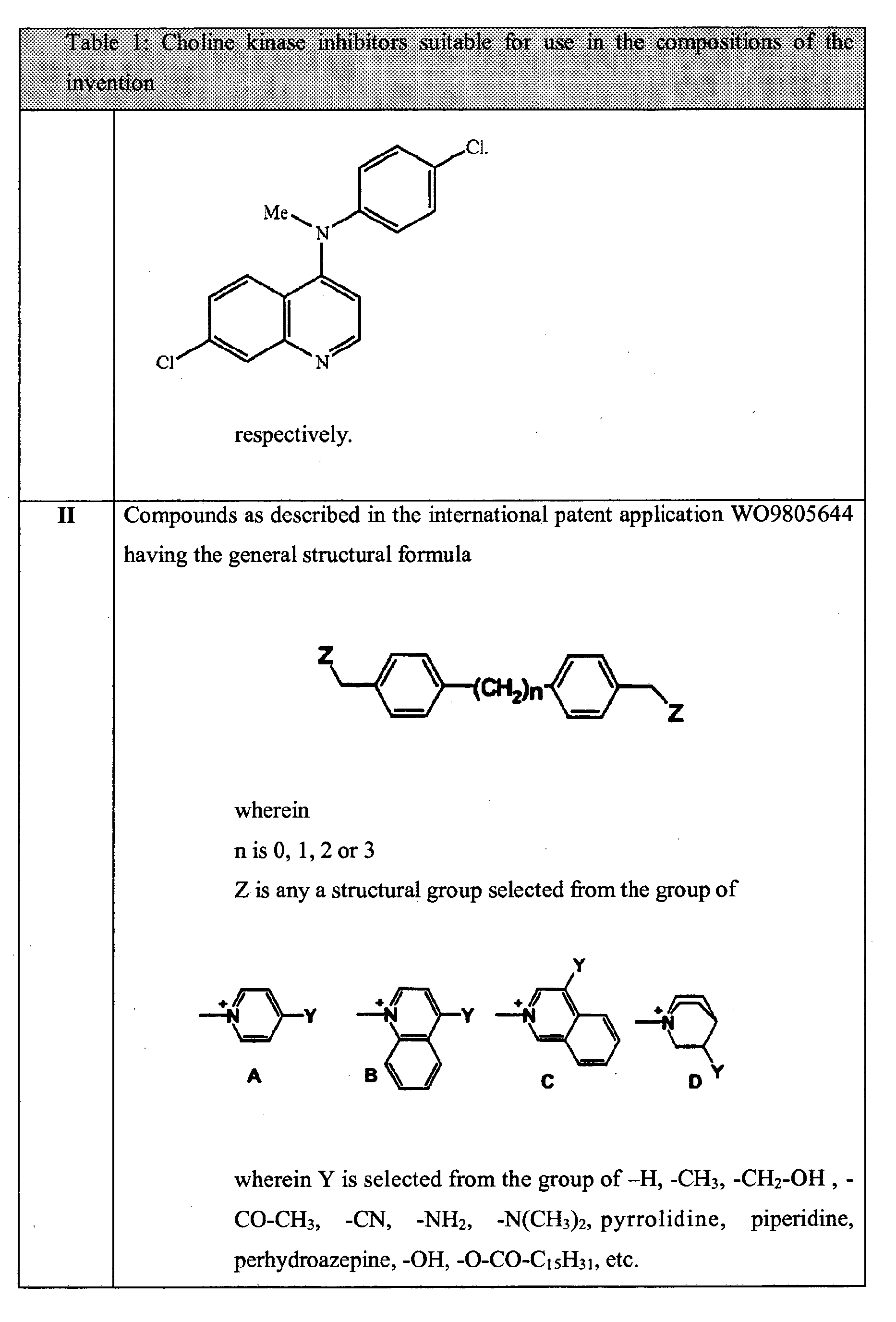

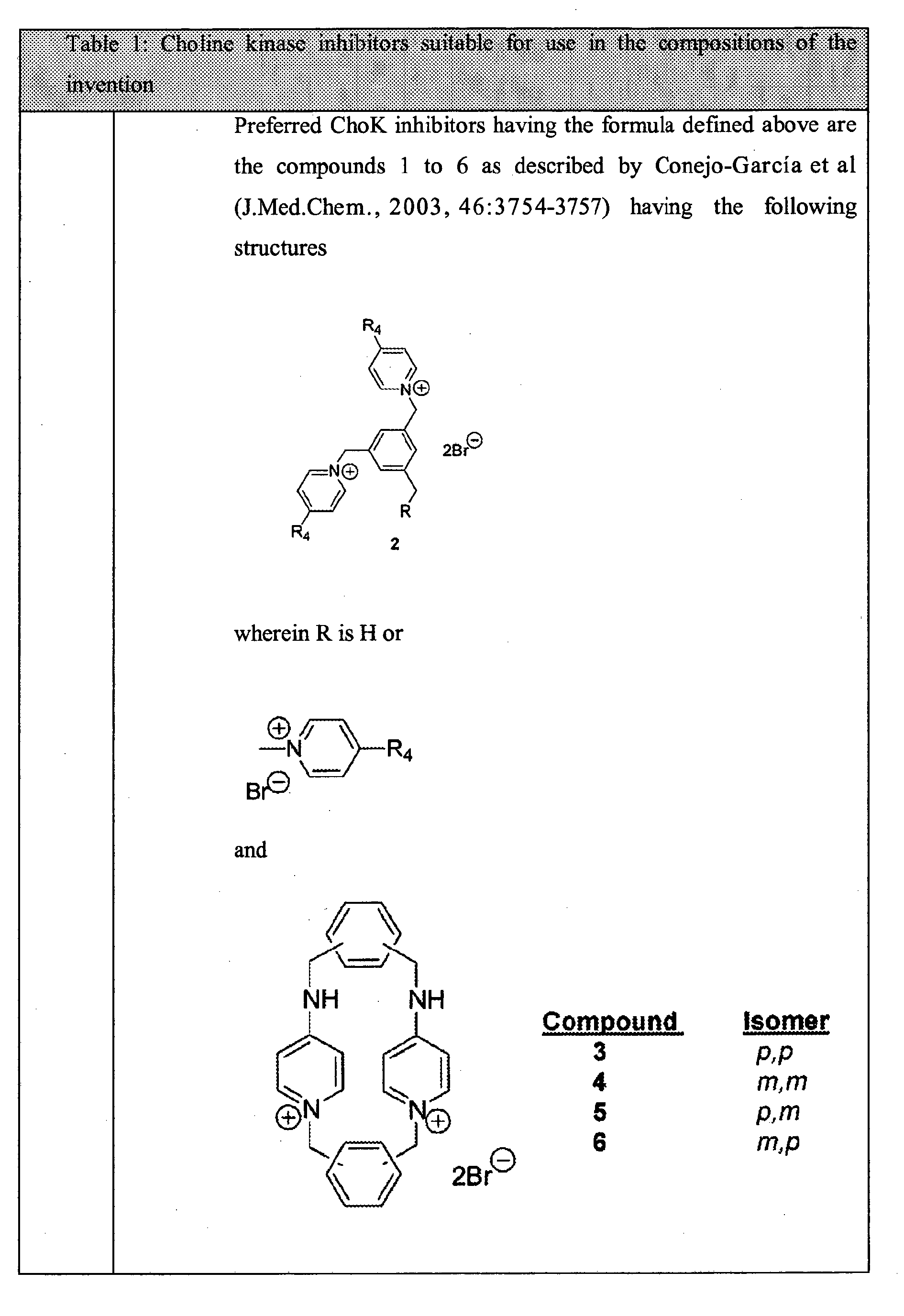

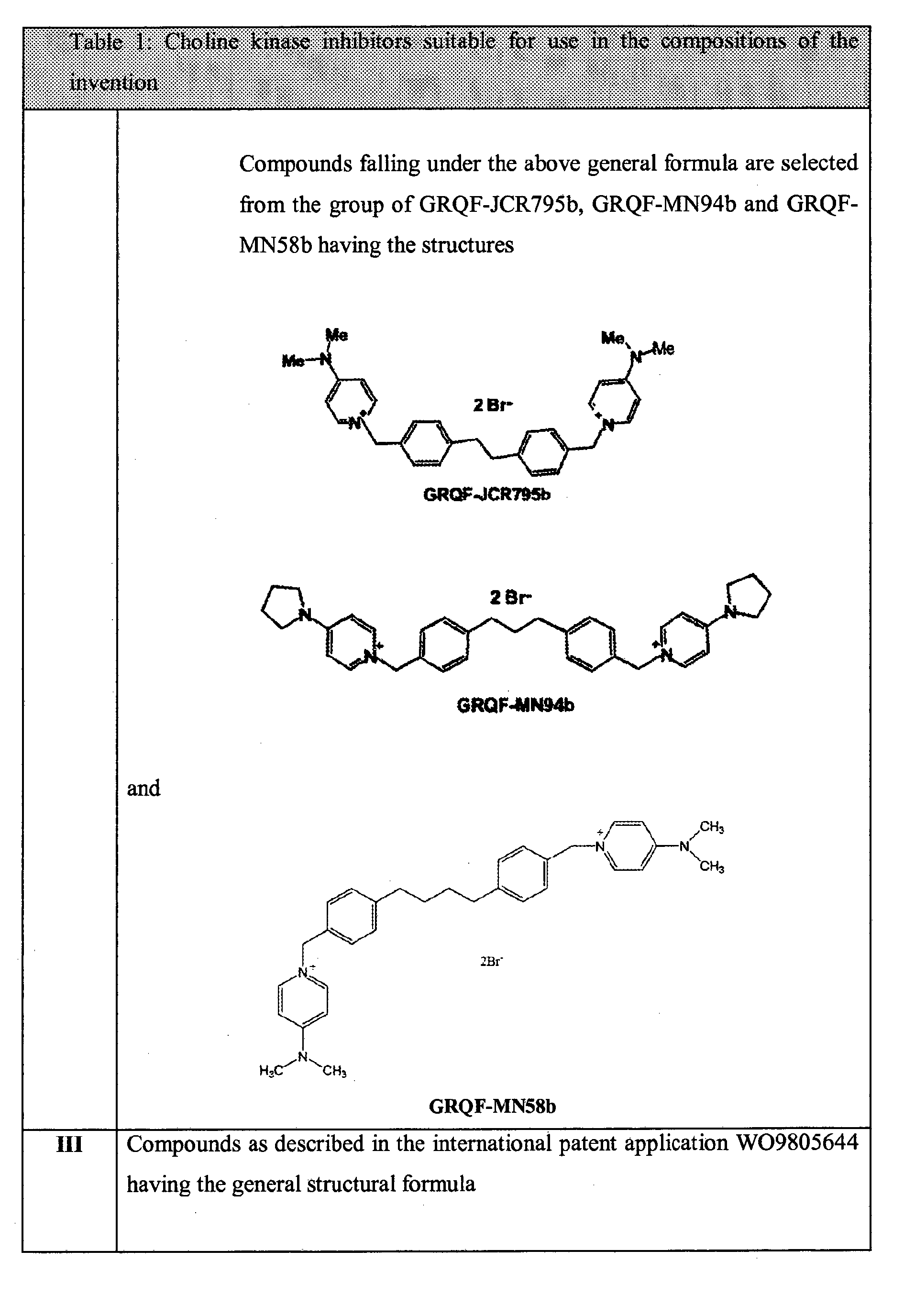

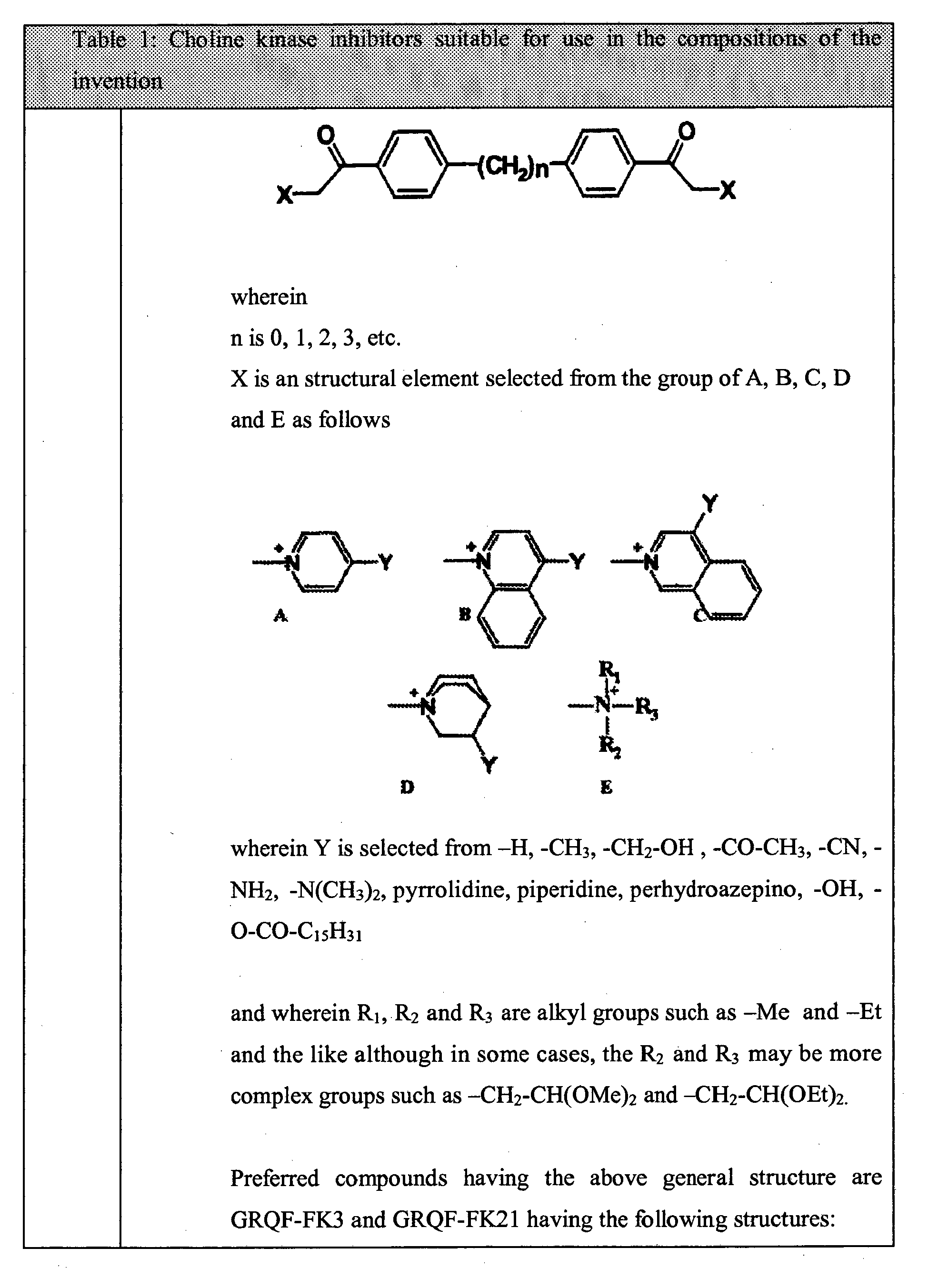

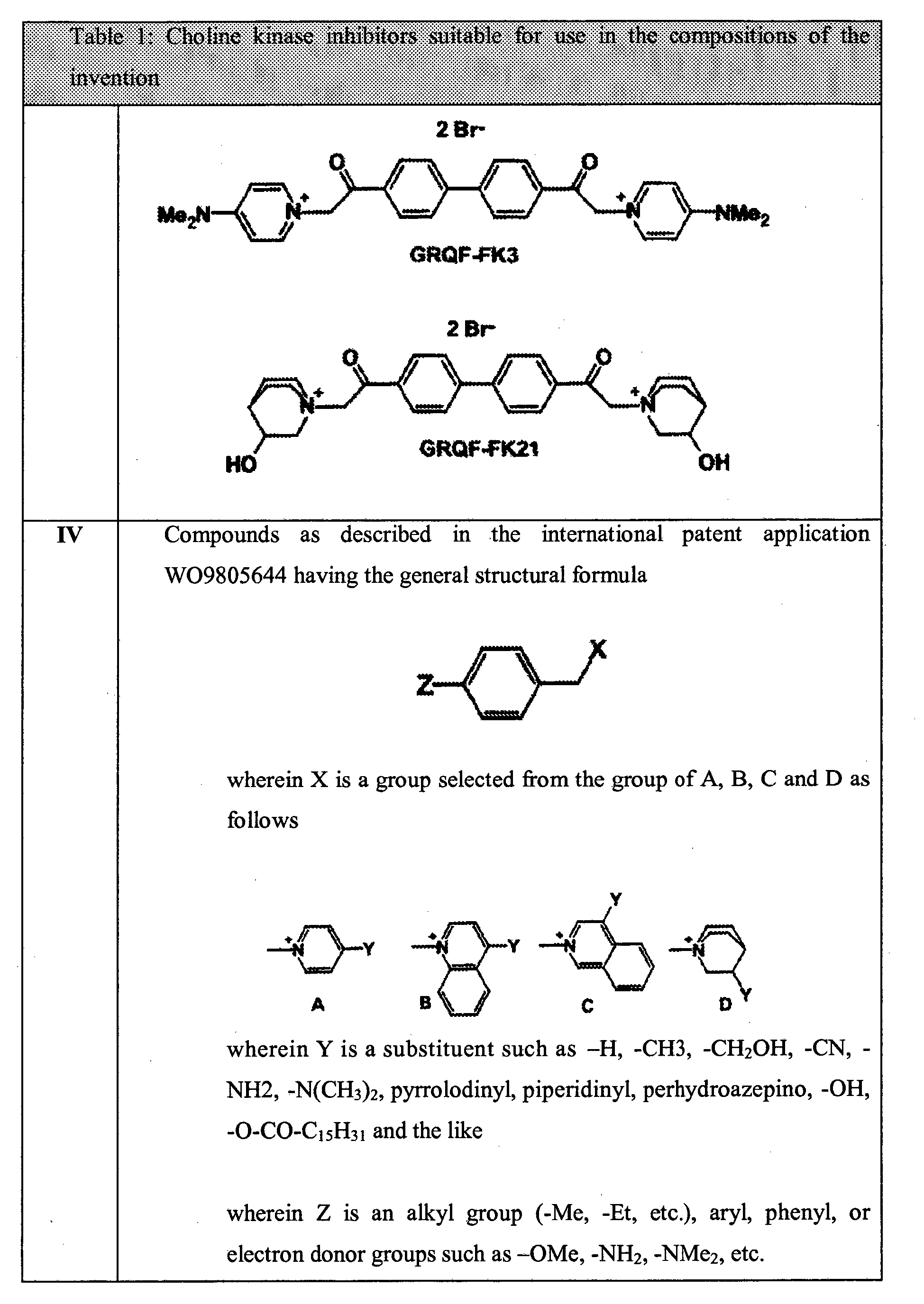

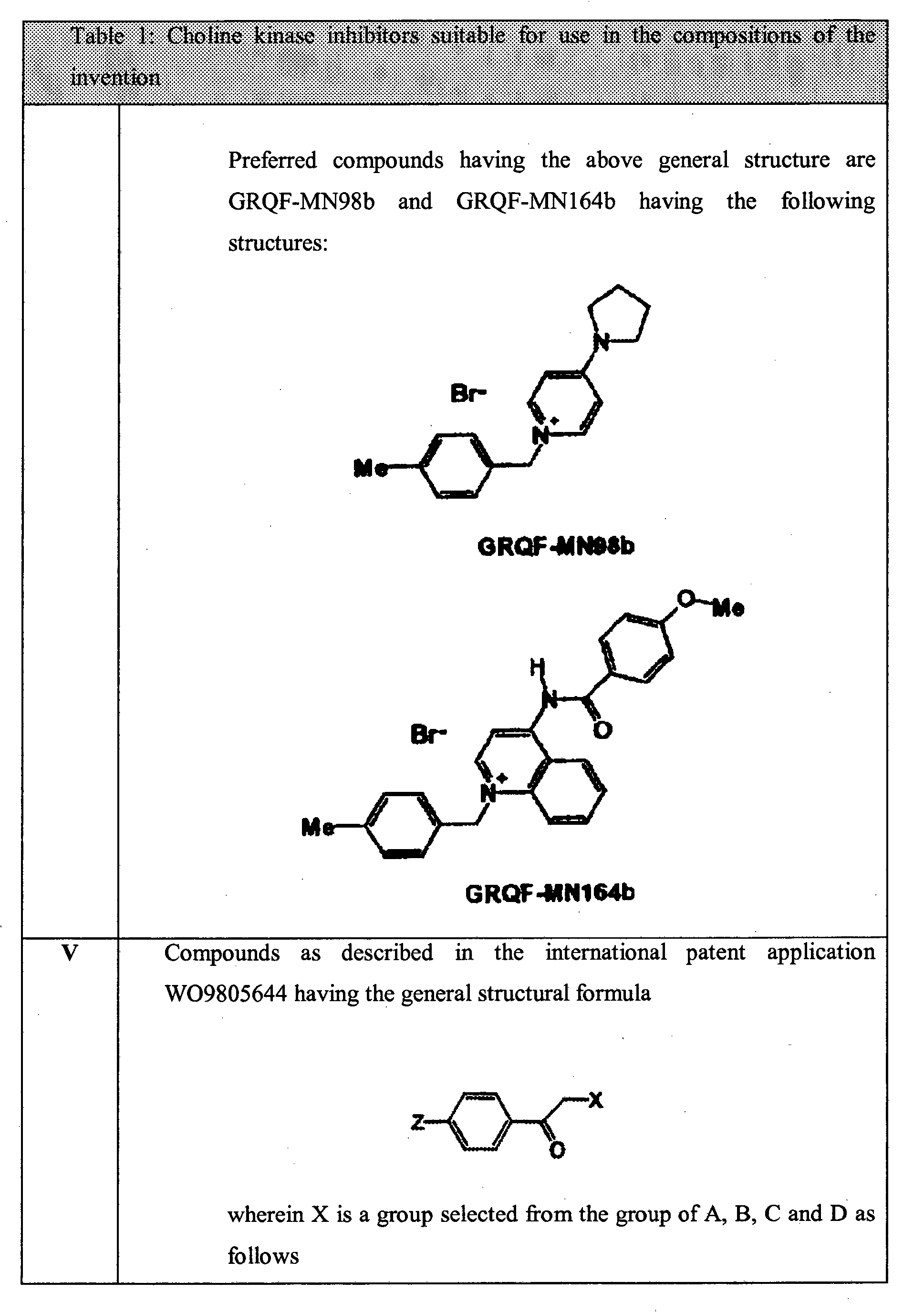

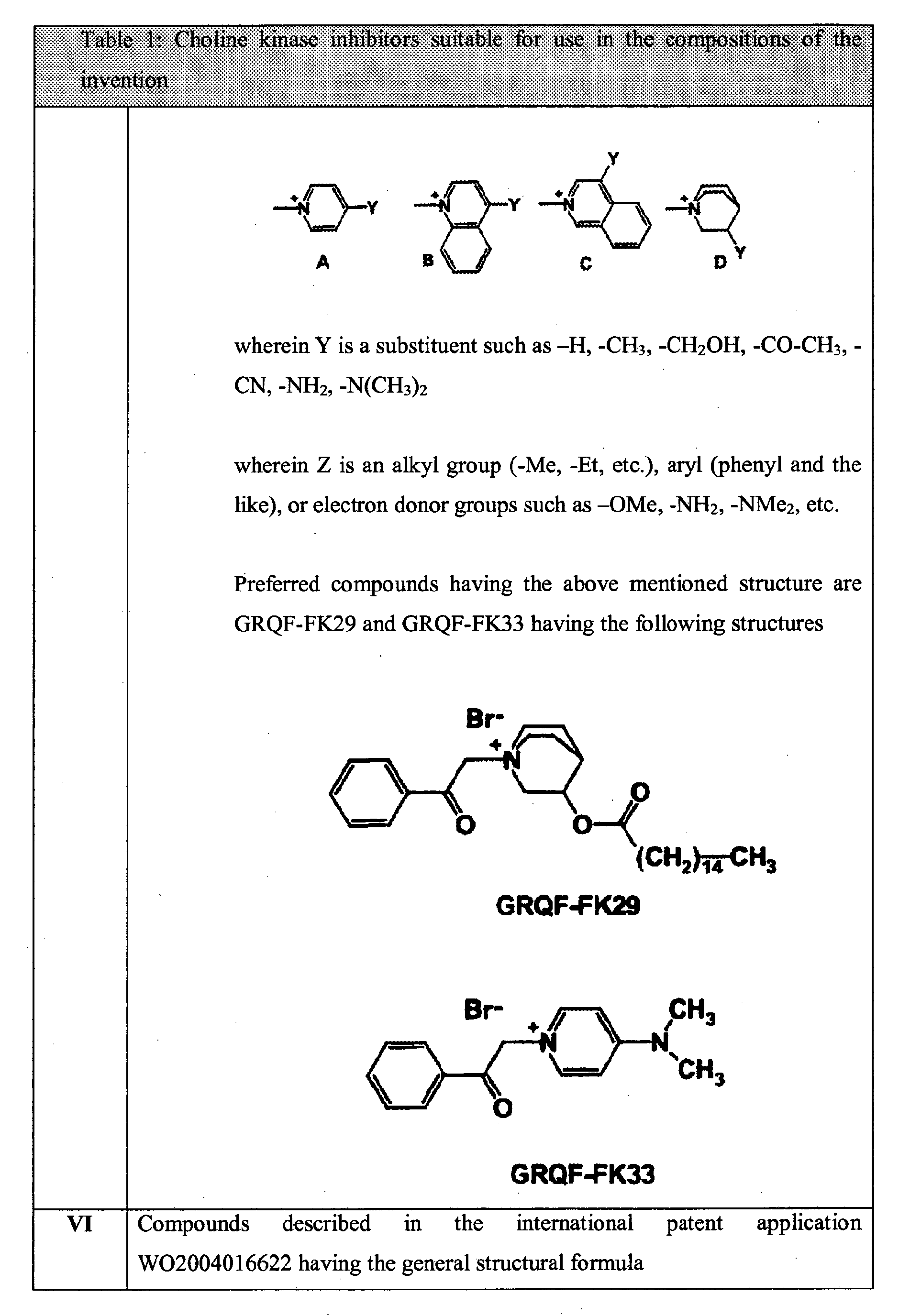

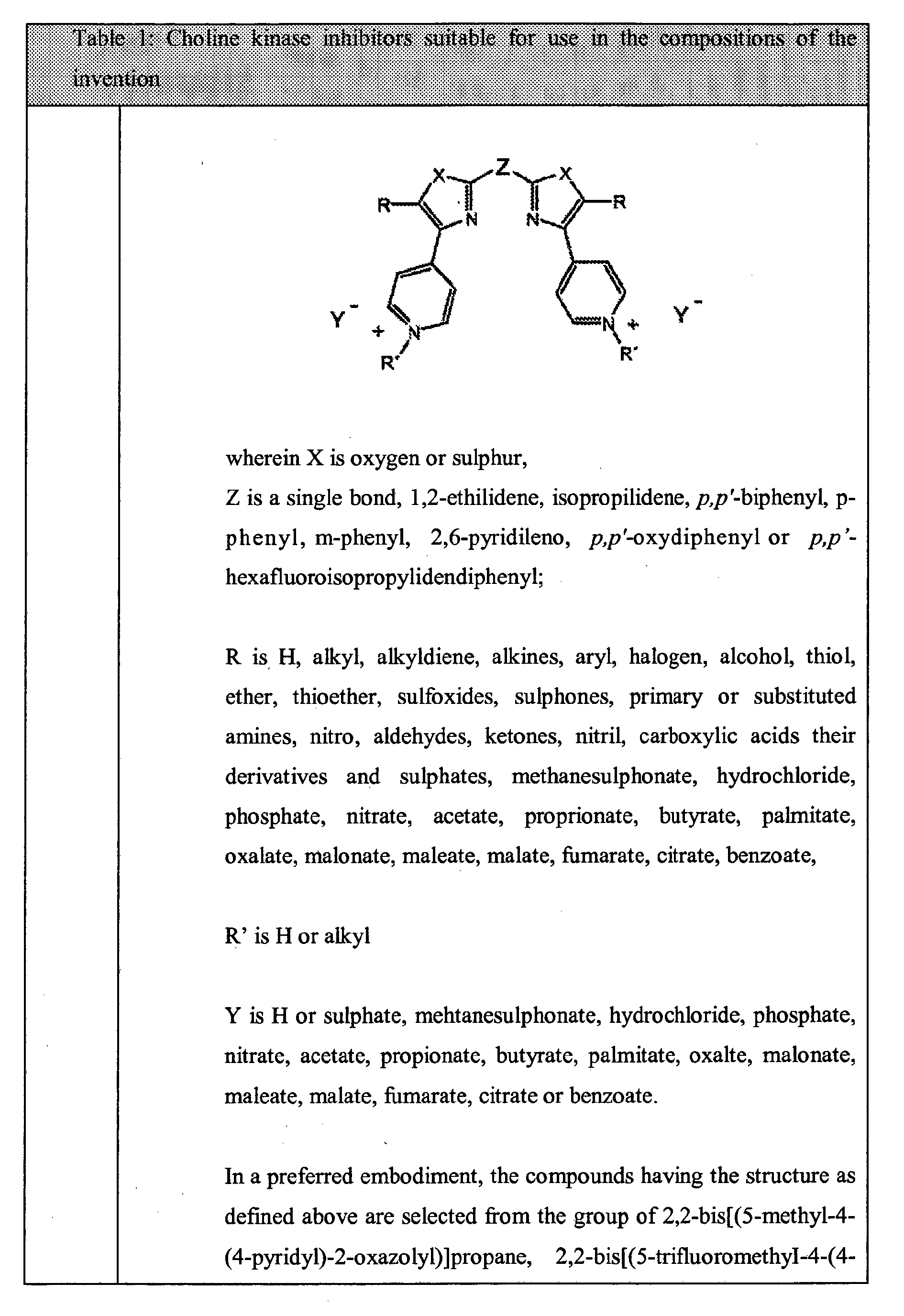

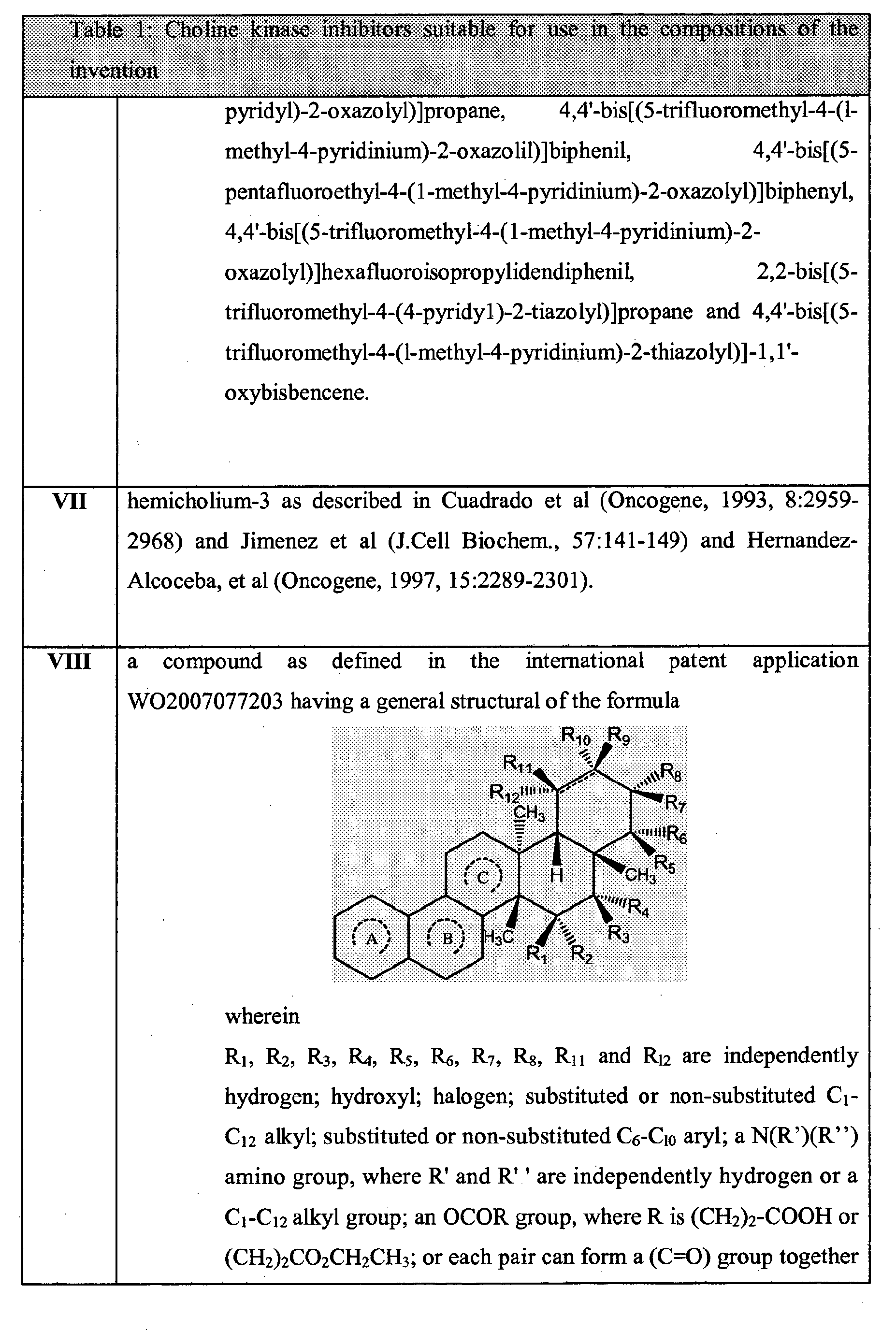

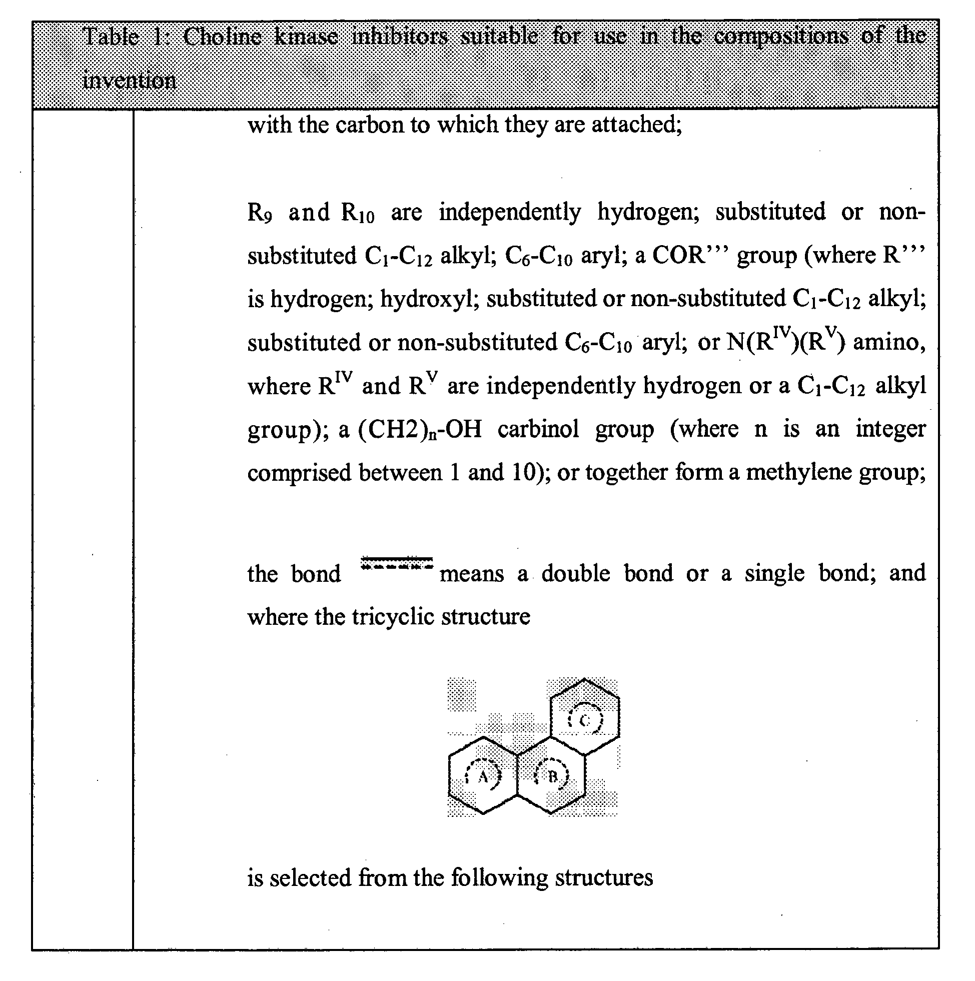

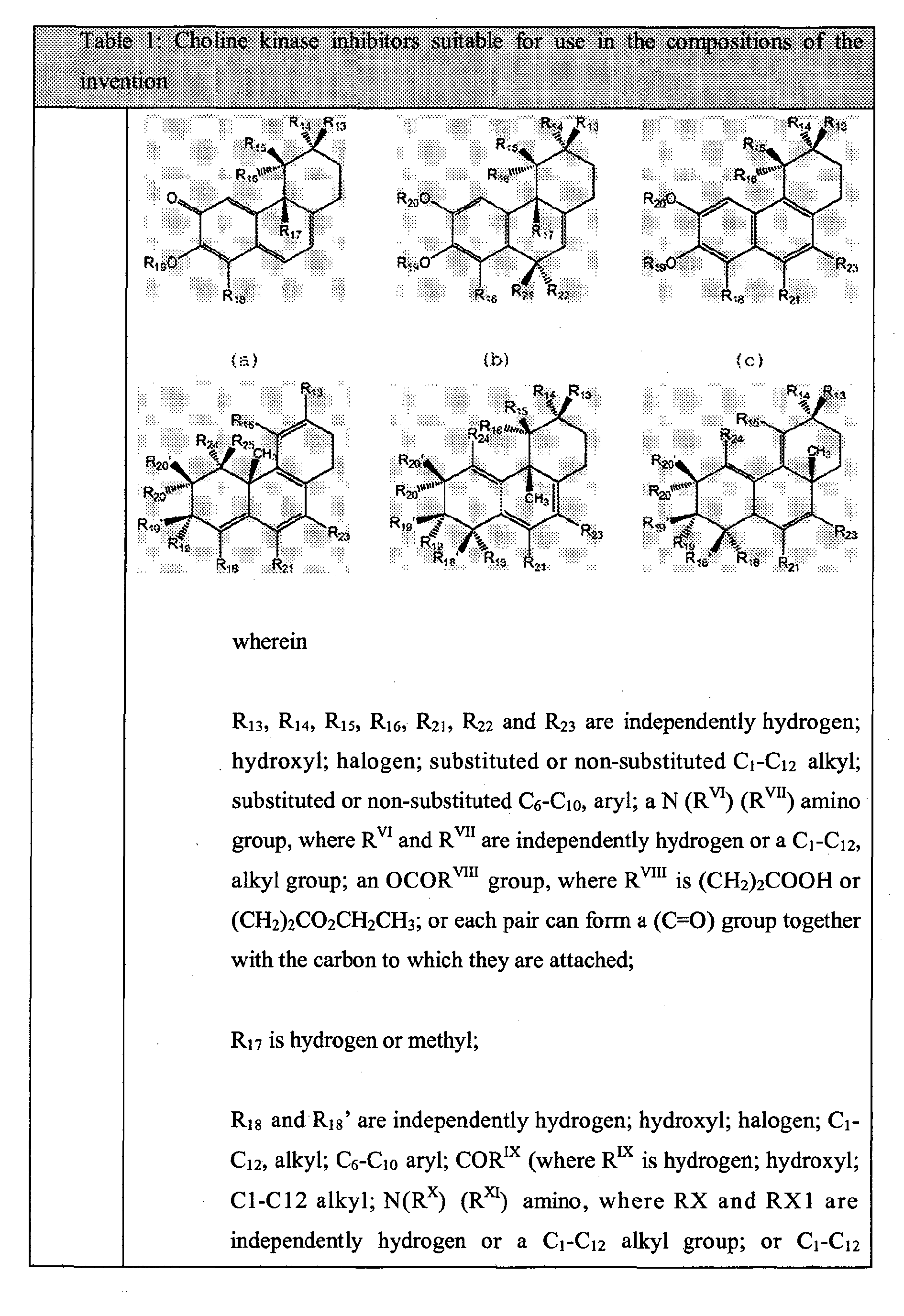

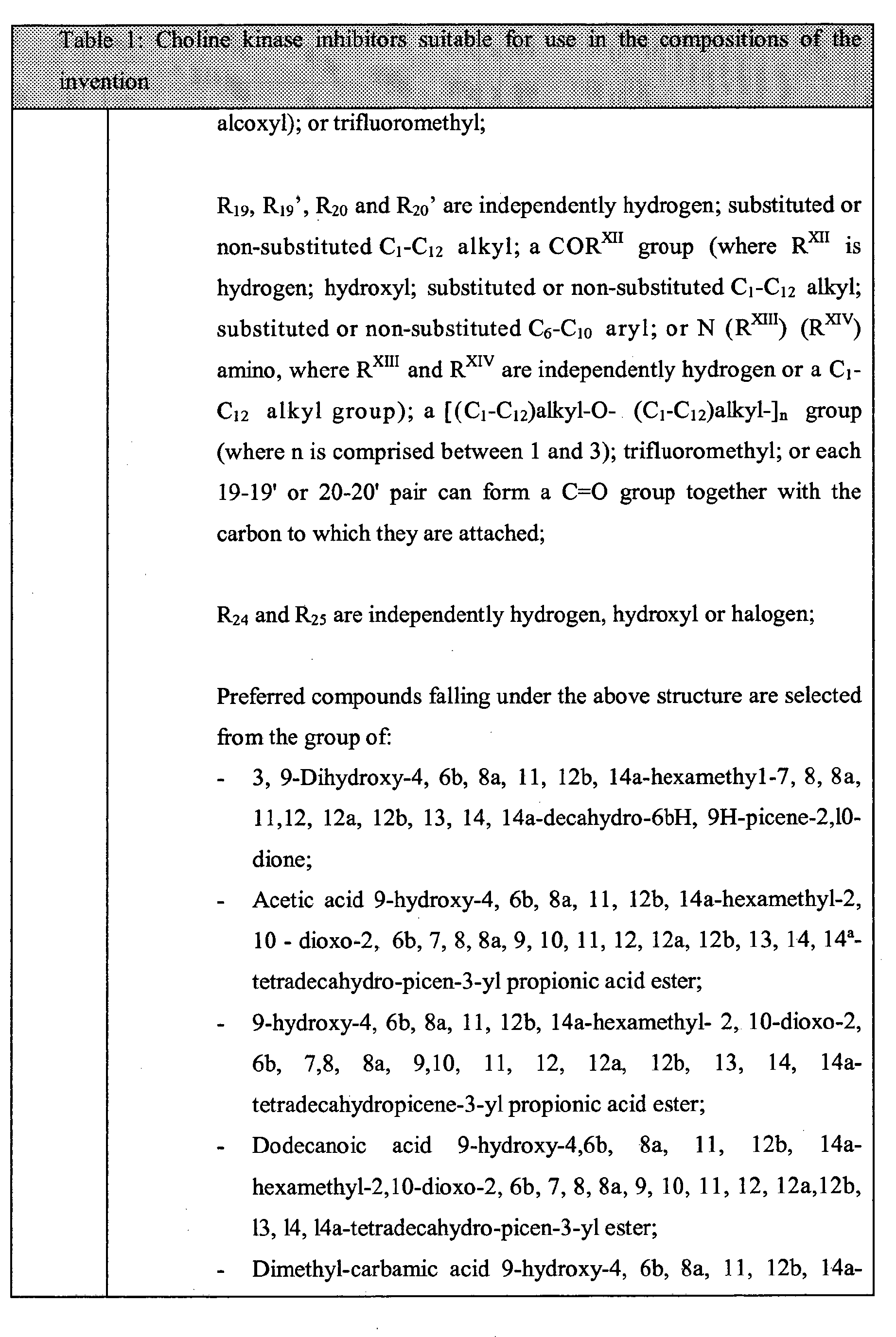

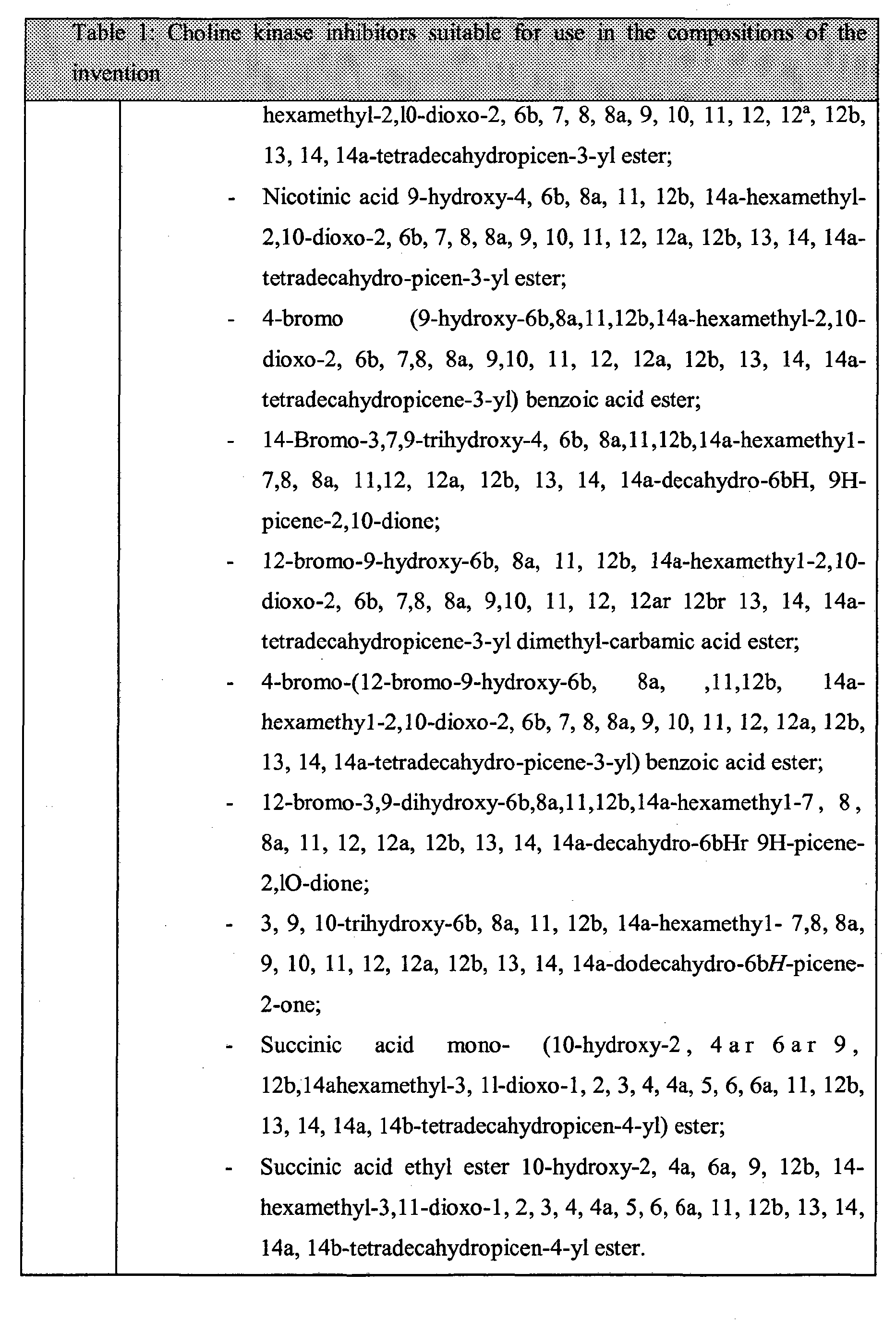

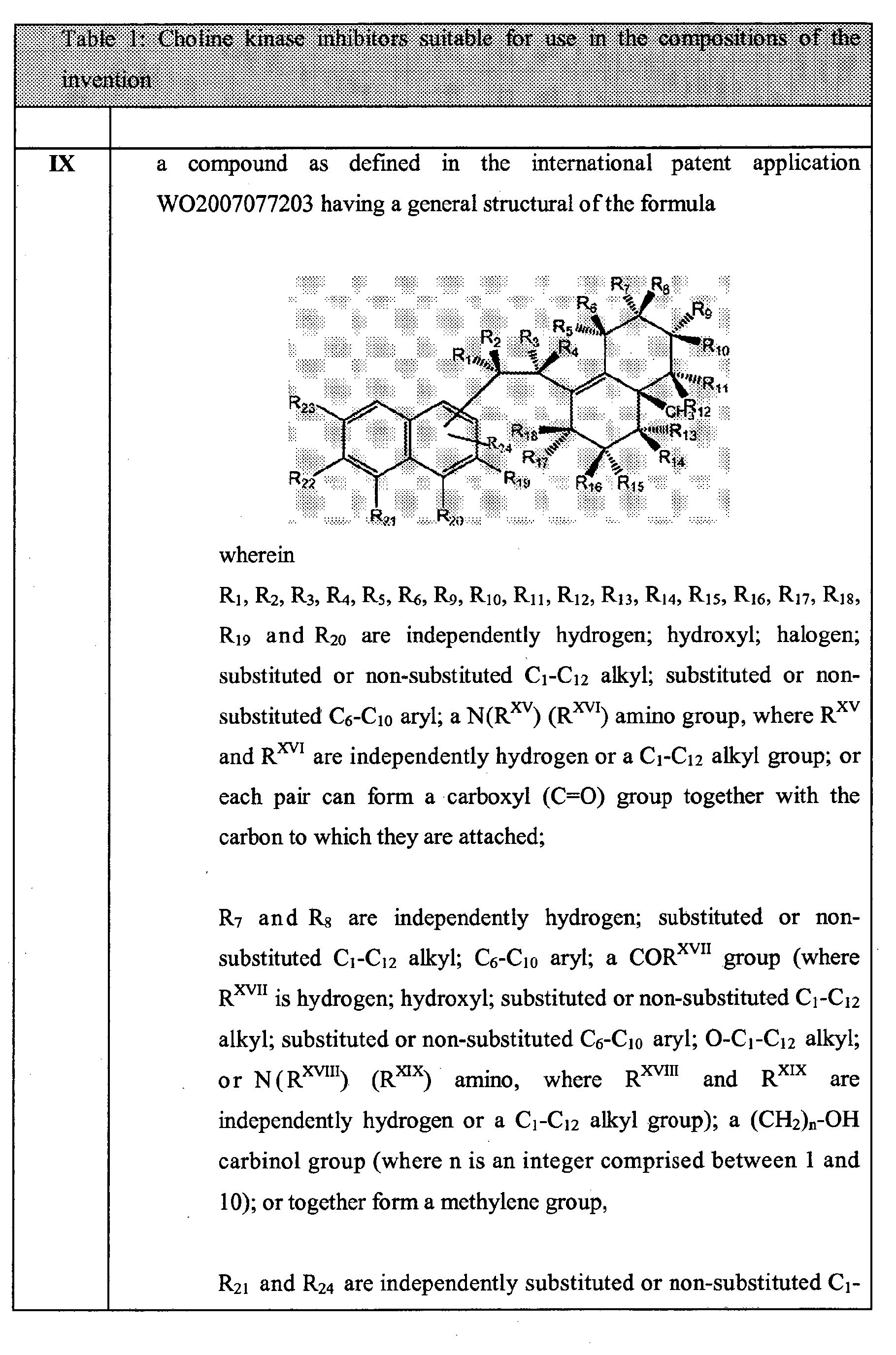

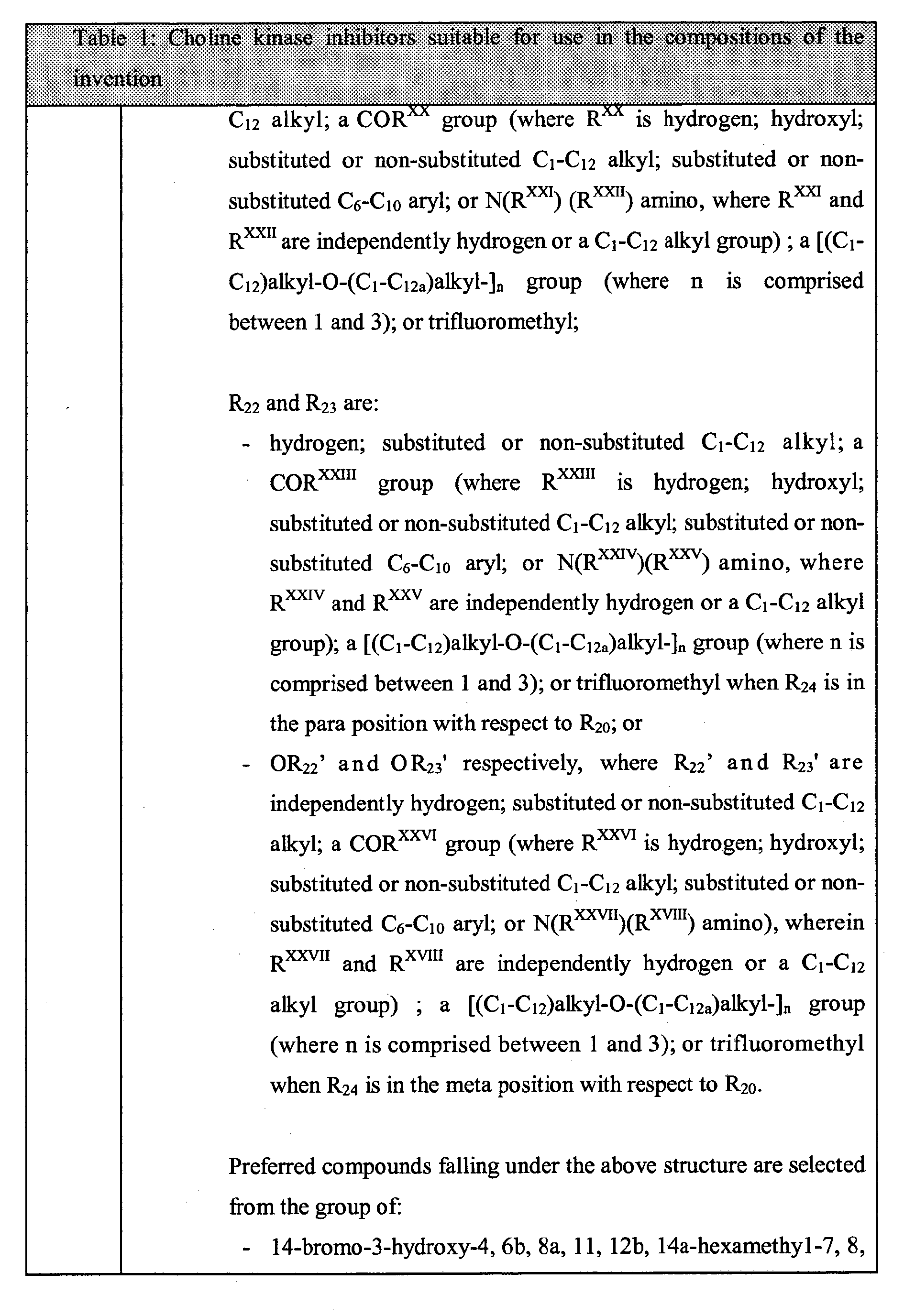

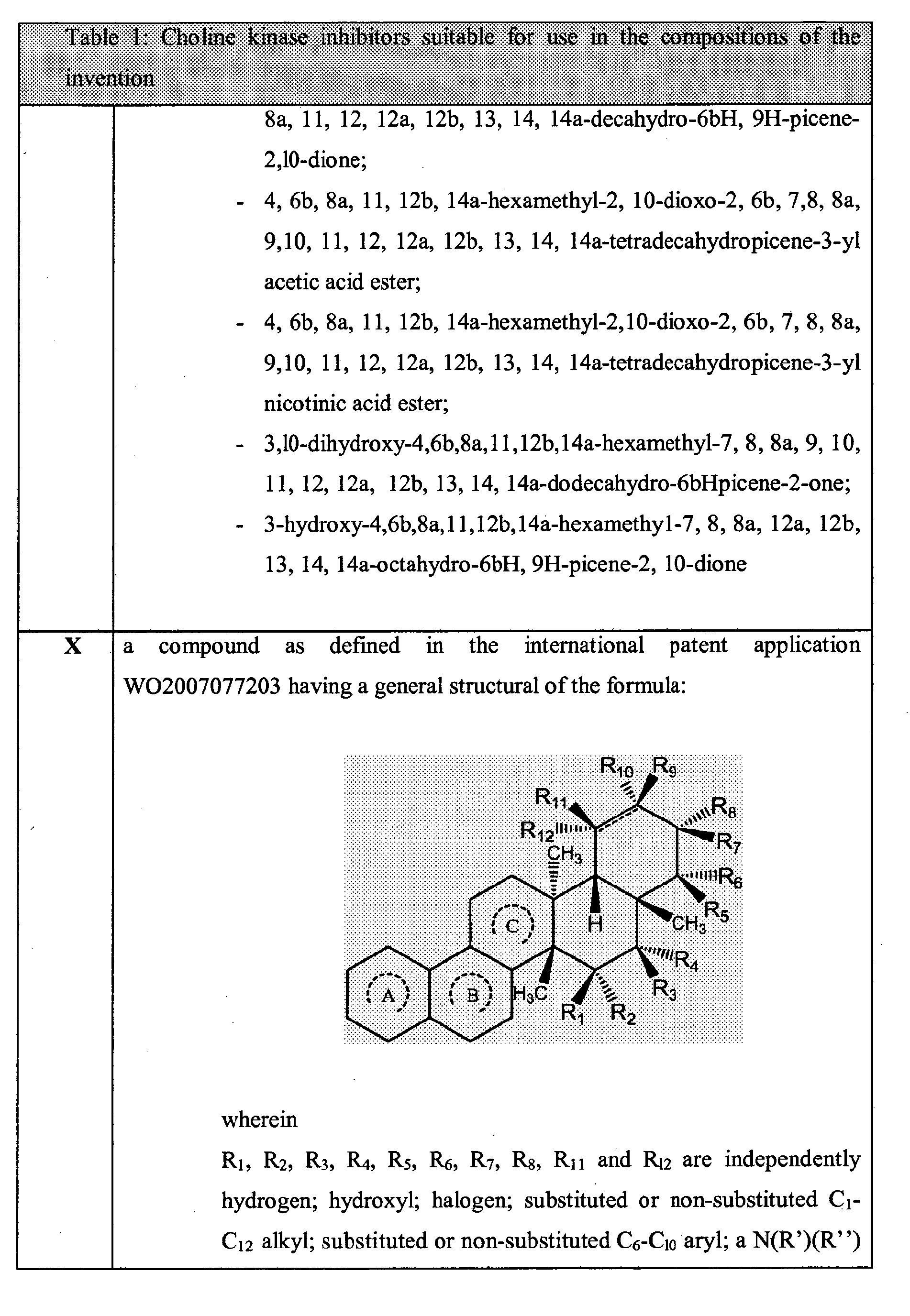

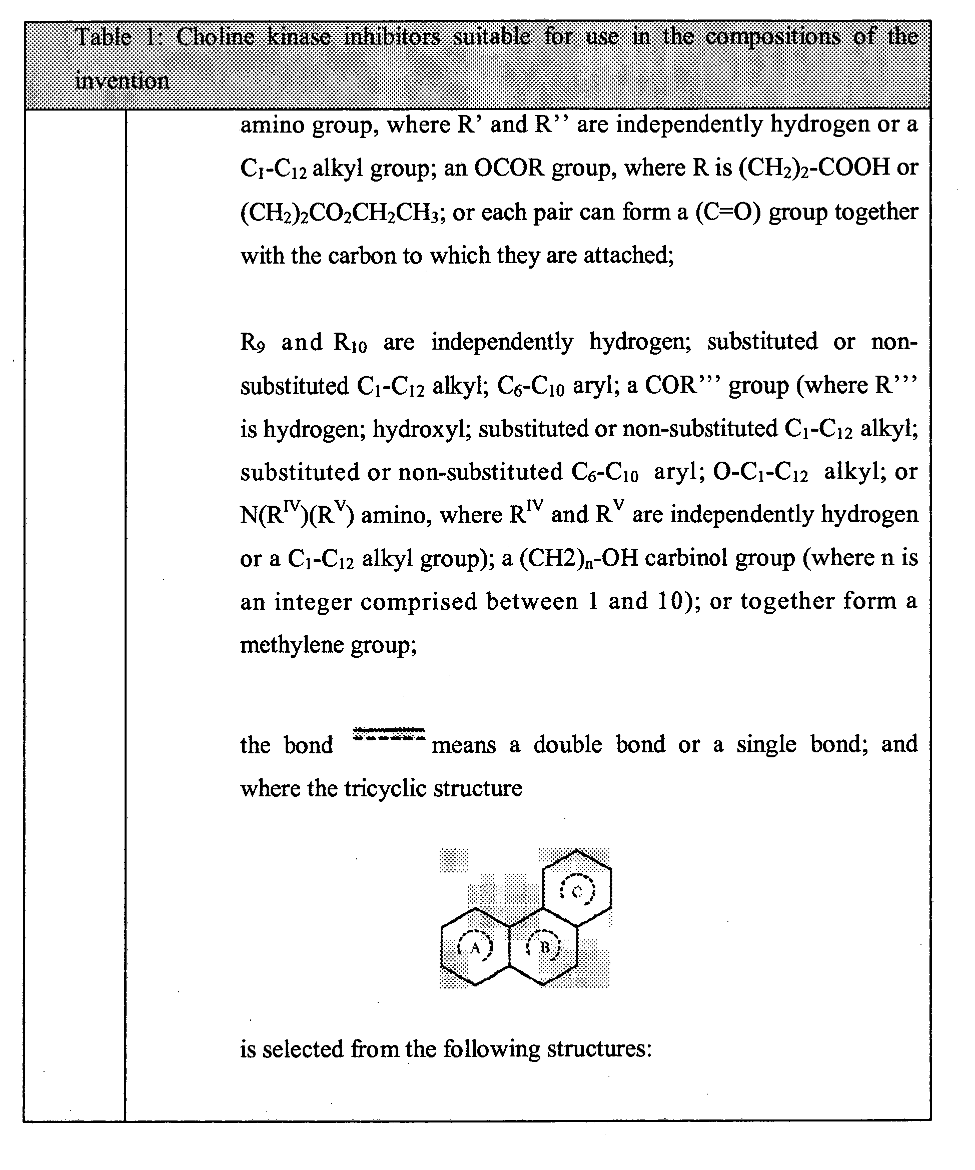

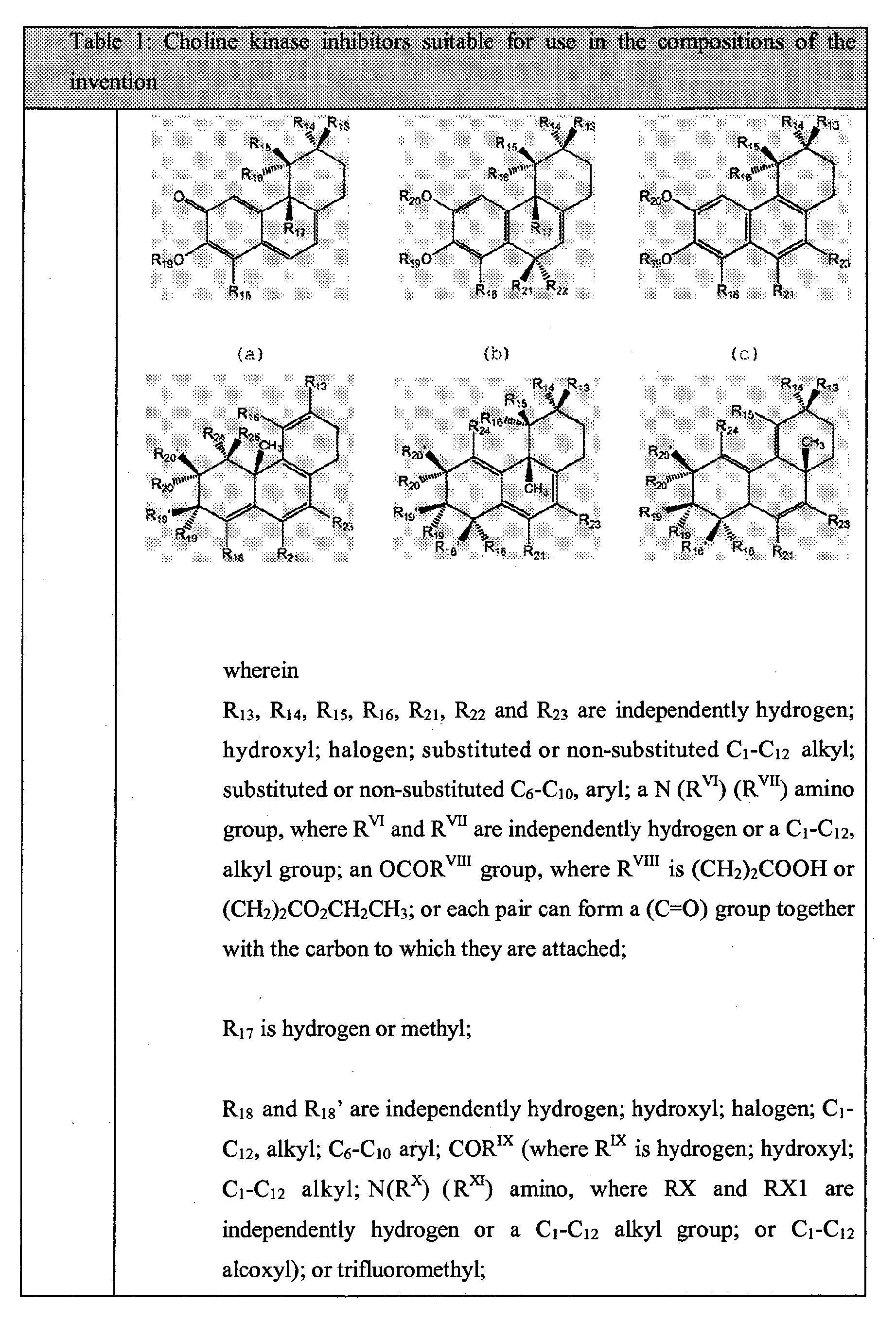

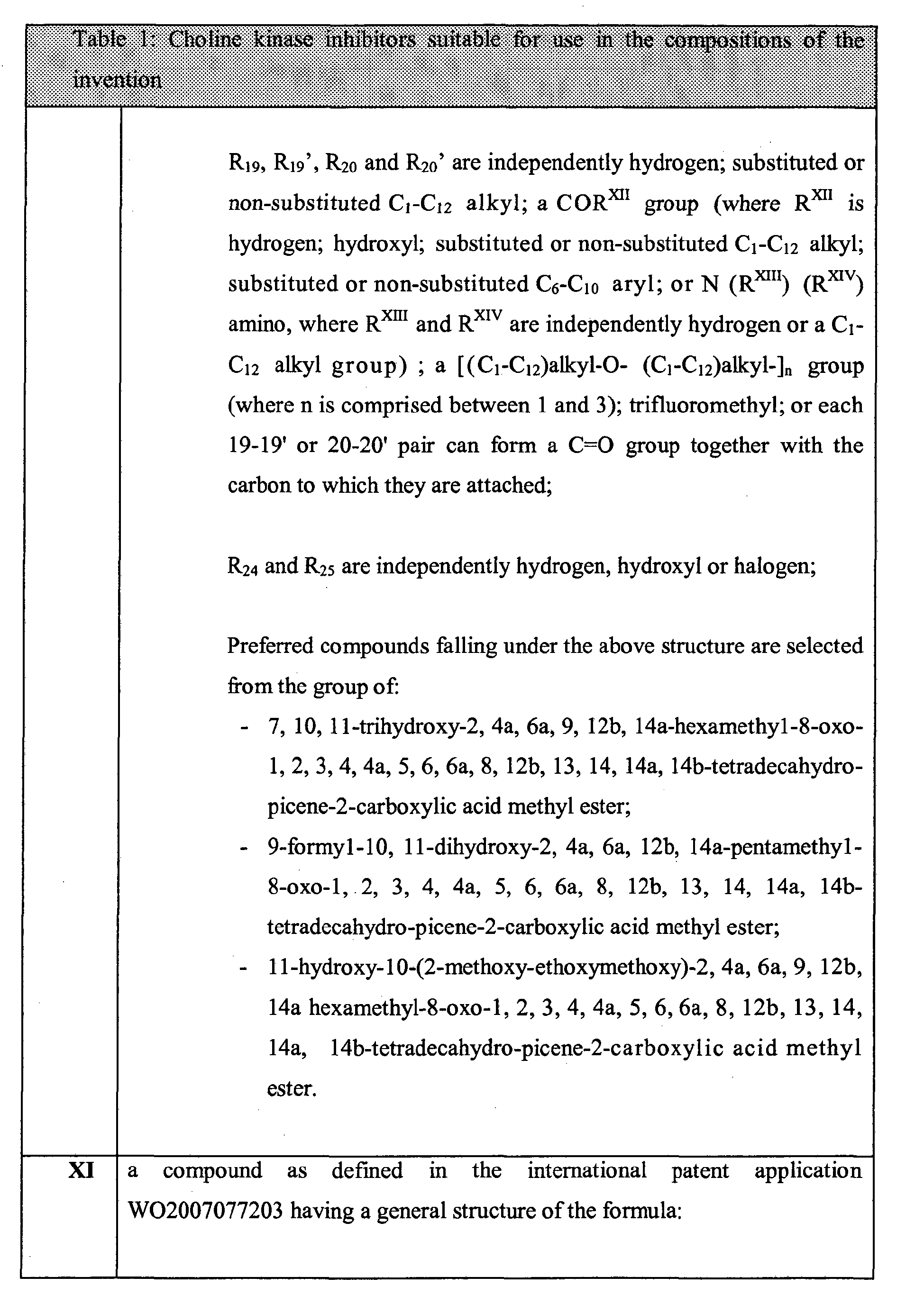

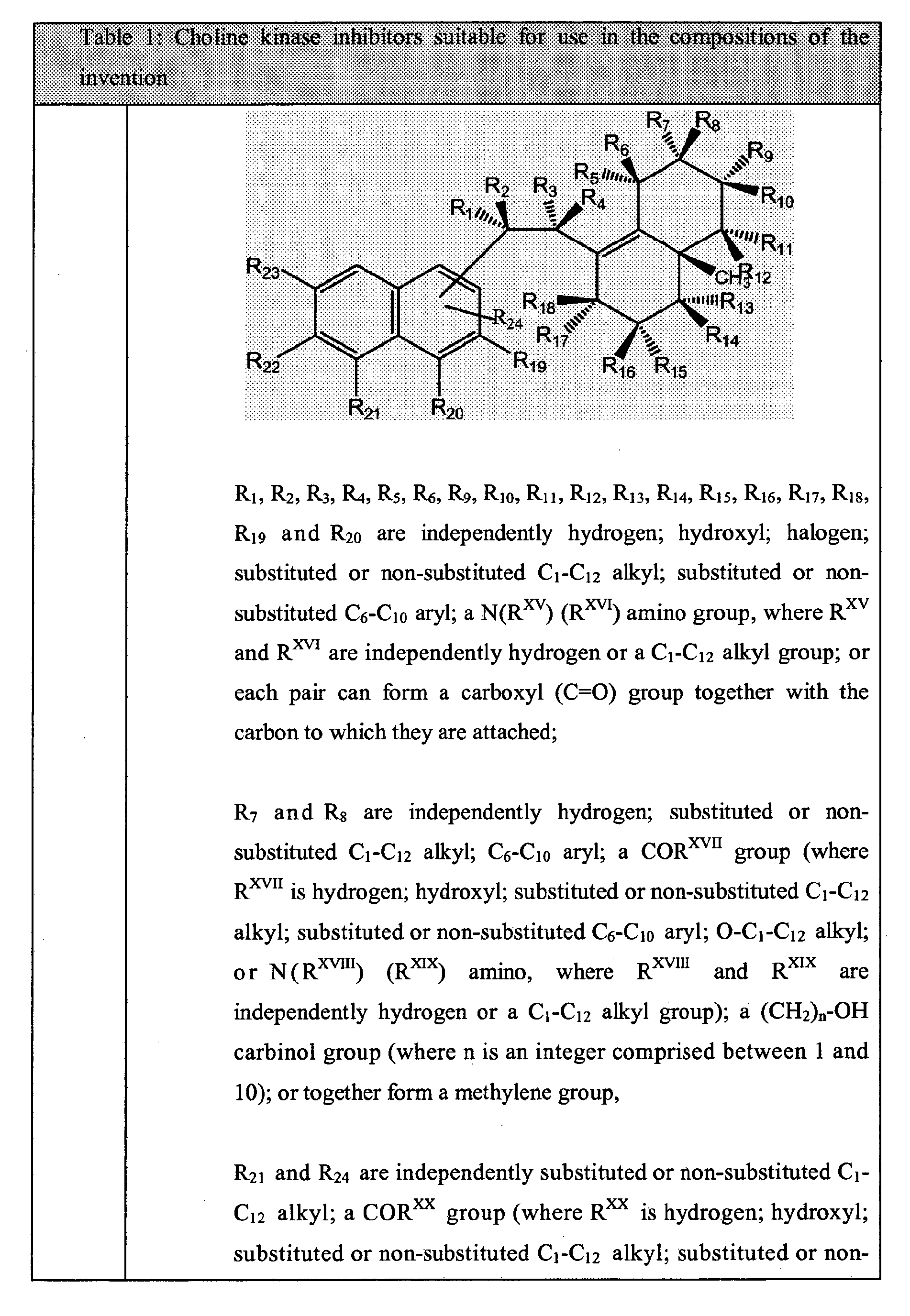

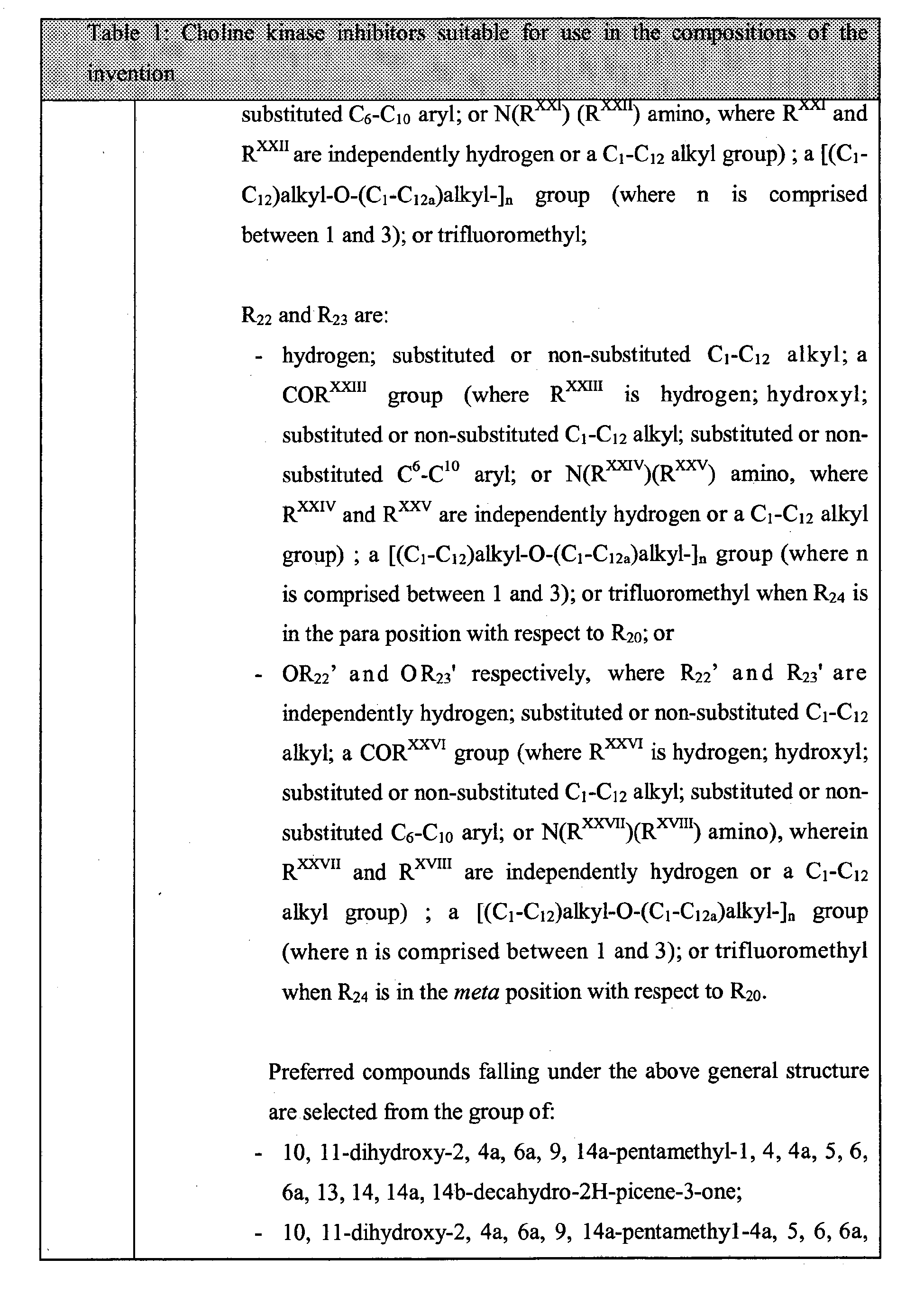

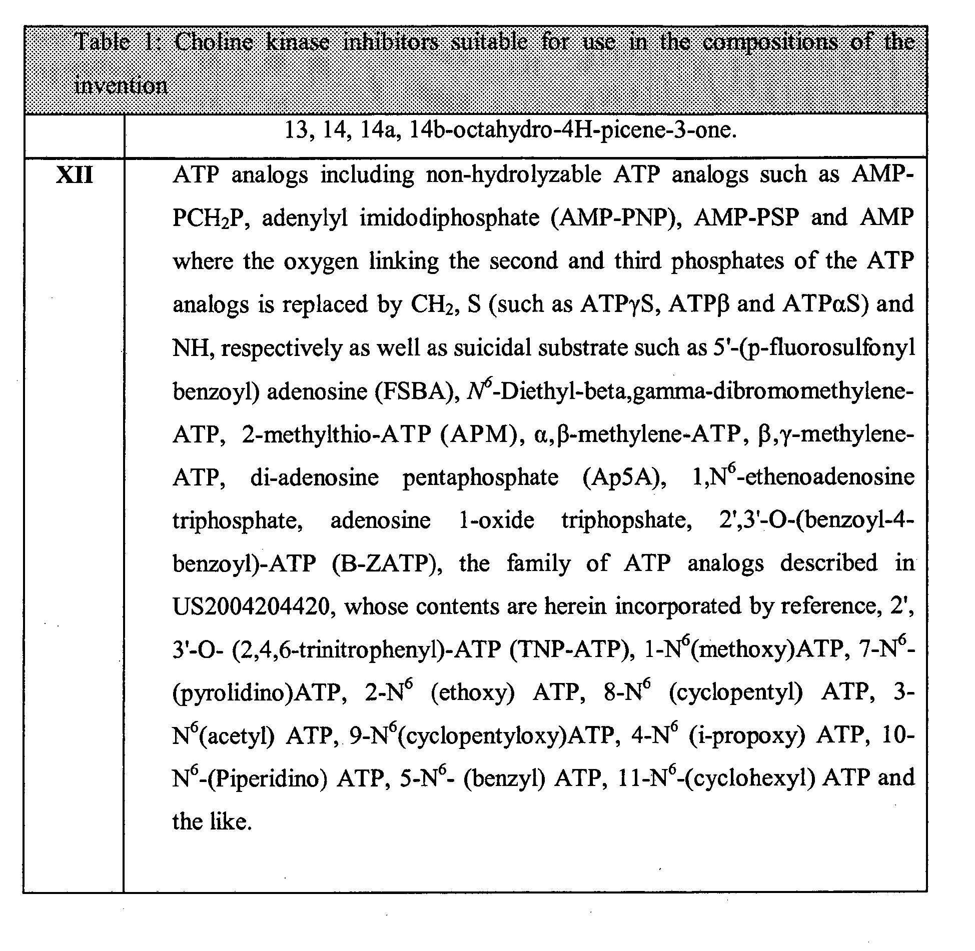

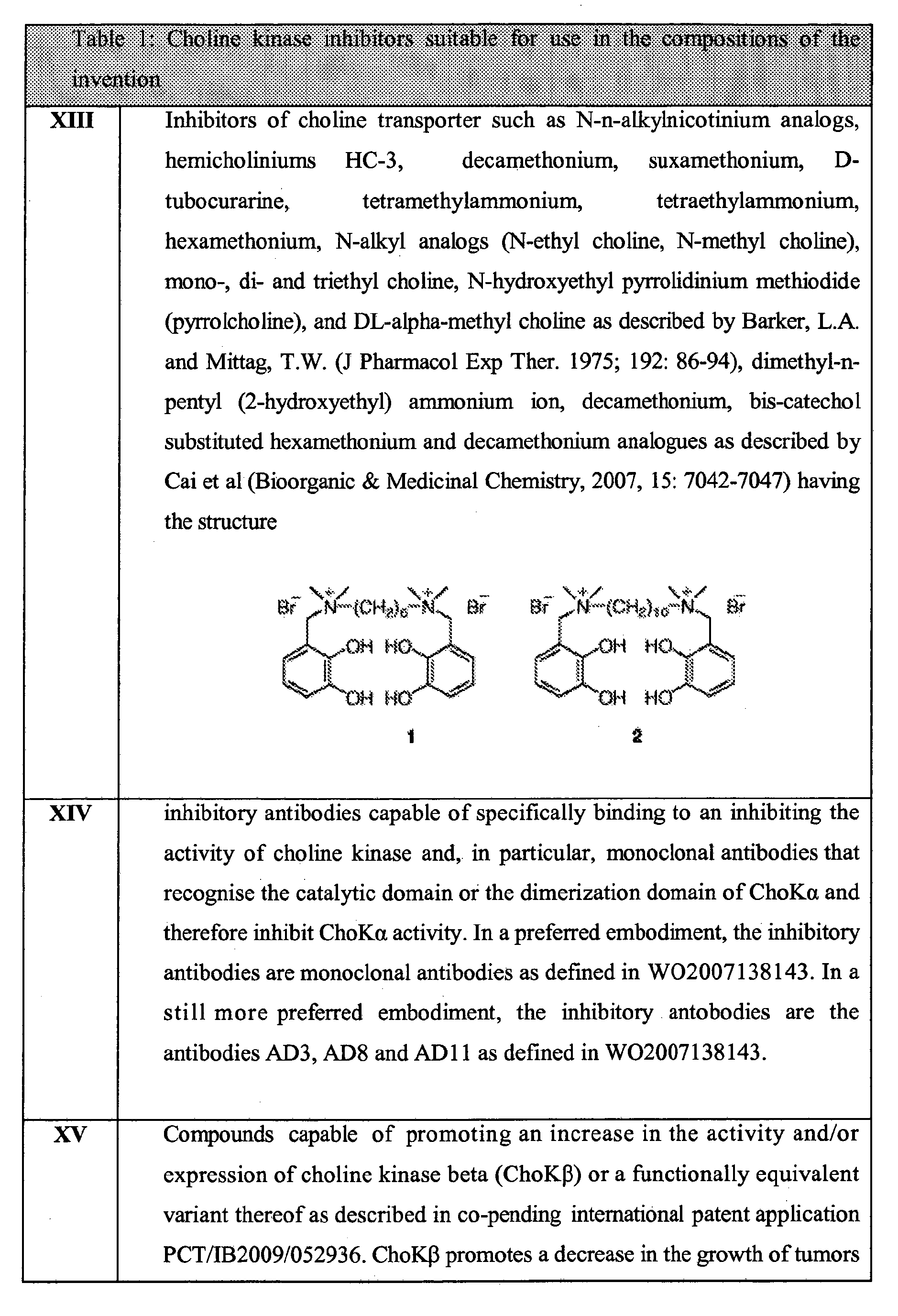

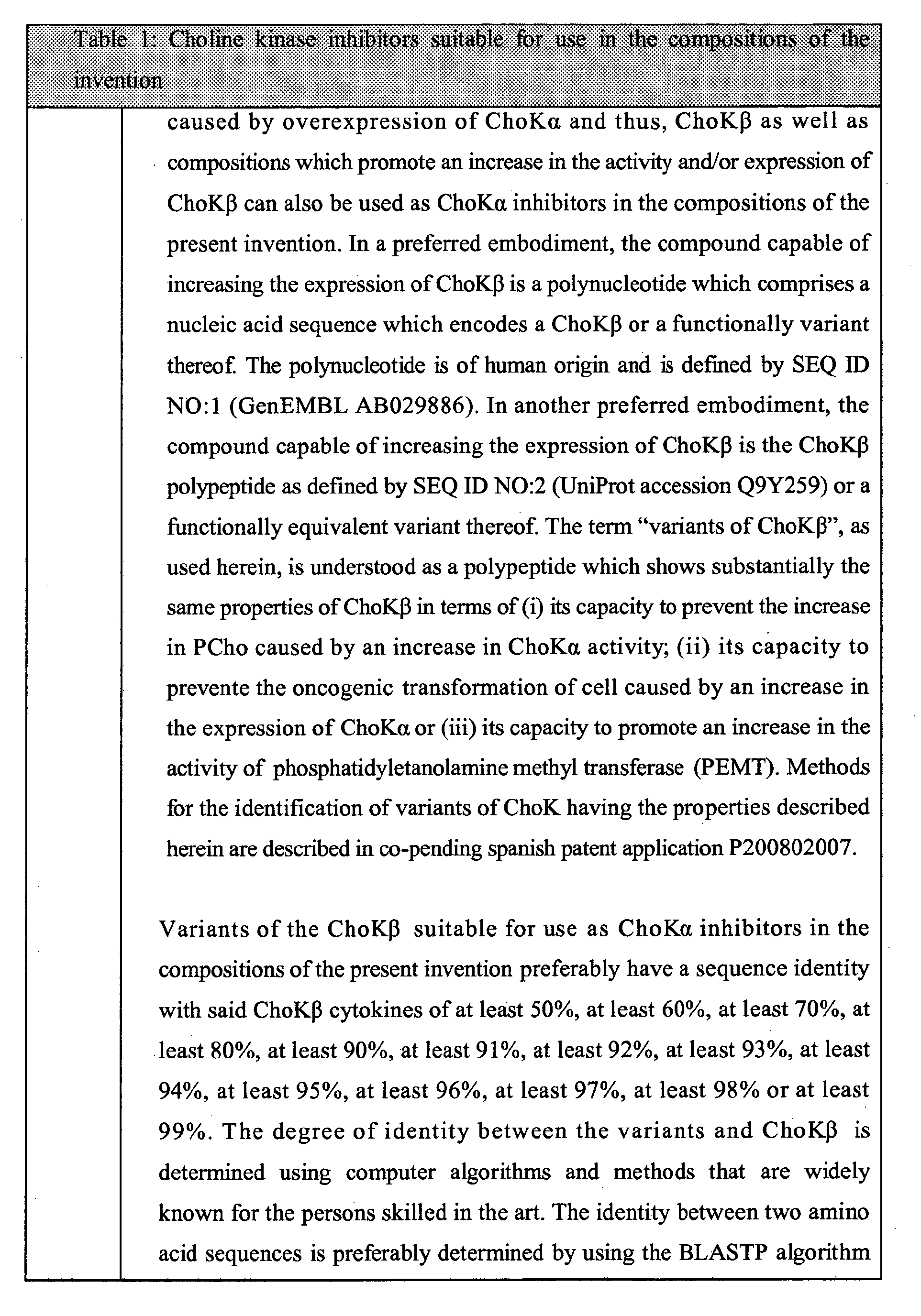

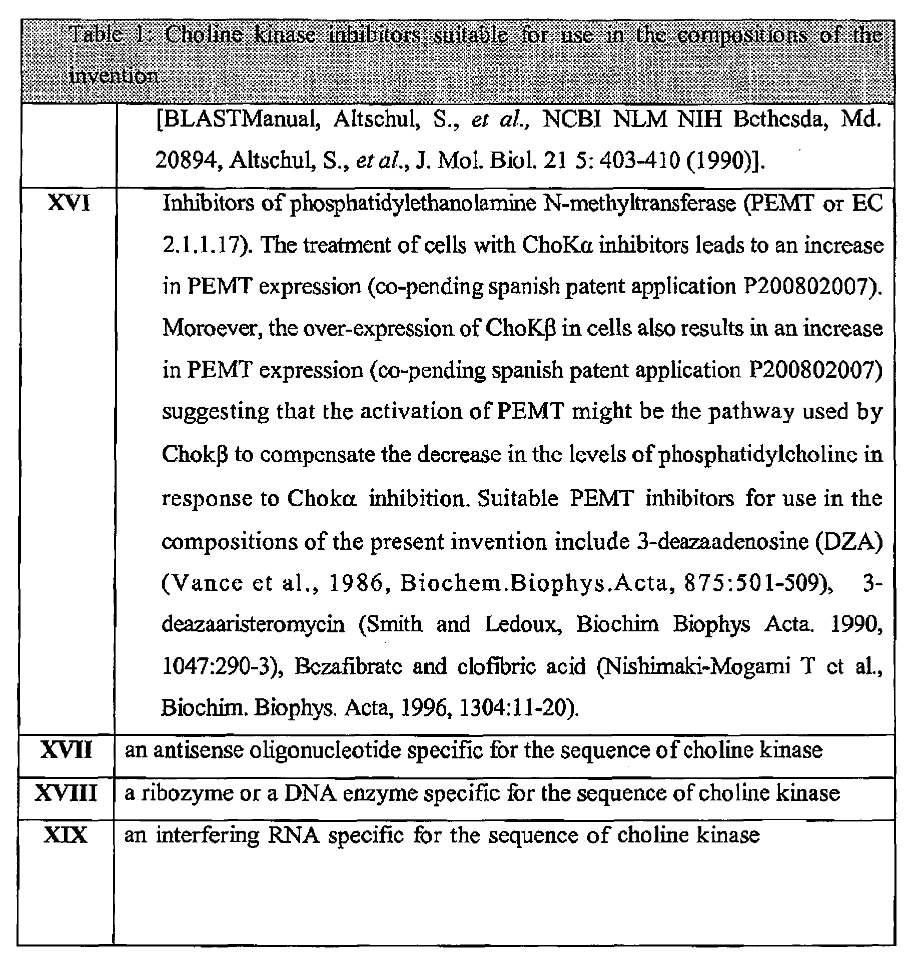

### Acid ceramidase inhibitors

Acid ceramidase inhibitors, as used herein, relates to any compound capable of causing a decrease in the acid ceramidase activity, including those compounds which prevent expression of the acid ceramidase gene, leading to reduced acid ceramidase mRNA or protein levels as well as compounds that bind to the active site of acid ceramidase causing a decrease in the activity of the enzyme.

The expression "Acid ceramidase inhibitors", as used herein, relates to any compound capable of causing a decrease in the acid ceramidase activity, including those compounds which prevent expression of the acid ceramidase gene, leading to reduced acid ceramidase mRNA or protein levels as well as compounds that bind to the active site of acid ceramidase causing a decrease in the activity of the enzyme.

Compounds leading to reduced acid ceramidase mRNA levels can be identified using standard assays for determining mRNA expression levels such as RT-PCR, RNA protection analysis, Northern blot, in situ hybridization, microarray technology and the like.

Compounds leading to reduced acid ceramidase protein levels can be identified using standard assays for determining protein expression levels such as Western-blot or Western transfer, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of protein biochips or microarrays which include specific antibodies or assays based on colloidal precipitation in formats such as dipsticks.

Acid ceramidase inhibitors causing a decrease in the enzymatic activity of acid ceramidase can be identified using standard assays to measure the activity of acid ceramidase using purified acid ceramidase or fractions enriched in acid ceramidase and a substrate thereof. For instance, the method described in ES2233204 which is based on the inhibition of the the hydrolysis of N-(12-(4-nitrobenzene-2-oxa-1,3-diazolo)dodecil) sphingosine (Cer-C12-NBD).

Exemplary non-limiting acid ceramidase inhibitors or acid ceramidase-like inhibitors that can be used in the first composition of the present invention are showun under item I to XIII of Table II

| Acid ceramidase inhibitors suitable for use in the compositions of the present invention | | |
|---|---|---|
| **I** | Sphingoid bases as described in EP1287815 having the following general structural formula | |
| | 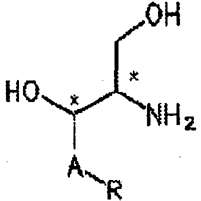 | |
| | wherein | |
| | A is CH₂-CH₂-(R), CH=CH-(R) or C(H)OH-CH₂-(R) or -CH=CH-CHOH-(R) and | |
| | R is a straight chain or branched alkyl group having 10 to 22 carbon atoms which may optionally contain one or more double bonds and/or may optionally be substituted with one or more hydroxyl groups. R is preferably a straight chain alkyl group having 12 to 18 carbon atoms, more preferably a straight chain alkyl group having 13 carbon atoms. Both asymmetric carbon atoms may either take the D or L configuration. | |
| **II** | Cyclopropenylamines variants as described in ES2143403 having the general structural formula | |
| | 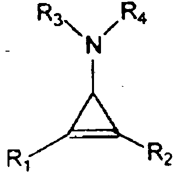 | |
| | wherein | |
| | R₁ and R₂ may be the same or different and correspond to linear or branched alkyl, or phenylalkyl groups of 1 to 18 carbon atoms having from 0 to several insaturations and substituents (X) at the end of the group wherein X is -OH, -OR wherein R is linear or branched C₁-C₅ alkyl or metiloxialkyl, -CO₂H, - CO2R wherein R is as defined previously, -CON(R)₂ wherein R is as defined previously and halogen (F, Cl, Br, I) and R₃ and R₄ are alkyl, alkenyl, aryl, which are the same or different or may form part of an aromatic ring or a fthlamide-type ring. | |
| | | |
| | In a preferred embodiment, the compound falling under the above general formula suitable for use in the present invention is N-(1-pentyl-2-butyl-3-cyclopro-fenil)ftalamide | |
| **III** | Cyclopropenylsphingosine derivatives as described in ES2233204 having the general structural formula | |
| | 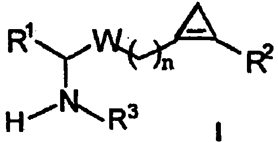 | |
| | | wherein |
| | n | is a whole number that may represent any value |
| | W | es -CH₂-, -CH(OH)-, -C(=O)-, -C(=NOH)-, -C(=N-H₂)-, -C(=S)-,-CH(SH)- |
| | R¹ | is selected from the group of: |
| | | (a) H |
| | | (b) a -CH₃, -CH₂OH, -CH₂SH, -CH₂-NH₂, -CH₂N₃, -CH₂-NH-OH, -CH=N-OH, -CH=N-NH₂, -C(=O)H,-C(=O)CH3, -C(=O)CF3, -C(=O)NH₂, -SCH₃, -OCH₃,-CH₂O-P(=O)(OH)₂ or -CH₂CH₂-P(=O) residue and |
| | | (c) a -(CH2)nR4, -C(OH)R4, -C(=O)R4, -C(SH)R4,-C(=S)R4, - C(=N)R4 or -C(NH)R4 wherein n is 0 or 1, R⁴ is linear or branched alkyl, alkenyl, alkinyl, an aryl containing or not heteroatoms and containing substituents in any position or an heterocycle that may be substituted in one or more position |
| | R² | is selected from the group of: |
| | | (a) H |
| | | (b)a linear or branched alkyl, alkenyl, alkinyl, an aryl containing or not heteroatoms and containing substituents in any position or an heterocycle that may be substituted in one or more positions |
| | R³ | is selected from the group of |
| | | (a) H |
| | | (b) a linear or branched alkyl, alkenyl, alkinyl, an aryl containing or not heteroatoms and containing substituents in any position or an heterocycle that may be substituted in one or more position |
| | | (c) a -(C=X)-Y-R₅ or a -(C=X)-R₅ wherein X is O, S, N, Y is O, S, -NH or -CHOH and R₅ is H or a linear or branched alkyl, alkenyl, alkinyl, an aryl containing or not heteroatoms and containing substituents in any position or an heterocycle that may be substituted in one or more position. |
| | | Preferred compounds having the above general structure are compounds GT54, GT45, GT76, GT77, GT85, GT99 and GT98 as defined in Bedia et al. (ChemBioChem., 2007, 8:642-648) corresponding to |
| | 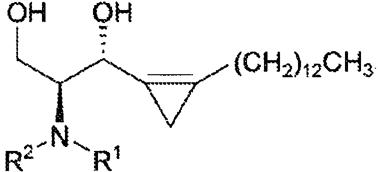 | |
| | | GT54: R¹=CH₃; R²=CO(CH₂)₆CH₃ |
| | | GT45: R¹=H; R²=COO(CH₂)₇CH₃ |
| | | GT76: R¹=H; R²=CONH(CH₂)₇CH₃ |
| | | GT77: R¹=H; R²=CSNH(CH₂)₇CH₃ |
| | | GT85: R¹⁼H; R²=COCOCH₂CH₃ |
| | | GT99: R¹=H; R²=COCO(CH₂)₅CH₃ |
| | | GT98: R¹⁼H; R²=COCOC₆H₅ |
| | | and compounds GT11, GT1, GT2, GT3, GT4, GT5, GT6 and GT7 as escribed by Bedia et al (Org. Biomol.Chem., 2005, 3:3707-3712) having the following structures |
| | **GT11:** | R¹=H, R²=CO(CH₂)₆CH₃ |
| | | **1:** R¹=CH₃, R²=CO(CH₂)₆CH₃ |
| | | **2:** R¹=H, R²=COO(CH₂)₇CH₃ |
| | | **3:** R¹=H, R²=CONH(CH₂)₇CH₃ |
| | | **4:** R¹=H, R²=CSNH(CH₂)₇CH₃ |
| | | **5:** R¹=H, R²=COCOCH₂CH₃ |
| | | **6:** R¹=H, R²=COCO(CH₂)₅CH₃ |
| | | **7:** R¹=H, R²=COCOC₆H₅ |
| **IV** | A compound as described in ES2273560 having the following general formula | |
| | 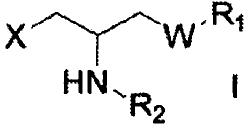 | |
| | | wherein |
| | W | is -O-, -S-, -S(=O), -S(=O)₂- |
| | X | is selected from |
| | (a) | OH |
| | (b) | a -OP(=O)-(OR³)2 wherein R³ may be the same or different and corresponds to H, CH₃ or CH₂CH₃. |
| | (c) | a -CH₂P(=O)-(OR³)₂ wherein R³ may be the same or different and corresponds to H, CH₃ or CH₂CH₃. |
| | R¹ | is a linear or branched alkyl, alkenyl, alkinyl, an aryl containing or not heteroatoms and containing substituents in any position or an heterocycle that may be substituted in one or more position |
| | R² | is selected from a group of |
| | (a) | H |
| | (b) | a linear or branched alkyl, alkenyl, alkinyl, an aryl containing or not heteroatoms and containing substituents in any position or an heterocycle that may be substituted in one or more position and |
| | (c) | a -(C=X)-Y-R₅ or a -(C=X)ₙ-R₅ wherein n is 0 or 1, X is O, S, N; Y is O, S, -NH or -CHOH or -CHZ wherein Z is an halogen and R₅ is H or a linear or branched alkyl, alkenyl, alkinyl, an aryl containing or not heteroatoms and containing substituents in any position or an heterocycle that may be substituted in one or more position. |
| | | Preferred compounds having the above general structure are compunds GT102, GT103 and GT104 as described by Bedia et al. (ChemBioChem., 2007, 8:642-648) corresponding to: |
| | 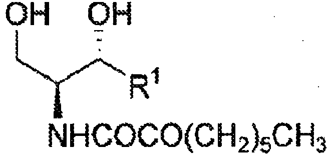 | |
| | 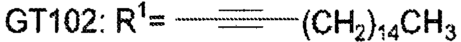 | |
| | 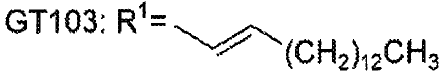 | |
| | 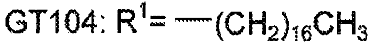 | |
| **V** | A compound as defined in WO2007136635 having the following general structural formula | |
| | 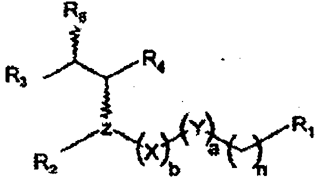 | |
| | wherein | |
| | R₁ is H, OH, SH, NH₂, Cl, Br, I, COOH, CONH₂, NH(C=NH)NH₂, NHR₂, N(R₂)₂, ⁺N(R₂)₃, or N-heterocycle having from 5 to 6 atoms in the ring; | |
| | R₂ is H or C₁-C₆ alkyl; | |
| | R₃ is phenyl, optionally substituted with one or more R₅; five-membered monocyclic heterocycle; six-membered monocyclic heterocycle; five-and five- membered bicyclic heterocycle; six and six membered bicyclic heterocycle; five-and six-membered bicyclic heterocycle; five-, five-, and five-membered tricylic heterocycle; six-, six-, and six membered tricylic heterocycle; five-, five- , and six-membered tricylic heterocycle; five-, six-, and six-membered tricylic heterocycle; six-, five-, and six-membered tricylic heterocycle; five-, six-, and five-membered tricylic heterocycle; each of the foregoing being optionally substituted with one or more R₅, wherein R₅ is C₁-C₆ alkyl, F, Cl, Br, I, NHR₂, NO₂, or an amide of formula | |
| | 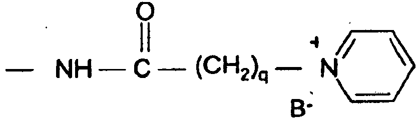 | |
| | wherein R₂ is as defined above | |
| | R₄ is H, C₁-C₆ alkyl, CH₂OH, SH, NH₂, CH₂Cl, CH₂Br, CH₂I, COOH, CONH₂; | |
| | R₆ is H or OH; | |
| | X is CH₂, CHC₁-C₆ alkyl, CO, or CS; | |
| | Y is CH₂, CO, NH, O, or C(OH); | |
| | Z is N or ⁺NH; | |
| | A is 0 or 1; | |
| | B is 0 or 1; | |
| | n is an integer from 2 to 22; | |
| | q is an integer from 2 to 18; and | |
| | | B⁻ is a pharmaceutically acceptable counter-anion |
| | Preferred compounds falling under the above general formula include the B13 and (1S,2R)-N-myristoylamino-phenylpropanol-1 (D-e-MAPP) variants as described by Bielawska et al (Bioorg. Med. Chem., 2008, 16:1032-1034) and Szulc et al (Bioorg. Med. Chem., 2008, 16:1015-1031) having the structures | |
| | 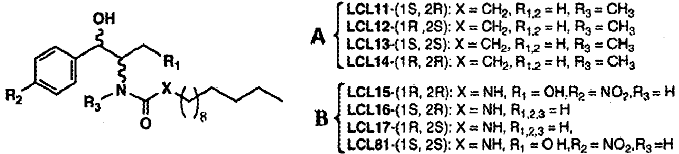 | |
| | 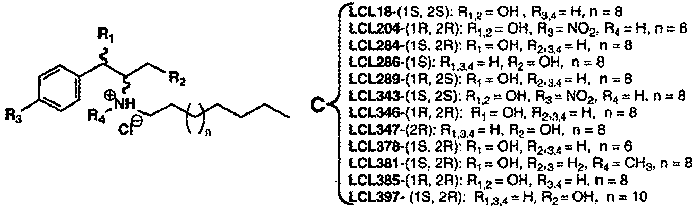 | |
| | 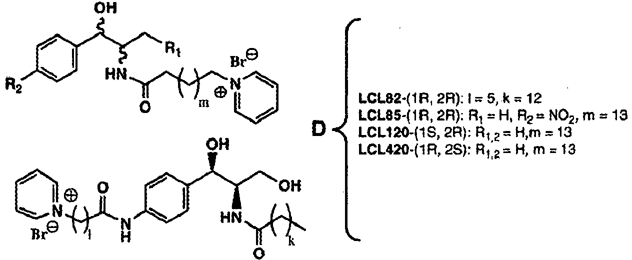 | |
| **VI** | N-oleoylethanolamine (NOE) as described by Sugita et al (Biochim.Biphys.Acta, 1975, 398:125-131) having the following structure | |
| | 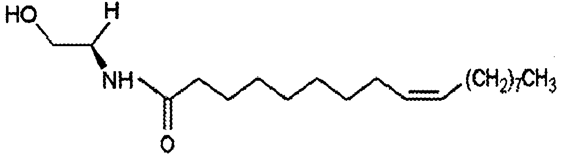 | |
| | and variants thereof as described by Grijalvo et al., (Chem.Phys.Lipids, 2006, 144:69-84) having the following general structure | |
| | 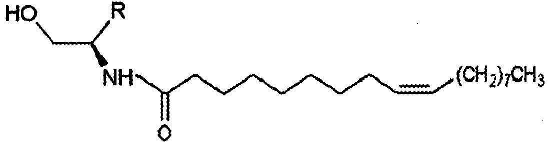 | |
| | wherein R is selected from the group of | |
| | **1;**R = CH₂SCH₂CH₃ | **16;**R = CH₂SCH₂CH₃ |
| | **2;**R = CH₂S(CH₂)₄CH₃ | **17;**R = CH₂S(CH₂)₄CH₃ |
| | **3;**R = CH₂S(CH₂)₉CH₃ | **18;**R = CH₂S(CH₂)₉CH₃ |
| | **4;**R = CH₂S(CH₂)₁₅CH₃ | **19;**R = CH₂S(CH₂)₁₅CH₃ |
| | 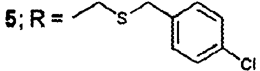 | 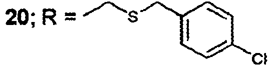 |
| | 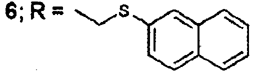 | 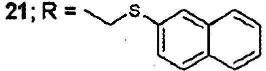 |
| | 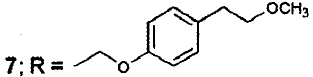 | 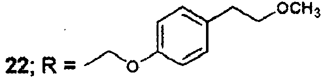 |
| | 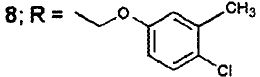 | 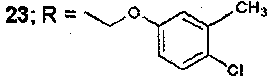 |
| | 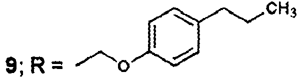 | 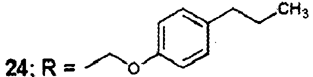 |
| | 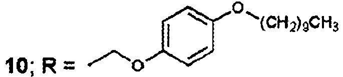 | 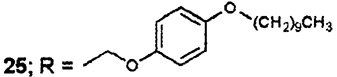 |
| | 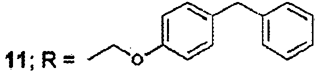 | |
| | 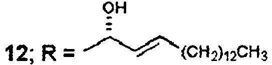 | |
| | 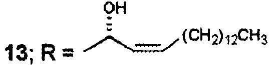 | |
| | 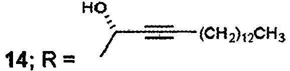 | |
| | **15;**R = -(CH₂)₁₅CH₃ | |
| **VII** | 2-oxoctanamides as described by Grijalvo et al., (Chem.Phys.Lipids, 2006, 144:69-84) having the general structure | |
| | 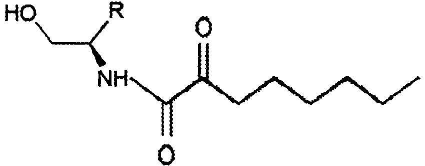 | |
| | wherein R is selected from the group of | |
| | **1;**R = CH₂SCH₂CH₃ | **16;**R = CH₂SCH₂CH₃ |
| | **2;**R = CH₂S(CH₂)₄CH₃ | **17;**R = CH₂S(CH₂)₄CH₃ |
| | **3;**R = CH₂S(CH₂)₉CH₃ | **18;**R = CH₂S(CH₂)₉CH₃ |
| | **4;**R = CH₂S(CH₂)₁₅CH₃ | **19;**R = CH₂S(CH₂)₁₅CH₃ |
| | 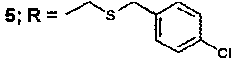 | 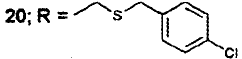 |
| | 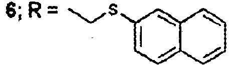 | 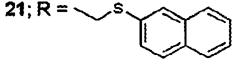 |
| | 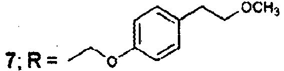 | 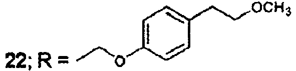 |
| | 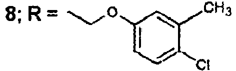 | 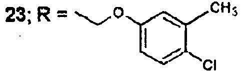 |
| | 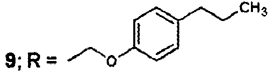 | 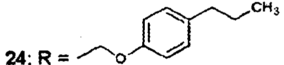 |
| | 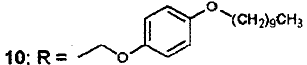 | 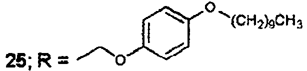 |
| | 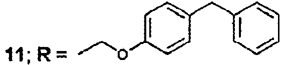 | |
| | 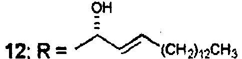 | |
| | 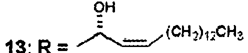 | |
| | 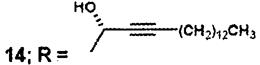 | |
| | **15;**R = -(CH₂)₁₅CH₃ | |
| **VIII** | (1R,2R)-N-myristoylamino-4'-nitro-phenylpropandiol-1, 3 (B13) (D-NMAPPD) as described by Selzner et al (Cancer Res., 2001, 61:1233-1240) having the structure | |
| | 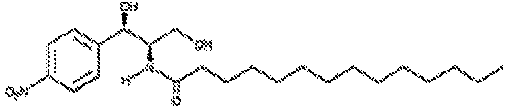 | |
| | and analogs thereof | |
| **IX** | desipramine having the structure | |
| | | 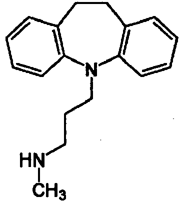 |
| **X** | inhibitory antibodies capable of specifically binding and inhibiting the activity of acid ceramidase or the acid ceramidase-like , | |
| **XI** | an antisense oligonucleotide specific for the sequence of acid ceramidase or the acid ceramidase-like | |
| **XII** | a ribozyme or a DNA enzyme specific for the sequence of acid ceramidase or the acid ceramidase-like | |
| **XIII** | an interfering RNA specific for the sequence of acid ceramidase or of acid ceramidase-like such as those described in Morales et al (Oncogene, 2007, 26:905-916) (SEQ ID NO:5), used for sensitizing hepatoma cells to treatment with daunorubicin or in WO2007136635 (SEQ ID NO:6), used for the sensitization of head and neck cancer cell lines to the treatment with FasL gene therapy | |

### Acid ceramidase- or choline kinase-specific inhibitory antibodies

Antibodies against an epitope located in either acid ceramidase or in choline kinase alpha isoform may effectively block the function of these proteins and, therefore, can be used as inhibitors in the compositions of the present invention. "Inhibitory antibody", as used herein, refers to antibodies which are capable of inhibiting at least partially the biological activities of acid ceramidase or of choline kinase alpha isoform actity.

The determination of the inhibitory capacity on the biological activity of acid ceramidase is detected using standard assays to measure the activity of acid ceramidase using purified acid ceramidase or fractions enriched in acid ceramidase such as the methods based on the capacity of the antibody of inhibiting the hydrolysis of N-(12-(4-nitrobenzene-2-oxa-1,3-diazolo)dodecil) sphingosine (Cer-C12-NBD) as described e.g. in ES2233204.

The determination of the inhibitory capacity on the biological activity of choline kinase is detected using standard assays to measure the activity of choline kinase such as the methods based on the detection of the phosphorylation of [¹⁴C] labelled choline by ATP in the presence of purified recombinant choline kinase or a fraction enriched in choline kinase followed by detection of the phosphorylated choline using standard analytical techniques (e.g. TLC) as described in EP1710236.

Inhibitory antibodies or fragments specific for choline kinase alpha isoform or acid ceramidase may be readily available, or may be readily produced using conventional molecular biology techniques. For example, using immunogens derived from, for example, acid ceramidase or choline kinase alpha isoform it is possible to obtain anti-protein/anti-peptide antisera or monoclonal antibodies by using standard protocols (See, for example, Antibodies: A Laboratory Manual ed. by Harlow and Lane (Cold Spring Harbor Press: 1988)). A mammal, such as a mouse, a hamster or rabbit can be immunized with an immunogenic form of the peptide (e.g., acid ceramidase or choline kinase alpha isoform or an antigenic fragment thereof, which is capable of eliciting an antibody response). Techniques for conferring immunogenicity on a protein or peptide, include conjugation to carriers or other techniques, are well known in the art. An immunogenic portion of a polypeptide can be administered in the presence of adjuvant. The progress of immunization can be monitored by detection of antibody titers in plasma or serum. Standard ELISA or other immunoassays can be used with the immunogen as antigen to assess the levels of antibodies. In a preferred embodiment, the antibodies forming part of the compositions of the invention are immuno-specific for antigenic determinants of acid ceramidase or choline kinase alpha isoform (or a variant at least 80%, 85%, 90%, 95%, or 98% identical thereto). In certain embodiment, the immunospecific subject antibodies do not substantially cross react with a non-vertebrate (such as yeast) acid ceramidase or choline kinase-related protein. By "not substantially cross react," it is meant that the antibody has a binding affinity for a non-homologous protein which is at least one order of magnitude, more preferably at least 2 orders of magnitude, and even more preferably at least 3 orders of magnitude less than the binding affinity of the antibody for acid ceramidase or choline kinase.

Thus, the antibody of the invention is capable of binding an epitope of the choline kinase alpha isoform or of acid ceramidase; typically, at least 6, 8, 10, or 12, contiguous amino acids are required to form an epitope, however, epitopes which involve non-contiguous amino acids may require more, e.g., at least 15, 25, or 50 amino acid. The term "antibody of the invention" includes, for example, polyclonal antibodies, monoclonal antibodies, Fab and single chain Fv (scFv) fragments thereof, bispecific antibodies, heteroconjugates, human and humanized antibodies. Such antibodies may be produced in a variety of ways, including hybridoma cultures, recombinant expression in bacteria or mammalian cell cultures, and recombinant expression in transgenic animals. Also antibodies can be produced by selecting a sequence from a library of sequences expressed in display systems such as filamentous phage, bacterial, yeast or ribosome. There is abundant guidance in the literature for selecting a particular production methodology, e.g., Chadd and Chamow, Curr. Opin. BiotechnoL, 12:188-194 (2001). The choice of manufacturing methodology depends on several factors including the antibody structure desired, the importance of carbohydrate moieties on the antibodies, ease of culturing and purification, and cost. Many different antibody structures may be generated using standard expression technology, including full-length antibodies, antibody fragments, such as Fab and Fv fragments, as well as chimeric antibodies comprising components from different species. Antibody fragments of small size, such as Fab and Fv fragments, having no effector functions and limited pharmokinetic activity may be generated in a bacterial expression system. Single chain Fv fragments show low immunogenicity and are cleared rapidly from the blood.

The antibodies of the invention may be polyclonal antibodies. Such polyclonal antibodies can be produced in a mammal, such as a non-human mammal, for example, following one or more injections of an immunizing agent, and preferably, an adjuvant. Typically, the immunizing agent and/or adjuvant will be injected into the mammal by a series of subcutaneous or intraperitoneal injections. The immunizing agent may include choline kinase alpha isoform or acid ceramidase or fragments thereof or a fusion protein thereof or a cell expressing either choline kinase or acid ceramidase. Such proteins, fragments or preparations are introduced into the non-human mammal in the presence of an appropriate adjuvant. Other form of administration of an immunogen is as a trasmembrane protein in the surface of a cell (methods described in, e.g., Spiller et al. J. Immunol. Methods, 224: 51-60 (1999)). These cells can be either cells which naturally express the antigen or in which this expression can be obtained after transfecting the cell with a DNA construct that contains among other DNA sequences those coding the antigen, those necessary for its sufficient expression in the cell. This approach is possible not only when the cell membrane is the natural site in which the antigen is expressed even the antigen once synthesized in the cell is directed at these location by a signal peptide which is added at the antigen coding sequence. If the serum contains polyclonal antibodies to undesired epitopes, the polyclonal antibodies can be purified by immunoaffinity chromatography.

Alternatively, said antibodies may be monoclonal antibodies. Monoclonal antibodies may be produced by hybridomas, wherein a mouse, hamster, or other appropriate host animal, is immunized with an immunizing agent to elicit lymphocytes that produce or are capable of producing antibodies that will specifically bind to the immunizing agent, e.g. Kohler and Milstein, Nature 256:495 (1975). The immunizing agent will typically include a choline kinase alpha isoform, acid ceramidase or a receptor or a fragment thereof or a fusion protein thereof and optionally a carrier or a crude protein preparation which has been enriched for a choline kinase alpha isoform or acid ceramidase or a cell expressing any of said proteins. Such proteins, fragments or preparations are introduced into the non-human mammal in the presence of an appropriate adjuvant. Other form of administration of an immunogen is as a trasmembrane protein in the surface of a cell (methods described in, e.g., Spiller et al. J. Immunol. Methods, 224: 51-60 (1999)). These cells can be everyone which naturally express the antigen in its cell membrane or in which this expression can be obtained after transfecting the cell with a DNA construct that contains among other DNA sequences those coding the antigen, those necessary for its sufficient expression in the cell. This approach is possible not only when the cell membrane is the natural site in which the antigen is expressed even the antigen once synthesized in the cell is directed at these location by a signal peptide which is added at the antigen coding sequence. Alternatively, lymphocytes may be immunized *in vitro.* Generally, spleen cells or lymph node cells are used if non-human mammalian sources are desired, or peripheral blood lymphocytes ("PBLs") are used if cells of human origin are desired. The lymphocytes are fused with an immortalized cell line using a suitable fusing agent, such as polyethylene glycol, to produce a hybridoma cell. In general, immortalized cell lines are myeloma cells of rat, mouse, bovine or human origin. The hybridoma cells are cultured in a suitable culture medium that preferably contains one or more substances that inhibit the growth or survival of unfused, immortalized cells. Clones are isolated using the limiting dilution method and the culture medium (supernatant) in which the hybridoma cells are cultured can be assayed for the presence of monoclonal antibodies directed against ChoK alpha isoform by conventional techniques, such as by flow cytometry or by immunoprecipitation or by other *in vitro* binding assay, such as RIA or ELISA. Clones can also be cultured *in vivo* as ascites tumours in an animal.

Preferably, the binding specificity of monoclonal antibodies produced by a clone of hybridoma cells is determined by immunoprecipitation or by an in *vitro* binding assay, such as radioimmunoassay (RIA) or enzyme-linked immunoabsorbent assay (ELISA) or by immunofluorescent techniques such as fluorescence microscopy or flow cytometry. The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in US 4,816,567. DNA encoding the monoclonal antibodies of the invention can be isolated from the choline kinase alpha isoform or acid ceramidase receptor-specific hybridoma cells and sequenced by using conventional procedures, e.g., by using oligonucleotide probes that are capable of binding specifically to genes encoding the heavy and light chains of murine antibodies. The hybridoma cells serve as a preferred source of such DNA. Once isolated, the DNA may be inserted into an expression vector, which is then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for the murine heavy and light chain constant domains for the homologous human sequences, or by covalently joining to the immunoglobulin coding sequence all or part of the coding sequence for a non-immunoglobulin polypeptide. The non-immunoglobulin polypeptide can be substituted for the constant domains of an antibody of the invention, or can be substituted for the variable domains of one antigen-combining site of an antibody of the invention to create a chimeric bivalent antibody.

Another method of generating specific antibodies, or antibody fragments, reactive against a target molecule is to screen expression libraries encoding immunoglobulin genes, or portions thereof, expressed in bacteria, yeast, filamentous phages, ribosomes or ribosomal subunits and other display systems. These methods normally use large libraries of antibody sequences or antibody fragment sequences obtained from diverse sources such healthy donors or patients or animals healthy or not. These sequences are cloned and expressed in an appropriate system and selected by its binding affinity for the antigen. Diverse approaches have been described to select antibodies or fragments with desired properties e.g. neutralizing, agonist, etc (Fernández, Curr. Op. Biotech., 15: 364-373 (2004); Schmidt, Eur. J. Biochem., 268: 1730-1738 (2001)). In an embodiment, antibodies and antibody fragments characteristic of hybridomas of the invention can also be produced by recombinant means by extracting messenger RNA, constructing a cDNA library, and selecting clones which encode segments of the antibody molecule.

The antibodies may also be engineered to alter its clinical uses. Numerous approaches make use of the molecular biology and genetic techniques such as the good knowledge of the genetics ad structure of the immunoglobulins to construct different modifications of immunoglobulin molecule with the aim of improve its properties for clinical or other uses. Some of them tend to reduce the immunogenicity of the molecule in the species in which should be used and the resultant molecule has a sequence more homologous with this species. Various methods have been used to obtain mAbs of human origin avoiding the non ethically admissible proceedings in healthy humans. In other approaches the molecular weigh an size are reduced e.g. in order of improving the distribution of the molecule into solid tumours. Other possibilities are conjugation in a molecule of binding domains for more than one target molecule (bispecific antibody or also triespecific, etc) or the conjugation of an antibody or a fragment with another molecule with the desired function e.g. a toxic agent, a hormone, growth factor, a immunomodulating agent (immunosuppressor or immunostimulator), an inhibitor of cell growth, etc. In general all the resultant molecules retain almost one variable domain of an antibody which gives the high specificity and affinity characteristic of the antigen-antibody binding. Some examples of these constructions are:
- Chimeric antibodies:
   These refers to antibodies constructed with variable regions from an antibody of some species (normally a mammal in which the mAb was generated) and constant regions of other species (the one in which the chimeric antibody is to be used). The objective of such construction is to obtain an antibody with the original mAb but less immunogenic and better tolerated in the subject to be treated, with improved serum half-life and which can be recognized for effector immunological mechanisms i.e. complement, Fc receptor of cytotoxic cells or others specific receptor for immuglobulins that show species specifity.
- Humanized antibodies:
   By "humanized antibody" is meant an antibody derived from a non-human antibody, typically a murine antibody, that retains the antigen-binding properties of the parent antibody, but which is less immunogenic in humans. This may be achieved by various methods, including (a) grafting the entire non-human variable domains onto human constant regions to generate chimeric antibodies; (b) grafting only the non-human complementarity determining regions (CDRs) into human framework and constant regions with or without retention of critical framework residues; and (c) transplanting the entire non-human variable domains, but "cloaking" them with a human-like section by replacement of surface residues. Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Reichmann et al., Nature, 332:323-327 (1988); Verhoeyen et al., Science, 239:1534-1536 (1988)), by substituting hypervariable region sequences for the corresponding sequences of a human antibody. In practice, humanized antibodies are typically human antibodies in which some hypervariable region residues and possibly some framework region (FR) residues are substituted by residues from analogous sites in rodent antibodies. The choice of human variable domains, both light and heavy, to be used in making the humanized antibodies is very important to reduce immunogenicity retaining the specifity and affinity for the antigen. According to the so called "best-fit" method, the sequence of the variable domain of a rodent antibody is screened against the entire library of known human variable-domain sequences. The human sequence which is closest to that of the rodent is then accepted as the human framework region (FR) for the humanized antibody (Suns et al., J. Immunol., 151:2296 (1993); Chothia et al., J. Mol. Biol, 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993)).
   It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences.
- Primatized antibodies:
   A next step in this approach of making an antibody more similar at humans' are the so called primatized antibodies, i.e. a recombinant antibody which has been engineered to contain the variable heavy and light domains of a monkey (or other primate) antibody, in particular, a cynomolgus monkey antibody, and which contains human constant domain sequences, preferably the human immunoglobulin gamma 1 or gamma 4 constant domain (or PE variant). The preparation of such antibodies is described in Newman et al., Biotechnology, 10:1455-1460 (1992); US5658570 and
   US 6113898. These antibodies have been reported to exhibit a high degree of homology to human antibodies, i.e., 85-98%, display human effector functions, have reduced immunogenicity, and may exhibit high affinity to human antigens. Another highly efficient means for generating recombinant antibodies is disclosed by Newman, Biotechnology, 10:1455-1460 (1992).
- Human antibodies:
   By "human antibody" is meant an antibody containing entirely human light and heavy chain as well as constant regions, produced by any of the known standard methods.
   As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region PH) gene in chimeric and germ-line mutant mice results in complete inhibition of endogenous antibody production. Transfer of the human germ-line immunoglobulin gene array in such germ line mutant mice will result in the production of human antibodies after immunization. See, e.g., Jakobovits et al., Proc. Natl. Acad. Sci. USA, 90:2551 (1993); Jakobovits et al., Nature, 362:255-258 (1993).
   Alternatively, phage display technology (McCafferty et al., Nature 348:552-553 (1990)) can be used to produce human antibodies and antibody fragments *in vitro,* from immunoglobulin variable (V) domain gene repertoires from donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review see, e.g., Johnson, Kevin S. and Chiswell, David J., Current Opinion in Structural Biology 3:564-571 (1993).
   Human antibodies may also be generated by *in vitro* activated B cells or SCID mice with its immune system reconstituted with human cells.
   Once obtained a human antibody, its coding DNA sequences can be isolated, cloned and introduced in an appropriate expression system i.e. a cell line preferably from a mammal which subsequently express and liberate into culture media from which the antibody can be isolated.
- Antibody fragments:
   An antibody fragment is a fragment of an antibody such as, for example, Fab, F(ab')2, Fab' and scFv. Various techniques have been developed for the production of antibody fragments. Traditionally, these fragments were derived via proteolytic digestion of intact antibodies but more recently these fragments can be produced directly by recombinant host cells. In other embodiments, the antibody of choice is a single chain Fv (scFv) fragment which additionally may be monospecific or bispecific.
   Papain digestion of antibodies produces two identical antigen-binding fragments, called "Fab" fragments, each with a single antigen-binding site, and a residual "Fc" fragment, which name reflects its ability to crystallize readily. Pepsin treatment yields an F(ab')2 fragment that has two antigen-binding sites and is still capable of cross-linking antigen.
   "Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the VH-VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.
   The Fab fragment also contains the constant domain of the light chain and the first constant domain (CH1) of the heavy chain. Fab' fragments differ from Fab fragments by the addition of a few residues at the carboxy terminus of the heavy chain CHI domain including one or more cysteines from the antibody hinge region. Fab'-SH is the designation herein for Fab' in which the cysteine residue(s) of the constant domains bear at least one free thiol group. F(ab')₂ antibody fragments originally were produced as pairs of Fab' fragments which have hinge cysteines between them. Other chemical couplings of antibody fragments are also known.
   "Single-chain Fv" or "scFv" antibody fragments comprise the VH and VL domains of an antibody, wherein these domains are present in a single polypeptide chain. Preferably, the Fv polypeptide further comprises a polypeptide linker between the VH and VL domains which enables the scFv to form the desired structure for antigen binding. For a review of scFv see Pluckthun in The Pharmacology of Monoclonal Antibodies, voL 113, Rosenburg and Moore eds., Springer-Verlag, N.Y., pp. 269-315 (1994).
   The term "diabodies" refers to small antibody fragments with two antigen-binding sites, which fragments comprise a heavy-chain variable domain (VH) connected to a light chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et aL, Proc. Natl. Acad. Sci. USA, 90:6444-6448 (1993).
   Functional fragments of antibodies which bind ChoK alpha isoform included within the present invention retain at least one binding function and/or modulation function of the full-length antibody from which they are derived. Preferred functional fragments retain an antigen-binding function of a corresponding full-length antibody (e.g., the ability to bind a mammalian ChoK alpha isoform). Particularly preferred functional fragments retain the ability to inhibit one or more functions characteristic of a mammalian ChoK alpha isoform, such as a binding activity, a signaling activity, and/or stimulation of a cellular response. For example, in one embodiment, a functional fragment can inhibit the interaction of ChoK alpha isoform with one or more of its ligands and/or can inhibit one or more receptor-mediated functions.
- Bispecific antibodies:
   Bispecific antibodies are antibodies that have binding specificities for at least two different epitopes. Exemplary bispecific antibodies may bind to two different epitopes of the B cell surface marker. Other such antibodies may bind a first B cell marker and further bind a second B cell surface marker. Alternatively, an anti-B cell marker binding arm may be combined with an arm which binds to a triggering molecule on a leukocyte such as a T-cell receptor molecule (e.g. CD2 or CD3), or Fc receptors for IgG (FcyR), such as FcyRI (CD64), FcyRII (CD32) and FcyRIII (CD 16) so as to focus cellular defense mechanisms to the B cell. Bispecific antibodies may also be used to localize cytotoxic agents to the B cell. These antibodies possess a B cell marker-binding arm and an arm which binds the cytotoxic agent (e.g. saporin, anti-interferon-a, vinca alkaloid, ricin A chain, methotrexate or radioactive isotope hapten). Bispecific antibodies can be prepared as full length antibodies or antibody fragments (e.g. F(ab)2 bispecific antibodies).
   Methods for making bispecific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J, 10:3655-3659 (1991).
   According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 region. It is preferred to have the first heavy-chain constant region (CHI) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.
   Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells. Heteroconjugate antibodies may be made using any convenient cross-linking methods. Suitable cross-linking agents are well known in the art, and are disclosed in US 4,676,980, along with a number of cross-linking techniques.
   Techniques for generating bispecific antibodies from antibody fragments have also been described in the literature. For example, bispecific antibodies can be prepared using chemical linkage.

### Acid ceramidase- or choline kinase-specific RNA interference (RNAi)

In another embodiment, the inhibitors of the acid ceramidase or choline kinase alpha isoform that form part of the compositions of the invention are RNAi which are capable of knocking down the expression of acid ceramidase and/or choline kinase alpha isoform or any component gene necessary for acid ceramidase and/or choline kinase alpha isoform function. RNAi is a process of sequence-specific post-transcriptional gene repression which can occur in eukaryotic cells. In general, this process involves degradation of an mRNA of a particular sequence induced by double-stranded RNA (dsRNA) that is homologous to that sequence. For example, the expression of a long dsRNA corresponding to the sequence of a particular single-stranded mRNA (ss mRNA) will labilize that message, thereby "interfering" with expression of the corresponding gene. Accordingly, any selected gene may be repressed by introducing a dsRNA which corresponds to all or a substantial part of the mRNA for that gene. It appears that when a long dsRNA is expressed, it is initially processed by a ribonuclease III into shorter dsRNA oligonucleotides of as few as 21 to 22 base pairs in length. Accordingly, RNAi may be effected by introduction or expression of relatively short homologous dsRNAs. Indeed, the use of relatively short homologous dsRNAs may have certain advantages as discussed below.

The double stranded oligonucleotides used to effect RNAi are preferably less than 30 base pairs in length and, more preferably, comprise about 25, 24, 23, 22, 21, 20, 19, 18 or 17 base pairs of ribonucleic acid. Optionally the dsRNA oligonucleotides of the invention may include 3' overhang ends. Exemplary 2-nucleotide 3' overhangs may be composed of ribonucleotide residues of any type and may even be composed of 2'-deoxythymidine residues, which lowers the cost of RNA synthesis and may enhance nuclease resistance of siRNAs in the cell culture medium and within transfected cells (see Elbashir et al., Nature 411: 494-8, 2001). Longer dsRNAs of 50, 75, 100 or even 500 base pairs or more may also be utilized in certain embodiments of the invention. Exemplary concentrations of dsRNAs for effecting RNAi are about 0.05 nM, 0.1 nM, 0.5 nM, 1.0 nM, 1.5 nM, 25 nM or 100 nM, although other concentrations may be utilized depending upon the nature of the cells treated, the gene target and other factors readily discernable to the skilled artisan. Exemplary dsRNAs may be synthesized chemically or produced in vitro or in vivo using appropriate expression vectors. Exemplary synthetic RNAs include 21 nucleotide RNAs chemically synthesized using methods known in the art (e.g., Expedite RNA phosphoramidites and thymidine phosphoramidite (Proligo, Germany). Synthetic oligonucleotides are preferably deprotected and gel-purified using methods known in the art (see, e.g., Elbashir et al., Genes Dev. 15: 188-200, 2001). Longer RNAs may be transcribed from promoters, such as T7 RNA polymerase promoters, known in the art. A single RNA target, placed in both possible orientations downstream of an in vitro promoter, will transcribe both strands of the target to create a dsRNA oligonucleotide of the desired target sequence. Any of the above RNA species will be designed to include a portion of nucleic acid sequence represented in a target nucleic acid, such as, for example, a nucleic acid that hybridizes, under stringent and/or physiological conditions, to the polynucleotide encoding human acid ceramidase or human ChoK alpha isoform.

The specific sequence utilized in design of the oligonucleotides may be any contiguous sequence of nucleotides contained within the expressed gene message of the target. Programs and algorithms, known in the art, may be used to select appropriate target sequences. In addition, optimal sequences may be selected utilizing programs designed to predict the secondary structure of a specified single stranded nucleic acid sequence and allowing selection of those sequences likely to occur in exposed single stranded regions of a folded mRNA. Methods and compositions for designing appropriate oligonucleotides may be found, for example, in U.S. Pat. No. 6,251,588, the contents of which are incorporated herein by reference. Messenger RNA (mRNA) is generally thought of as a linear molecule which contains the information for directing protein synthesis within the sequence of ribonucleotides, however studies have revealed a number of secondary and tertiary structures that exist in most mRNAs. Secondary structure elements in RNA are formed largely by Watson-Crick type interactions between different regions of the same RNA molecule. Important secondary structural elements include intramolecular double stranded regions, hairpin loops, bulges in duplex RNA and internal loops. Tertiary structural elements are formed when secondary structural elements come in contact with each other or with single stranded regions to produce a more complex three dimensional structure. A number of researchers have measured the binding energies of a large number of RNA duplex structures and have derived a set of rules which can be used to predict the secondary structure of RNA (see, e.g., Jaeger et al., Proc. Natl. Acad. Sci. USA 86: 7706, 1989; and Turner et al., Annu. Rev. Biophys. Biophys. Chem. 17:167, 1988). The rules are useful in identification of RNA structural elements and, in particular, for identifying single stranded RNA regions which may represent preferred segments of the mRNA to target for silencing RNAi, ribozyme or antisense technologies. Accordingly, preferred segments of the mRNA target can be identified for design of the RNAi mediating dsRNA oligonucleotides as well as for design of appropriate ribozyme and hammerhead ribozyme compositions of the invention.

Several different types of molecules have been used effectively in the RNAi technology. Small interfering RNA (siRNA), sometimes known as short interfering RNA or silencing RNA, are a class of 20-25 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g., as an antiviral mechanism or in shaping the chromatin structure of a genome. Synthetic siRNAs have been shown to be able to induce RNAi in mammalian cells. This discovery led to a surge in the use of siRNA/RNAi for biomedical research and drug development.

MicroRNA (miRNA) are a related class of gene regulatory small RNAs, typically 21-23 nt in length. They typically differ from siRNA because they are processed from single stranded RNA precursors and show only partially complementary to mRNA targets. Initial studies have indicated that miRNAs regulate gene expression post-transcriptionally at the level of translational inhibition at P-Bodies in the cytoplasm. However, miRNAs may also guide mRNA cleavage similar to siRNAs. This is often the case in plants where the target sites are typically highly complementary to the miRNA. While target sites in plant mRNAs can be found in the 5'UTR, open-reading frames and 3'UTR, in animals, it is the 3' UTR that is the main target. miRNAs are first transcribed as part of a primary microRNA (pri-miRNA). This is then processed by the Drosha with the help of Pasha/DGCR8 (=Microprocessor complex) into pre-miRNAs. The ca. 75 nt pre-miRNA is then exported to the cytoplasm by exportin-5, where it is then diced into 21-23 nt siRNA-like molecules by Dicer. In some cases, multiple miRNAs can be found on the pri-miRNA.

Short hairpin RNA (shRNA) is yet another type of RNA that may be used to effect RNAi. It is a sequence of RNA that makes a tight hairpin turn that can be used to silence gene expression. shRNA is transcribed by RNA polymerase III.

Currently, short-interfering RNAs (siRNAs) and short-hairpin RNAs (shRNAs) are being extensively used to silence various genes to silence functions carried out by the genes. It is becoming easier to harness RNAi to silence specific genes, owing to the development of libraries of readymade shRNA and siRNA gene-silencing constructs by using a variety of sources. For example, RNAi Codex, which consists of a database of shRNA related information and an associated website, has been developed as a portal for publicly available shRNA resources and is accessible at http://codex.cshl.org. RNAi Codex currently holds data from the Hannon-Elledge shRNA library and allows the use of biologist-friendly gene names to access information on shRNA constructs that can silence the gene of interest. It is designed to hold user-contributed annotations and publications for each construct, as and when such data become available. Olson et al. (Nucleic Acids Res. 34(Database issue): D153-D157, 2006, incorporated by reference) have provided detailed descriptions about features of RNAi Codex, and have explained the use of the tool. All these information may be used to help design the various siRNA or shRNA targeting acid ceramidase, choline kinase alpha isoform or other proteins of interest.

### Acid ceramidase- or choline kinase-specific ribozymes

Ribozyme molecules designed to catalytically cleave a target mRNA transcripts can also be used to prevent translation of acid ceramidase and/or choline kinase alpha isoform mRNAs. Accordingly, in another embodiment, the compositions of the invention comprise ribozymes specifically directed to the mRNA acid ceramidase and/or choline alpha isoform kinase. Ribozymes are enzymatic RNA molecules capable of catalyzing the specific cleavage of RNA. (For a review, see Rossi, Current Biology 4: 469-471, 1994). The mechanism of ribozyme action involves sequence specific hybridization of the ribozyme molecule to complementary target RNA, followed by an endonucleolytic cleavage event. The composition of ribozyme molecules preferably includes one or more sequences complementary to a target mRNA, and the well known catalytic sequence responsible for mRNA cleavage or a functionally equivalent sequence (see, e.g., U.S. Pat. No. 5,093,246, incorporated herein by reference in its entirety).

While ribozymes that cleave mRNA at site specific recognition sequences can be used to destroy target mRNAs, the use of hammerhead ribozymes is preferred. Hammerhead ribozymes cleave mRNAs at locations dictated by flanking regions that form complementary base pairs with the target mRNA. Preferably, the target mRNA has the following sequence of two bases: 5'-UG-3'. The construction and production of hammerhead ribozymes is well known in the art and is described more fully in Haseloff and Gerlach, Nature 334: 585-591, 1988; and see PCT Appln. No. WO89/05852, the contents of which are incorporated herein by reference. Hammerhead ribozyme sequences can be embedded in a stable RNA such as a transfer RNA (tRNA) to increase cleavage efficiency in vivo (Perriman et al., Proc. Natl. Acad. Sci. USA, 92: 6175-79, 1995; de Feyter, and Gaudron, Methods in Molecular Biology, Vol. 74, Chapter 43, "Expressing Ribozymes in Plants," Edited by Turner, P. C, Humana Press Inc., Totowa, N.J.). In particular, RNA polymerase III-mediated expression of tRNA fusion ribozymes are well known in the art (see, Kawasaki et al., Nature 393: 284-9, 1998; Kuwabara et al., Nature Biotechnol. 16: 961-5, 1998; and Kuwabara et al., Mol. Cell. 2: 617-27, 1998; Koseki et al., J Virol 73: 1868-77, 1999; Kuwabara et al., Proc Natl Acad Sci USA 96: 1886-91, 1999; Tanabe et al., Nature 406: 473-4, 2000). There are typically a number of potential hammerhead ribozyme cleavage sites within a given target CDNA sequence. Preferably the ribozyme is engineered so that the cleavage recognition site is located near the 5' end of the target mRNA--to increase efficiency and minimize the intracellular accumulation of non-functional mRNA transcripts. Furthermore, the use of any cleavage recognition site located in the target sequence encoding different portions of the C-terminal amino acid domains of, for example, long and short forms of target would allow the selective targeting of one or the other form of the target, and thus, have a selective effect on one form of the target gene product.

Gene targeting ribozymes necessarily contain a hybridizing region complementary to two regions, each of at least 5 and preferably each 6, 7, 8, 9, 10, 11, 12, 13, 14, 15, 16, 17, 18, 19 or 20 contiguous nucleotides in length of a target mRNA, such as an mRNA of a sequence represented in the acid ceramidase or in the choline kinase genes. In addition, ribozymes possess highly specific endoribonuclease activity, which autocatalytically cleaves the target sense mRNA. The present invention extends to ribozymes which hybridize to a sense mRNA encoding a target gene such as a therapeutic drug target candidate gene, thereby hybridizing to the sense mRNA and cleaving it, such that it is no longer capable of being translated to synthesize a functional polypeptide product.

The ribozymes used in the compositions of the present invention also include RNA endoribonucleases (hereinafter "Cech-type ribozymes") such as the one which occurs naturally in Tetrahymena thermophila (known as the IVS, or L-19 IVS RNA) and which has been extensively described by Thomas Cech and collaborators (Zaug et al., Science 224:574-578, 1984; Zaug et al., Science 231: 470-475, 1986; Zaug et al., Nature 324: 429-433, 1986; published International patent application No. WO88/04300 by University Patents Inc.; Been, et al., Cell 47: 207-216, 1986). The Cech-type ribozymes have an eight base pair active site which hybridizes to a target RNA sequence whereafter cleavage of the target RNA takes place. The invention encompasses those Cech-type ribozymes which target eight base-pair active site sequences that are present in a target gene or nucleic acid sequence.

Ribozymes can be composed of modified oligonucleotides (e.g., for improved stability, targeting, etc.) and should be delivered to cells which express the target gene in vivo. A preferred method of delivery involves using a DNA construct "encoding" the ribozyme under the control of a strong constitutive pol III or pol II promoter, so that transfected cells will produce sufficient quantities of the ribozyme to destroy endogenous target messages and inhibit translation. Because ribozymes, unlike antisense molecules, are catalytic, a lower intracellular concentration is required for efficiency.

In certain embodiments, a ribozyme may be designed by first identifying a sequence portion sufficient to cause effective knockdown by RNAi. The same sequence portion may then be incorporated into a ribozyme. In this aspect of the invention, the gene-targeting portions of the ribozyme or RNAi are substantially the same sequence of at least 5 and preferably 6, 7, 8, 9, 10, 11, 12, 13, 14, 15, 16, 17, 18, 19 or 20 or more contiguous nucleotides of a target nucleic acid, such as a nucleic acid of any of the human acid ceramidase or choline kinase sequences. In a long target RNA chain, significant numbers of target sites are not accessible to the ribozyme because they are hidden within secondary or tertiary structures (Birikh et al., Eur J Biochem 245: 1-16, 1997). To overcome the problem of target RNA accessibility, computer generated predictions of secondary structure are typically used to identify targets that are most likely to be single-stranded or have an "open" configuration (see Jaeger et al., Methods Enzymol 183: 281-306, 1989). Other approaches utilize a systematic approach to predicting secondary structure which involves assessing a huge number of candidate hybridizing oligonucleotides molecules (see Milner et al., Nat Biotechnol 15: 537-41, 1997; and Patzel and Sczakiel, Nat Biotechnol 16: 64-8, 1998). Additionally, U.S. Pat. No. 6,251,588, the contents of which are hereby incorporated herein, describes methods for evaluating oligonucleotide probe sequences so as to predict the potential for hybridization to a target nucleic acid sequence. The method of the invention provides for the use of such methods to select preferred segments of a target mRNA sequence that are predicted to be single-stranded and, further, for the opportunistic utilization of the same or substantially identical target mRNA sequence, preferably comprising about 10-20 consecutive nucleotides of the target mRNA, in the design of both the RNAi oligonucleotides and ribozymes of the invention.

### Acid ceramidase- or choline kinase-specific antisense nucleic acids

A further aspect of the invention relates to the use of the isolated "antisense" nucleic acids to inhibit expression, e.g., by inhibiting transcription and/or translation of acid ceramidase and/or choline kinase alpha isoform nucleic acids. The antisense nucleic acids may bind to the potential drug target by conventional base pair complementarity, or, for example, in the case of binding to DNA duplexes, through specific interactions in the major groove of the double helix. In general, these methods refer to the range of techniques generally employed in the art, and include any methods that rely on specific binding to oligonucleotide sequences.

An antisense construct of the present invention can be delivered, for example, as an expression plasmid which, when transcribed in the cell, produces RNA which is complementary to at least a unique portion of the cellular mRNA which encodes a ChoK polypeptide or an acid ceramidase polypeptide. Alternatively, the antisense construct is an oligonucleotide probe, which is generated ex vivo and which, when introduced into the cell causes inhibition of expression by hybridizing with the mRNA and/or genomic sequences of a target nucleic acid. Such oligonucleotide probes are preferably modified oligonucleotides, which are resistant to endogenous nucleases, e.g., exonucleases and/or endonucleases, and are therefore stable in vivo. Exemplary nucleic acid molecules for use as antisense oligonucleotides are phosphoramidate, phosphothioate and methylphosphonate analogs of DNA (see also U.S. Pat. Nos. 5,176,996; 5,264,564; and 5,256,775). Additionally, general approaches to constructing oligomers useful in antisense therapy have been reviewed, for example, by Van der Krol et al., BioTechniques 6: 958-976, 1988; and Stein et al., Cancer Res 48: 2659-2668, 1988.

With respect to antisense DNA, oligodeoxyribonucleotides derived from the translation initiation site, e.g., between the -10 and +10 regions of the target gene, are preferred. Antisense approaches involve the design of oligonucleotides (either DNA or RNA) that are complementary to mRNA encoding the target polypeptide. The antisense oligonucleotides will bind to the mRNA transcripts and prevent translation. Absolute complementarity, although preferred, is not required. In the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of the mRNA, e.g., the 5' untranslated sequence up to and including the AUG initiation codon, should work most efficiently at inhibiting translation. However, sequences complementary to the 3' untranslated sequences of mRNAs have recently been shown to be effective at inhibiting translation of mRNAs as well (Wagner, Nature 372: 333, 1994). Therefore, oligonucleotides complementary to either the 5' or 3' untranslated, non-coding regions of a gene could be used in an antisense approach to inhibit translation of that mRNA. Oligonucleotides complementary to the 5' untranslated region of the mRNA should include the complement of the AUG start codon. Antisense oligonucleotides complementary to mRNA coding regions are less efficient inhibitors of translation but could also be used in accordance with the invention. Whether designed to hybridize to the 5', 3' or coding region of mRNA, antisense nucleic acids should be at least six nucleotides in length, and are preferably less that about 100 and more preferably less than about 50, 25, 17 or 10 nucleotides in length.

It is preferred that in vitro studies are first performed to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Results obtained using the antisense oligonucleotide may be compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

The antisense oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g., for targeting host cell receptors), or agents facilitating transport across the cell membrane (see, e.g., Letsinger et al., Proc. Natl. Acad. Sci. U.S.A. 86: 6553-6556, 1989; Lemaitre et al., Proc. Natl. Acad. Sci. 84: 648-652, 1987; PCT Publication No. WO88/09810) or the blood-brain barrier (see, e.g., PCT Publication No. WO89/10134), hybridization-triggered cleavage agents (see, e.g., Krol et al., BioTechniques 6: 958-976, 1988) or intercalating agents (see, e.g., Zon, Pharm. Res. 5: 539-549, 1988). To this end, the oligonucleotide may be conjugated to another molecule, e.g., a peptide, hybridization triggered cross-linking agent, transport agent, hybridization-triggered cleavage agent, etc.

The antisense oligonucleotide may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxytiethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose. The antisense oligonucleotide can also contain a neutral peptide-like backbone. Such molecules are termned peptide nucleic acid (PNA)-oligomers and are described, e.g., in Perry-O'Keefe et al., Proc. Natl. Acad. Sci. U.S.A. 93: 14670, 1996, and in Eglom et al., Nature 365: 566, 1993. One advantage of PNA oligomers is their capability to bind to complementary DNA essentially independently from the ionic strength of the medium due to the neutral backbone of the DNA. In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet a further embodiment, the antisense oligonucleotide is an alpha-anomeric oligonucleotide. An alpha-anomeric oligonucleotide forms specific double-stranded hybrids with complementary RNA in which, contrary to the usual antiparallel orientation, the strands run parallel to each other (Gautier et al., Nucl. Acids Res. 15: 6625-6641, 1987). The oligonucleotide is a 2'-0-methylribonucleotide (Inoue et al., Nucl. Acids Res. 15: 6131-6148, 1987), or a chimeric RNA-DNA analogue (Inoue et al., FEBS Lett. 215: 327-330, 1987).

While antisense nucleotides complementary to the coding region of a target mRNA sequence can be used, those complementary to the transcribed untranslated region may also be used.

In certain instances, it may be difficult to achieve intracellular concentrations of the antisense sufficient to suppress translation on endogenous mRNAs. Therefore a preferred approach utilizes a recombinant DNA construct in which the antisense oligonucleotide is placed under the control of a strong pol III or pol II promoter. The use of such a construct to transfect target cells will result in the transcription of sufficient amounts of single stranded RNAs that will form complementary base pairs with the endogenous potential drug target transcripts and thereby prevent translation. For example, a vector can be introduced such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in mammalian cells. Expression of the sequence encoding the antisense RNA can be by any promoter known in the art to act in mammalian, preferably human cells. Such promoters can be inducible or constitutive. Such promoters include but are not limited to: the SV40 early promoter region (Bernoist and Chambon, Nature 290: 304-310, 1981), the promoter contained in the 3' long terminal repeat of Rous sarcoma virus (Yamamoto et al., Cell 22: 787-797, 1980), the herpes thymidine kinase promoter (Wagner et al., Proc. Natl. Acad. Sci. U.S.A. 78: 1441-1445, 1981), the regulatory sequences of the metallothionein gene (Brinster et al, Nature 296: 39-42, 1982), etc. Any type of plasmid, cosmid, YAC or viral vector can be used to prepare the recombinant DNA construct, which can be introduced directly into the tissue site.

Alternatively, target gene expression can be reduced by targeting deoxyribonucleotide sequences complementary to the regulatory region of the gene (i.e., the promoter and/or enhancers) to form triple helical structures that prevent transcription of the gene in target cells in the body (see generally, Helene, Anticancer Drug Des. 6(6): 569-84, 1991; Helene et al., Ann. N.Y. Acad. Sci., 660: 27-36, 1992; and Maher, Bioassays 14(12): 807-15, 1992).

Nucleic acid molecules to be used in triple helix formation for the inhibition of transcription are preferably single stranded and composed of deoxyribonucleotides. The base composition of these oligonucleotides should promote triple helix formation via Hoogsteen base pairing rules, which generally require sizable stretches of either purines or pyrimidines to be present on one strand of a duplex. Nucleotide sequences may be pyrimidine-based, which will result in TAT and CGC triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, containing a stretch of G residues. These molecules will form a triple helix with a DNA duplex that is rich in GC pairs, in which the majority of the purine residues are located on a single strand of the targeted duplex, resulting in CGC triplets across the three strands in the triplex.

Alternatively, the potential target sequences that can be targeted for triple helix formation may be increased by creating a so called "switchback" nucleic acid molecule. Switchback molecules are synthesized in an alternating 5'-3',3'-5' manner, such that they base pair with first one strand of a duplex and then the other, eliminating the necessity for a sizable stretch of either purines or pyrimidines to be present on one strand of a duplex.

In certain embodiments, the antisense oligonucleotides are morpholino antisenses. Morpholinos are synthetic molecules which are the product of a redesign of natural nucleic acid structure. Usually 25 bases in length, they bind to complementary sequences of RNA by standard nucleic acid base-pairing. Structurally, the difference between morpholinos and DNA is that while morpholinos have standard nucleic acid bases, those bases are bound to morpholine rings instead of deoxyribose rings, and linked through phosphorodiamidate groups instead of phosphates. Replacement of anionic phosphates with the uncharged phosphorodiamidate groups eliminates ionization in the usual physiological pH range, so morpholinos in organisms or cells are uncharged molecules. Morpholinos are not chimeric oligos; the entire backbone of a morpholino is made from these modified subunits. Morpholinos are most commonly used as single-stranded oligos, though heteroduplexes of a morpholino strand and a complementary DNA strand may be used in combination with cationic cytosolic delivery reagents.

Unlike many antisense structural types (e.g., phosphorothioates), morpholinos do not degrade their target RNA molecules. Instead, morpholinos act by "steric blocking," binding to a target sequence within an RNA and simply getting in the way of molecules which might otherwise interact with the RNA. Morpholino oligos are often used to investigate the role of a specific mRNA transcript in an embryo, such as eggs or embryos of zebrafish, African clawed frog (Xenopus), chick, and sea urchin, producing morphant embryos. With appropriate cytosolic delivery systems, morpholinos are effective in cell culture.

Bound to the 5'-untranslated region of messenger RNA (mRNA), morpholinos can interfere with progression of the ribosomal initiation complex from the 5' cap to the start codon. This prevents translation of the coding region of the targeted transcript (called "knocking down" gene expression). Morpholinos provide a convenient means of knocking down expression of the protein and learning how that knockdown changes the cells or organism. Some morpholinos knock down expression so effectively that after degradation of preexisting proteins the targeted proteins become undetectable by Western blot.

Morpholinos can also interfere with pre-mRNA processing steps, usually by preventing the splice-directing snRNP complexes from binding to their targets at the borders of introns on a strand of pre-RNA. Preventing U1 (at the donor site) or U2/U5 (at the polypyrimidine moiety and acceptor site) from binding can cause modified splicing, commonly leading to exclusions of exons from the mature mRNA. Targeting some splice targets results in intron inclusions, while activation of cryptic splice sites can lead to partial inclusions or exclusions. Targets of U11/U12 snRNPs can also be blocked. Splice modification can be conveniently assayed by reverse-transcriptase polymerase chain reaction (RT-PCR) and is seen as a band shift after gel electrophoresis of RT-PCR products.

Morpholinos have also been used to block miRNA activity, ribozyme activity, intronic splice silencers, and splice enhancers. U2 and U12 snRNP functions have been inhibited by Morpholinos. Morpholinos targeted to "slippery" mRNA sequences within protein coding regions can induce translational frameshifts. Activities of Morpholinos against this variety of targets suggest that Morpholinos can be used as a general-purpose tool for blocking interactions of proteins or nucleic acids with mRNA.

### Acid ceramidase- or choline kinase-specific DNA enzymes

A further aspect of the invention relates to compositions wherein
the acid ceramidase inhibitor and/or the choline kinase alpha isoform inhibitor is/are DNA enzymes. DNA enzymes incorporate some of the mechanistic features of both antisense and ribozyme technologies. DNA enzymes are designed so that they recognize a particular target nucleic acid sequence, much like an antisense oligonucleotide, however much like a ribozyme they are catalytic and specifically cleave the target nucleic acid.

There are currently two basic types of DNA enzymes, and both of these were identified by Santoro and Joyce (see, for example, U.S. Pat. No. 6,110,462). The 10-23 DNA enzyme comprises a loop structure which connect two arms. The two arms provide specificity by recognizing the particular target nucleic acid sequence while the loop structure provides catalytic function under physiological conditions.

Briefly, to design an ideal DNA enzyme that specifically recognizes and cleaves a target nucleic acid, one of skill in the art must first identify the unique target sequence. This can be done using the same approach as outlined for antisense oligonucleotides. Preferably, the unique or substantially sequence is a G/C rich of approximately 18 to 22 nucleotides. High G/C content helps insure a stronger interaction between the DNA enzyme and the target sequence.

When synthesizing the DNA enzyme, the specific antisense recognition sequence that will target the enzyme to the message is divided so that it comprises the two arms of the DNA enzyme, and the DNA enzyme loop is placed between the two specific arms.

Methods of making and administering DNA enzymes can be found, for example, in U.S. Pat. No. 6,110,462. Similarly, methods of delivery DNA ribozymes in vitro or in vivo include methods of delivery RNA ribozyme, as outlined in detail above. Additionally, one of skill in the art will recognize that, like antisense oligonucleotide, DNA enzymes can be optionally modified to improve stability and improve resistance to degradation.

Antisense RNA and DNA, ribozyme, RNAi and triple helix molecules of the invention may be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules may be generated by in vitro and in vivo transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines. Moreover, various well-known modifications to nucleic acid molecules may be introduced as a means of increasing intracellular stability and half-life. Possible modifications include but are not limited to the addition of flanking sequences of ribonucleotides or deoxyribonucleotides to the 5' and/or 3' ends of the molecule or the use of phosphorothioate or 2' O-methyl rather than phosphodiesterase linkages within the oligodeoxyribonucleotide backbone.

Preferred combinations of ChoK alpha isoform inhibitors and acid ceramidase inhibitors include MN58b (Structure provided in line II in Table 1) and NOE (structure provided in line 6, table 2), RSM-932A (structure provided in line I in table 1) and NOE, MN58b and D-NMAPPD (structure provided in line VIII in table 2) and RSM-932A and D-NMAPPD.

### COMBINATIONS OF ChoK INHIBITORS AND CHEMOTHERAPY AGENTS (SECOND COMPOSITION OF THE INVENTION)

The authors of the present invention have found that, surprisingly, the resistance to the treatment with the ChoK inhibitor MN58b does not correlate with resistances to conventional chemotherapeutic agentes such as cisplatin, taxol, virelbine or gemcitabine. This has been observed both in tumours resistant to ChoK inhibitors (see example 1) as well as in established tumor cell lines selected by repeated cycles of growth in the presence of a ChoK inhibitor (see example 6 of the invention). Without wishing to be bound by any theory, it is believed that the non-crossed resistance between ChoK inhibitors and cisplatin is due to the fact that both drugs act through different mechanisms. This hypothesis is supported by the fact that combinations of a ChoK inhibitor and cisplatin results in a synergistic effect in growth inhibition of tumor cells when compared to the treatment with the individual compounds (see example 7 of the invention) both when tested *in vitro* and *in vivo* in xenograft models (see example 7 of the invention). Moreover, the authors of the present invention have also observed that the combined use of choline kinase inhibitors and 5-fluorouracile results in a synergistic antitumor effect when tested in different colon cancer cell lines (see example 9).

Thus, in another aspect, the invention relates to a composition (hereinafter second composition of the invention) comprising, separately or together, a first component comprising one or more ChoK inhibitors specific for the choline kinase alpha isoform and a second component comprising one or more chemotherapy agents, wherein the chemotherapy agent is selected from the group consisting of a DNA-alkylating agent, an antimetabolite, a mitotic inhibitor, an anthracycline, a topoisomerase I inhibitor, a topoisomerase II inhibitor, cetuximab, gefitinib and imatinib.

The term "composition" refers to one or more compounds in various combinations according to alternative embodiments of this invention. Preferably, the composition comprises at least a ChoK inhibitor and at least an alkylating agent.

ChoK inhibitors suitable for use in the compositions of the invention include any of the ChoK inhibitors defined previously in Table 1 as forming part of the first composition of the invention. The ChoK inhibitor is an inhibitor specific for ChoK alpha isoform.

The term "chemotherapy agent", as used herein, refer to a chemical agent that inhibits the proliferation, growth, life-span or metastatic activity of cancer cells and include, without limitation, DNA-alkylating drugs, antimetabolites, mitotic inhibitors, anthracyclines, topoisomerase I and II inhibitors, cetuximab, gefitinib and imatinib. In a preferred embodiment, the chemotherapy agent is an alkylating agent and, more in particular, a DNA-alkylating agent or an antimetabolite.

The expression "alkylating agent", as used herein, relate to compounds capable of adding alkyl residues to the genetic material of rapidly dividing cells thus leading to replication arrest and cell death. Such agents include platinum-based compounds, nitrogen mustards, nitrosoureas, ethylenimine derivatives, alkyl sulfonates, and triazenes, including, but not limited to, mechlorethamine, cyclophosphamide (CytoxanTM), melphalan (L-sarcolysin), etoposide, carmustine (BCNU), lomustine (CCNU), semustine (methyl-CCNU), streptozocin, chlorozotocin, uracil mustard, chlormethine, ifosfamide, chlorambucil, pipobroman, triethylenemelamine, triethylenethiophosphoramine, busulfan, procarbazine, dacarbazine, and temozolomide.

In a preferred embodiment, the alkylating agent is a platinum-based compound. Exemplary platinum-based compounds that can be used in the present invention include, without limitation, cisplatin, carboplatin, iproplatin, tetraplatin, oxaliplatin, JM118, JM149, JM216, JM335, transplatino, cis, trans, cis-Pt(NH3)(C6H11NH2)(OOCC3H7)2Cl, nedaplatin, malanate-1,2-diaminociclohexanoplatin(II), 5-sulphosalycilate-trans-(1,2-diaminociclohexane)platin (II) (SSP), poly-[(trans-1,2-diaminocyclohexane)platin]-carboxyamilose (POLY-PLAT) and 4-hydroxy-sulphonylphenylacetate (trans-1,2-diaminocyclohexane) platino(II) (SAP).

The term "antimetabolite", as used herein, relates, in a broad sense, to substances which disturb normal metabolism and substances which inhibit the electron transfer system to prevent the production of energy-rich intermediates, due to their structural or functional similarities to metabolites that are important for living organisms (such as vitamins, coenzymes, amino acids and saccharides). Examples of antimetabolites that have antitumor activities include but are not limited to folic acid analogs (e.g., methotrexate (amethopterin)), denopterin, edatrexate, methotrexate, nolatrexed, pemetrexed, piritrexim, pteropterin, raltitrexed, trimetrexate; pyrimidine analogs (e.g., fluorouracil (5- fluorouracil; 5-FU(R)), floxuridine (fluorode-oxyuridine; FudR), doxifluridine and cytarabine (cytosine arabinoside)); purine analogs (e.g., mercaptopurine (6-mercaptopurine; 6-MP), thioguanine (6-thioguanine; TG), and pentostatin (2'-deoxycoformycin)).

Preferred combinations include, without limitation, MN58b and cisplatin and RSM-932A and cisplatin, MN58b and 5-fluorouracile and RSM-932A and 5-fluorouracile.

### COMBINATIONS OF ChoK INHIBITORS AND A DEATH RECEPTOR LIGAND (THIRD COMPOSITION)

The authors of the present invention have shown that treatment of tumor cells with a combination of a death receptor ligand and an inhibitor of ChoK results in an increased inhibition of cell proliferation when compared with the treatment with the death receptor ligand or the ChoK inhibitor separately. For instance, as shown in example 8 of the present invention, the treatment of colon cancer cells with a combination of the choline kinase inhibitor RSM-932A (ChoKI) and TRAIL results in an increased cytotoxicity when compared to the treatment of the same cells with each compound taken individually. Moreover, example 8 of the present invention shows that the combined use of the ChoK inhibitor MN58b and TRAIL results in an inhibition of tumor growth in a tumor xenograft model which is higher than that observed when each of the compounds is used separately.

Thu, in another aspect, it is also disclosed a composition comprising, together or separately, a first component comprising one or more inhibitors of ChoK and a second component which is one or more death receptor ligands.

ChoK inhibitors suitable for use in the compositions include any of the ChoK inhibitors defined previously in Table 1 as forming part of the first composition of the invention. In a preferred embodiment, the ChoK inhibitors is an inhibitor specific for ChoKα.

Death receptor ligands suitable for use in the compositions include NGF, CD40L, CD137L/4-1BBL, TNF-α CD134L/OX40L, CD27L/CD70, FasL/CD95, CD30L, TNF-β /LT- a, LT- β and TRAIL. In a preferred embodiment, the TNF family member is TRAIL, a functionally equivalent derivative thereof or a small mimic compound thereof. TRAIL (TNF-related apoptosis inducing ligand), also known as "Apo-2 ligand", "Apo-2L", "Apo2L", "Apo2L/TRAIL" and "Apo-2 ligand/TRAIL", is a molecule which is capable of inducing apoptosis in cells expressing the TRAIL cognate receptor. TRAIL was identified several years ago as a member of the TNF family of cytokines, (Pitti et al., 1996, J.Biol.Chem., 271:12687-12690 and US Patent 6,284,236). The full-length native sequence human TRAIL polypeptide is a 281 amino acid long, Type II transmembrane protein having a sequence as defined in SEQ ID NO: 7 (UniProt accession P50591). Crystallographic studies of soluble forms of TRAIL reveal a homotrimeric structure similar to the structures of TNF and other related proteins. TRAIL, unlike other TNF family members however, was found to have a unique structural feature in that three cysteine residues (at position 230 of each subunit in the homotrimer) together coordinate a zinc atom, and that the zinc binding is important for trimer stability and biological activity. The present invention contemplates the use of any of the three different TRAIL isoforms (TRAILα, TRAILβ and TRAILγ) o combinations thereof.

Functionally equivalent TRAIL variants include soluble TRAIL isoforms such as those described in WO08088582 and US6284236 or the TRAIL fragments 95-281, 114-281 described in US2002128438, scFv:sTRAIL fusions as described by Bremer et al (Neoplasia, 2004, 6:636-45), alternatively-spliced forms of TRAIL as described in US2002061525, TRAIL-receptor binding peptides as described in WO04101608, TRAIL variants with increased specificity for the pro-apoptotic receptors such as the 19 IL, 199V, 20 1 R, 213W, 215D and/or 193S TRAIL mutants as described in WO07063301 or variants selected by phage-display on receptors as described in WO04001009A, agonistic antibodies directed against TRAIL-cognate receptors TRAIL-R1 (DR4) and TRAIL-R2 (DR5) such as mapatumumab, lexatumumab, the antibodies described in WO07128231, or the antibodies described in WO02094880, the monoclonal antbibody AD5-10 described in WO06017961, TRAIL-specific tandem diabodies and tribodies as described in WO05056605, chimeric anti-DR4 antibodies as described in WO9937684, agonistic anti-DR5 antibodies as described in WO03038043, bispecific anti-TRAIL receptor antibodides as described in WO02085946, anti-DR4 specific antibodies as described in WO9832856, anti-DR2 ScFV as described by Park,K.J et al (Cancer Res., 2007, 67:7327-7334), trimeric TRAIL fusion proteins as described in WO08025516 and WO04014951, dodecameric TRAIL variants as described in WO07102690, pegylated TRAIL as described in WO07145457, DNA vectors comprising a polynucleotide encoding TRAIL such as those described in US2006153809, WO04087930, US2005031593.

Small molecule TRAIL mimics having pro-apoptotic effect include the compounds described in WO2008094319.

Preferred combinations include, without limitation, RSM-932A and the extracellular region of human TRAIL (amino acids 95-281) and MN58b and the extracellular region of human TRAIL (amino acids 95-281).

### PHARMACEUTICAL PREPARATIONS OF THE INVENTION

The compounds that form part of the first and second compositions of the invention include not only the compounds as such but also pharmaceutically acceptable salts, solvates, prodrugs thereof. The term "pharmaceutically acceptable salts, solvates, prodrugs" refers to any pharmaceutically salt, ester, solvate or any other compound which when administered to a receptor is able to provide (directly or indirectly) a compound as described in the present document. However, it will be observed that pharmaceutically unacceptable salts are also within the scope of the invention because the latter can be useful in the preparation of pharmaceutically acceptable salts. The preparation of salts, prodrugs and derivatives can be carried out by means of methods known in the art.

For example, pharmaceutically acceptable salts of compounds provided in the present document are synthesized by means of conventional chemical methods from an original compound containing a basic or acid residue. Such salts are generally prepared, for example, by reacting the free acid or base forms of the compounds with a stoichiometric amount of the suitable base or acid in water or an organic solvent or a mixture of both. Non-aqueous media such as DMSO (dimethylsulphoxide), ether, ethyl acetate, ethanol, isopropanol or acetonitrile are generally preferred. Examples of acid addition salts include mineral acid addition salts such as for example, hydrochloride, bromohydrate, iodohydrate, sulfate, nitrate, phosphate and organic acid addition salts such as for example, acetate, maleate, fumarate, citrate, oxalate, succinate, tartrate, malate, mandelate, methanesulfonate and p-toluenesulfonate. Examples of base addition salts include inorganic salts such as for example sodium, potassium, bromide, calcium, ammonium, magnesium, aluminium and lithium salts and organic base salts such as for example ethylenediamine, ethanolamine, N,N-dialkylenethanolamine, triethanolamine, glucamine and basic amino acid salts.

The particularly preferred derivatives or prodrugs are those increasing the bioavailability of the compounds of this invention when such compounds are administered to a patient (for example, by making a compound administered orally be absorbed more easily by blood), or enhancing the release of the original compound in a biological compartment (for example, the brain or the lymphatic system) in relation to the original species.

The invention also provides compositions wherein at least one of the compounds are found as prodrug. The term "prodrug" is used in its widest sense and includes those derivatives which are converted in vivo into the compounds of the invention. Such derivatives are evident for the persons skilled in the art and depending on the functional groups present in the molecule and without limitation, include the following derivatives of the present compounds: esters, amino acid esters, phosphate esters, metal salt sulfonate esters, carbamates and amides. Examples of methods for producing a prodrug of a given active compound are known by the person skilled in the art and can be found for example in Krogsgaard-Larsen et al. "Textbook of Drug design and Discovery" Taylor & Francis (April 2002).

The compounds of the invention can be in crystalline form as free compounds or as solvates and it is intended that both of them are within the scope of the present invention. The solvation methods are generally known in the art. The suitable solvates are pharmaceutically acceptable solvates. In a particular embodiment, the solvate is a hydrate. The compounds forming the compositions of the invention can include enantiomers, depending on the presence of chiral centers on a C, or isomers, depending on the presence of multiple bonds (for example, Z, E). The individual isomers, enantiomers or diastereoisomers and the mixtures thereof are included within the scope of the present invention.

The different substituents selected for the different compounds of the invention provide a series of factors considerably affecting the values of log P. Thus, hydroxyl groups act as hydrogen bond donors and intra or intermolecular links can be established even in the case of phenols. The presence of carbonyl or carboxyl groups generates proton acceptor groups in the molecule. The presence of halogens generates very deficient carbons and considerably modifies the biological properties. The amino groups generate good nucleophiles on the molecule and in most cases significantly modify its polarity and polarizability and the presence of additional alkyl and/or aryl groups increases the lypophilicity of the molecules.

In another aspect, the invention provides pharmaceutical compositions comprising the first or second compositons of the invention, their pharmaceutically acceptable salt, derivative, prodrug, solvate or stereoisomer thereof together with a pharmaceutically acceptable carrier, adjuvant or vehicle for the administration to a patient. The phrase "pharmaceutically acceptable carrier" as used herein means a pharmaceutically acceptable material, composition or vehicle, such as a liquid or solid filler, diluent, excipient, solvent or encapsulating material, involved in carrying or transporting the subject agents from one organ, or portion of the body, to another organ, or portion of the body. Each carrier must be "acceptable" in the sense of being compatible with the other ingredients of the formulation. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol, solutol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations such as DMSO (dimethylsulphoxide) and its derivatives.

The pharmaceutical compositions can be administered by any suitable administration route, for example an oral, topical, rectal or parenteral route (including subcutaneous, intraperitoneal, intradermal, intramuscular and intravenous route).

Suitable pharmaceutical forms for oral administration include any solid composition (tablets, pastilles, capsules, granules, etc.) or liquid composition (solutions, suspensions, emulsions, syrups, etc.) and can contain conventional excipients known in the art, such as binding agents, for example syrup, acacia, gelatin, sorbitol, tragacanth, or polyvinylpyrrolidone; fillers, for example lactose, sugar, cornstarch, calcium fosfate, sorbitol or glycine; lubricants for the preparation of tablets, for example magnesium stearate, disintegrants, for example starch, polyvinylpirrolidone, sodium starch glycolate or microcrystalline cellulose; or pharmaceutically acceptable wetting agents such as sodium laurylsulfate.

Solid oral compositions can be prepared by means of conventional methods for mixing, filling or preparing tablets. The repeated mixing operations can be used to distribute the active ingredient through the entire compositions by using large amounts of filler agents. Such operations are conventional in the art. The tablets can be prepared, for example by means of wet or dry granulation and can be optionally coated according to methods well known in normal pharmaceutical practice, particularly with an enteric coating.

The pharmaceutical compositions can also be adapted for parenteral administration such as sterile solutions, suspensions or lyophilized products in a suitable unitary pharmaceutical form. Suitable excipients such as bulk agents, buffering agents or surfactants can be used. The mentioned formulations will be prepared using usual methods such as those described or referred to in Spanish Pharmacopoeia and the Pharmacopoeia of the United States and in similar reference texts.

The administration of the compounds or compositions used in the present invention can be by any suitable method, such as intravenous infusion, oral preparations and intraperitoneal and intravenous administration. Nevertheless, the preferred administration route will depend on the patient's condition. Oral administration is preferred due to the comfort for the patient and the chronic character of the diseases which are to be treated.

For their application in therapy, the compositions of the invention will preferably be found in pharmaceutically acceptable or substantially pure form, i.e. the compositions of the invention have a pharmaceutically acceptable purity level excluding the pharmaceutically acceptable excipients and not including material considered to be toxic at the normal dosage levels. The purity levels for the inhibitors of acid ceramidase or for the inhibitors of choline kinase preferably exceed 50%, more preferably exceed 70%, more preferable exceed 90%. In a preferred embodiment, they exceed 95%.

The therapeutically effective amounts of the inhibitors of acid ceramidase or of the chemotherapeutic agent, and of the inhibitors of choline kinase alpha isoform in the compositions of the invention will generally depend, among other factors, on the individual who is to be treated, on the severity of the disease said individual suffers from, on the administration form chosen etc. For this reason, the doses mentioned in this invention must be considered as guides for the person skilled in the art and the latter must adjust the doses according to the variables mentioned previously. Nevertheless, an inhibitor of acid ceramidase can be administered once or more times a day, for example, 1, 2, 3 or 4 times a day in a typical daily total amount comprised between 1 and 200 mg/kg body mass/day, preferably 1-10 mg/kg body mass/day. In the same manner an inhibitor of choline kinase can be administered once or more times a day, for example, 1, 2, 3 or 4 times a day in a typical daily total amount comprised between 1 and 200 mg/kg body mass/day, preferably 1-10 mg/kg body mass/day.

The compositions according to the present invention can be formulated as a single preparation or, alternatively, they may be provided as a product for the simultaneous, concurrent, separate or sequential administration.

The compositions described in this invention, their pharmaceutically acceptable salts, prodrugs and/or solvates, as well as the pharmaceutical compositions containing them can be used together with other additional drugs to provide a combination therapy. Said additional drugs can form part of the same pharmaceutical composition or can alternatively be provided in the form of a separate composition for its simultaneous or non-simultaneous administration with the pharmaceutical composition comprising an inhibitor of acid ceramidase and an inhibitor of choline kinase or a pharmaceutically acceptable prodrug, solvate or salt thereof. The other drugs can form part of the same composition or be provided as a separate composition for its administration at the same time or at different times.

The compositions of the invention can be administered in combination with other chemotherapeutic agents known in the art such as
- Antimetabolite agents such as folic acid analogs, pyrimidine analogs, purine analogs, and adenosine deaminase inhibitors, including, but not limited to cytarabine (CYTOSAR-U), cytosine arabinoside, fluorouracil (5-FU), floxuridine (FudR), 6-thioguanine, 6-mercaptopurine (6-MP), pentostatin, methotrexate, 10-propargyl-5, 8-dideazafolate (PDDF, CB3717), 5, 8-dideazatetrahydrofolic acid (DDATHF), leucovorin, fludarabine phosphate, pentostatine, and gemcitabine.
- Suitable natural products and their derivatives, (e. g., vinca alkaloids, antitumor antibiotics, enzymes, lymphokines, and epipodophyllotoxins), include, but are not limited to, Ara-C, paclitaxel (Taxol (k), docetaxel (Taxotere), deoxycoformycin, mitomycin-C, L- asparaginase, azathioprine; brequinar; alkaloids, e. g. vincristine, vinblastine, vinorelbine, vindesine, etc.; podophyllotoxins, e. g. etoposide, teniposide, etc.; antibiotics, e. g. anthracycline, daunorubicin hydrochloride (daunomycin, rubidomycin, cerubidine), idarubicin, doxorubicin, epirubicin and morpholino derivatives, etc.; phenoxizone biscyclopeptides, e. g. dactinomycin; basic glycopeptides, e. g. bleomycin; anthraquinone glycosides, e. g. plicamycin (mithramycin); anthracenediones, e. g. mitoxantrone; azirinopyrrolo indolediones, e. g. mitomycin; macrocyclic immunosuppressants, e. g. cyclosporine, FK-506 (tacrolimus, prograf), rapamycin, etc.; and the like. Other anti-proliferative cytotoxic agents are navelbene, CPT-11, anastrazole, letrazole, capecitabine, reloxafine, cyclophosphamide, ifosamide, and droloxafine.
- Microtubule affecting agents that have antiproliferative activity are also suitable for use and include, but are not limited to, allocolchicine (NSC 406042), Halichondrin B (NSC 609395), colchicine (NSC 757), colchicine derivatives (e. g., NSC 33410), dolstatin 10 (NSC 376128), maytansine (NSC 153858), rhizoxin (NSC 332598), paclitaxel (Taxol), T ol derivatives, docetaxel (Taxotere), thiocolchicine (NSC 361792), trityl cysterin, vinblastine sulfate, vincristine sulfate, natural and synthetic epothilones including but not limited to, eopthilone A, epothilone B, discodermolide; estramustine, nocodazole, and the like.
- Tyrosine kinase inhibitors such as gefitinib, imatinib, sorafenib, dasatinib, and erlotinib.
- Topoisomerase II inhibitors include, but are not limited to, epipodophyllotoxins such as topotecan, irinotecan, etoposide and teniposide.
- Anthracyclines (such as daunorubicin, doxorubicin, epirubicin, idarubicin, mitoxantrone).
- Monoclonal antibodies such as cetuximab, bevacizumab, rituximab, alemtuzumab and trastuzumab.

In addition, in the case of the first composition of the invention, the composition may addditionaly comprise an alkylating agent. Suitable alkylating agents for use in the first composition of the invention include platinum-based compounds such as carboplatin, cisplatin, nedaplatin, oxaliplatin, triplatin tetranitrate, satraplatin and combinations thereof; alkyl sulfonates such as Busulfan, ethyleneimines and methylmelamines such as hexamethylmetamine, altretamine or Thiotepa, nitrogen mustards such as cyclophosphamide, mechlorethamine or mustine, uramustine or uracil mustard, Melphalan, Chlorambucil or Ifosfamide, nitrosoureas such as carmustine or streptozocin, triazenes such as dacarbazine and imidazotetrazines such as temozolomide. In addition, in the case of the first and second compositions of the invention, the compositions may comprise as well a death receptor ligand. Preferably, said death receptor ligand is selected from the group consisting of NGF, CD40L, CD137L/4-1BBL, TNF-α CD134L/OX40L, CD27L/CD70, FasL/CD95, CD30L, TNF-β /LT- a, LT- β and TRAIL. In a preferred embodiment, the TNF family member is TRAIL, a functionally equivalent derivative thereof or a small mimic compound thereof TRAIL (TNF-related apoptosis inducing ligand), also known as "Apo-2 ligand", "Apo-2L", "Apo2L", "Apo2L/TRAIL" and "Apo-2 ligand/TRAIL", is a molecule which is capable of inducing apoptosis in cells expressing the TRAIL cognate receptor. TRAIL was identified several years ago as a member of the TNF family of cytokines, (Pitti et al., 1996, J.Biol.Chem., 271:12687-12690 and US Patent 6,284,236). The full-length native sequence human TRAIL polypeptide is a 281 amino acid long, Type II transmembrane protein. Crystallographic studies of soluble forms of TRAIL reveal a homotrimeric structure similar to the structures of TNF and other related proteins. TRAIL, unlike other TNF family members however, was found to have a unique structural feature in that three cysteine residues (at position 230 of each subunit in the homotrimer) together coordinate a zinc atom, and that the zinc binding is important for trimer stability and biological activity. The present invention contemplates the use of any of the three different TRAIL isoforms (TRAILα, TRAILβ and TRAILγ) o combinations thereof.

Functionally equivalent TRAIL variants include soluble TRAIL isoforms such as those described in WO08088582 and US6284236 or the TRAIL fragments 95-281, 114-281 described in US2002128438, scFv:sTRAIL fusions as described by Bremer et al (Neoplasia, 2004, 6:636-45), alternatively-spliced forms of TRAIL as described in US2002061525, TRAIL-receptor binding peptides as described in WO04101608, TRAIL variants with increased specificity for the pro-apoptotic receptors such as the 19 IL, 199V, 201R, 213W, 215D and/or 193S TRAIL mutants as described in WO07063301 or variants selected by phage-display on receptors as described in WO04001009A, agonistic antibodies directed against TRAIL-cognate receptors TRAIL-R1 (DR4) and TRAIL-R2 (DR5) such as mapatumumab, lexatumumab, the antibodies described in WO07128231, or the antibodies described in WO02094880, the monoclonal antbibody AD5-10 described in WO06017961, TRAIL-specific tandem diabodies and tribodies as described in WO0505660 chimeric anti-DR4 antibodies as described in WO9937684, agonistic anti-DR5 antibodies as described in WO03038043, bispecific anti-TRAIL receptor antibodides as described in WO02085946, anti-DR4 specific antibodies as described in WO9832856, anti-DR2 ScFV as described by Park,K.J et al (Cancer Res., 2007, 67:7327-7334), trimeric TRAIL fusion proteins as described in WO08025516 and WO04014951, dodecameric TRAIL variants as described in WO07102690, pegylated TRAIL as described in WO07145457, DNA vectors comprising a polynucleotide encoding TRAIL such as those described in US2006153809, WO04087930, US2005031593.

Small molecule TRAIL mimics having pro-apoptotic effect include the compounds described in WO2008094319.

### THERAPEUTIC USES OF THE COMPOSITIONS OF THE INVENTION

The compositions of the invention, in view of their synergistic effect in inhibiting growth of tumor cells, can be used in medicine. Thus, in another aspect, the invention relates to the compositions of the invention for use in medicine.

In a another aspect, the invention relates to the use of a composition comprising (i) an inhibitor of acid ceramidase and an inhibitor of choline kinase specific for the choline kinase alpha isoform or, (ii) an inhibitor of choline kinase specific for the choline kinase alpha isoform and a chemotherapeutic agent, wherein the chemotherapy agent is selected from the group consisting of a DNA-alkylating agent, an antimetabolite, a mitotic inhibitor, an anthracycline, a topoisomerase I inhibitor, a topoisomerase II inhibitor, cetuximab, gefitinib and imatinib, for the manufacture of a medicament for the treatment of cancer. Alternatively, the invention relates to a composition comprising (i) an inhibitor of acid ceramidase and an inhibitor of choline kinase specific for the choline kinase alpha isoform or, (ii) an inhibitor of choline kinase specific for the choline kinase alpha isoform and a chemotherapeutic agent , wherein the chemotherapy agent is selected from the group consisting of a DNA-alkylating agent, an antimetabolite, a mitotic inhibitor, an anthracycline, a topoisomerase I inhibitor, a topoisomerase II inhibitor, cetuximab, gefitinib and imatinib, for use in the treatment of cancer.

The invention also contemplates the administration of any of the pharmaceutical compositons of the invention including additional antineoplastic agents as defined above. Preferably, the cancer is selected from the group consisting of heavy chain disease, leukemias (e.g., acute myeloid leukemia, chronic myeloid leukemia, chronic myelomonocytic leukemia, acute promyelocytic leukemia, myelodysplastic syndrome, juvenile myelomonocytic leukemia, etc.), metastases, neoplasms, tumors (e.g., acoustic neuroma, adenocarcinoma, adrenal cortical cancer, anal carcinoma, angiosarcoma, astrocytoma, basal cell carcinoma, bile duct carcinoma, bladder carcinoma, brain cancer, breast cancer, bronchogenic carcinoma, cancer of the peritoneum, cervical cancer, chondrosarcoma, chordoma, choriocarcinoma, colon carcinoma, colorectal cancer, craniopharyngioma, cystadenocarcinoma, embryonal carcinoma, endometrial carcinoma, endotheliosarcoma, ependymoma, epithelial carcinoma, esophageal cancer, Ewing's tumor, fibrosarcoma, gastrointestinal cancer, genitourinary tract cancer , glioblastoma, glioma, head cancer, hemangioblastoma, hepatoma, Hodgkin's Disease, kidney cancer, leiomyosarcoma, liposarcoma, liver cancer, lung carcinoma, lymphangioendotheliosarcoma, lymphangiosarcoma, lymphomas, malignant hypercalcemia, malignant pancreatic insulanoma, medullary carcinoma, medulloblastoma, melanoma, meningioma, mesothelioma, neck cancer, neuroblastoma, non-Hodgkin's lymphoma, non-small cell lung carcinoma, oligodendroglioma, osteogenic sarcoma, ovarian cancer, pancreatic cancer, papillary adenocarcinomas, papillary carcinoma, penile carcinoma, pinealoma, premalignant skin lesions, primary brain tumors, primary macroglobulinemia, primary thrombocytosis, prostate cancer, rectal cancer, renal cell carcinoma, retinoblastoma, rhabdomyosarcoma, salivary gland carcinoma, sarcoma, sebaceous gland carcinoma, seminoma, small cell lung carcinoma, squamous cell carcinoma, stomach cancer, synovioma, sweat gland carcinoma, testicular tumor, thyroid cancer, uterine carcinoma, vulval cancer, and Wilms tumor), or any disease or disorder characterized by uncontrolled cell growth.

In a preferred embodiment, the cancer is lung cancer. "Lung cancer", as used herein, relates to any neoplastic modification affecting one of more cells present in the lung tissue. Exemplary non-limiting types of lung cancer that can be treated using the compositions of the invention include small and non-small cell lung cancer, including squamous cell carcinoma, adenocarcinoma and large cell carcinoma and mesothelioma. In a more preferred embodiment, the lung cancer is non-small cell lung cancer.

In another preferred embodiment, the cancer is colon cancer or colorectal cancer. As used herein, the term "colorectal cancer" includes any type of colon, rectal and appendix neoplasia and refers both to early and late adenomas and to carcinomas as well as to the hereditary, familial or sporadic cancer. Hereditary CRC includes those syndromes which include the presence of polyps, such as the hamartomatous polyposis syndromes and the most known, familial adenomatous polyposis (FAP) as well as nonpolyposis syndromes such as hereditary nonpolyposis colorectal cancer (HNPCC) or Lynch syndrome I. The present invention allows the diagnosis of colorectal cancer in its different stages such as stages A, B, C1, C2 and D according to Dukes' classification, stages A, B1, B2, B3, C1, C2, C3 and D according to the Astler-Coller classification, stages TX, T0, Tis, T1, T2, T3, NX, N0, N1, N2, MX, M0 and M1 according to the TNM system as well as stages 0, I, II, III and IV according to the AJCC (American Joint Committee on Cancer) classification.

The compositions according to the present invention can be formulated as a single preparation or, alternatively, they may be provided as a product for the simultaneous, concurrent, separate or sequential administration.

### USE OF ACID CERAMIDASE INHIBITORS, CHEMOTHERAPEUTIC AGENTS OR DEATH RECEPTOR LIGANDS TO SENSITIZE TUMOR CELLS TO THE TREATMENT WITH ChoK INHIBITORS

The authors of the present invention have also observed that the response to inhibitors of choline kinase is enhanced when the cells are treated previously or simultaneously with an acid ceramidase inhibitor (see example 6 of the present invention), with a chemotherapeutic agent (see examples 7 and 9) or with a death receptor ligand (see example 8).

Thus, in another aspect, it is also disclosed a method for increasing the sensitivity of a tumor cells to a choline kinase inhibitor which comprises treating said-tumor cells with an acid ceramidase inhibitor, with a chemotherapeutic agent or with a death receptor ligand. In yet another aspect, the invention relates to the use of an inhibitor of acid ceramidase, a chemotherapeutic agent or a death receptor ligand to increase the sensitivity of a tumor cell to a choline kinase inhibitor.

The term "sensitivity", as used herein, refers to the response of a cell to a given choline kinase inhibitor and is usually determined as the minimal dosis of the choline kinase inhibitor which causes a 50% inhinbition in the growth of a tumor cell. Thus, the acid ceramidase inhibitor, with a chemotherapeutic agent or with a death receptor ligand act by increasing the sensitivity or decreasing the minimal dosis needed to achieve 50% growth inhibition.

The sensitization of tumor cells to the treatment with ChoK inhibitors can be carried by treating the cells with an acid ceramidase inhibitor, with the chemotherapeutic agent or with the death receptor ligand simultaneously, after or prior to the administration of the ChoK inhibitors. The acid ceramidase inhibitor, the chemotherapeutic agent and the death receptor ligand that can be used to increase the sensitivity of a tumor cell to a ChoK inhibitor can be any of the compounds previously described as forming part of the compositions of the invention. In addition, the ChoK inhibitors that can be used for the treatment of cells which have been sensitized with the acid ceramidase inhibitors or with the death receptor ligand are essentially any of the inhibitors previously described as components of the compositions of the invention.

### METHOD FOR THE IDENTIFICACION OF CANCER PATIENTS SHOWING RESISTANCE TO ChoK INHIBITORS

Moreover, the findings of the authors of the invention open the possibility of identifying cancer patients which are likely to show resistance to the treatment with ChoK inhibitors by determining the levels of acid ceramidase in a sample of said patient. If acid ceramidase levels are increased with respect to a reference sample, this will be indicative that the patients are likely to show resistance to ChoK inhibitors since the pro-apoptotic ceramidase produced in response to ChoK inhibition will be hydrolysed giving rise to sphingosine which has pro-mitotic effects. On the contrary, if the levels of acid ceramidase are decreased or at least not increased with respect to the levels in a reference sample, this is indicative that the patient will respond favourably to the treatment with ChoK inhibitors.

Thus, in another aspect, it is also disclosed a method (hereinafter first method) for the identification of cancer patients resistant to therapy with ChoK inhibitors comprising determining the levels of acid ceramidase in a sample from said patient wherein the patient is identified as being resistant to ChoK inhibitors when the acid ceramidase levels in said sample are higher than a reference sample.

In order to carry out the first method, a sample is obtained from the subject under study. The term "sample" as used herein, relates to any sample which can be obtained from the patient. The present method can be applied to any type of biological sample from a patient, such as a biopsy sample, tissue, cell or fluid (serum, saliva, semen, sputum, cerebral spinal fluid (CSF), tears, mucus, sweat, milk, brain extracts and the like). In a particular embodiment, said sample is a tissue sample or portion thereof, preferably tumour tissue sample or portion thereof. Said sample can be obtained by conventional methods, e.g., biopsy, by using methods well known to those of ordinary skill in the related medical arts. Methods for obtaining the sample from the biopsy include gross apportioning of a mass, or microdissection or other art-known cell-separation methods. Tumour cells can additionally be obtained from fine needle aspiration cytology. In order to simplify conservation and handling of the samples, these can be formalin-fixed and paraffin-embedded or first frozen and then embedded in a cryosolidifiable medium, such as OCT-Compound, through immersion in a highly cryogenic medium that allows for rapid freeze.

Once the sample from the patient is available, the first method comprises the determination of the levels of acid ceramidase. As the person skilled in the art appreciates, the "levels of acid ceramidase" can be determined by measuring the levels of the mRNA coding acid ceramidase, by determining the levels of the acid ceramidase or by measuring the enzymatic activity of acid ceramidase.

In the case that the "expression levels" are determined by measuring the mRNA expression levels acid ceramidase, the biological sample may be treated to physically or mechanically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art, for example, Sambrook, J., et al., 2001. Molecular cloning: a Laboratory Manual, 3rd ed., Cold Spring Harbor Laboratory Press, N.Y., Vol. 1-3. Preferably, care is taken to avoid degradation of the RNA during the extraction process.

In a particular embodiment, the expression level is determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample.

While all techniques of gene expression profiling (RT-PCR, SAGE, or TaqMan) are suitable for use in performing the foregoing aspects of the invention, the gene mRNA expression levels are often determined by reverse transcription polymerase chain reaction (RT-PCR). In a particular embodiment, the expression levels of the acid ceramidase mRNA are determined by quantitative PCR, preferably, Real-Time PCR. The detection can be carried out in individual samples or in tissue microarrays.

In order to normalize the values of mRNA expression among the different samples, it is possible to compare the expression levels of the mRNA of interest in the test samples with the expression of a control RNA. A "Control RNA" as used herein, relates to a RNA whose expression levels do not change or change only in limited amounts in tumour cells with respect to non-tumourigenic cells. Preferably, the control RNA is mRNA corresponding to housekeeping genes and which code for proteins which are constitutively expressed and carry out essential cellular functions. Preferred housekeeping genes for use in the present invention include β-2-microglobulin, ubiquitin, 18-S ribosomal protein, cyclophilin, GAPDH and actin. In a preferred embodiment, the control RNA is β-actin mRNA. In one embodiment relative gene expression quantification is calculated according to the comparative Ct method using β-actin as an endogenous control and commercial RNA controls as calibrators. Final results, are determined according to the formula 2-(ΔCt sample-ΔCt calibrator), where ΔCT values of the calibrator and sample are determined by subtracting the CT value of the target gene from the value of the housekeeping gene.

Once the mRNA expression levels of the acid ceramidase mRNA have been determined, the first method involves comparing the expression levels with those found in a reference sample. By "reference sample", as used herein, it is understood a sample showing reference levels of acid ceramidase mRNA. For instance, the reference sample maybe a tumor sample obtained from a patient similar to the tumor of the patient under study but which is not resistant to ChoK inhibitors. Alternatively, the reference sample may be a pool of tumor tissues samples derived from several patients suffering from the same type of tumor which is under study. Alternatively, it is also possible to determine the mRNA expression levels in a collection of tumor samples and determine a median value from all the individual values. The resulting median value is then used as reference to determine whether the mRNA expression values obtained in the sample under study are considered as increased or not.

Due to inter-subject variability (e.g. aspects relating to age, race, etc.) it is very difficult (if not practically impossible) to establish absolute reference values for the levels of mRNA. Thus, in a particular embodiment, the reference values for "increased" or "decreased" acid ceramidase mRNA levels are determined by calculating percentiles by conventional means involving the testing of a group of samples isolated from normal subj ects (i.e. people with no diagnosis of NSCLC) for the expression levels of the acid ceramidase mRNA. The "increased" levels can then be assigned, preferably, to samples wherein expression levels for the acid ceramidase mRNA are equal to or in excess of percentile 50 in the normal population, including, for example, expression levels equal to or in excess to percentile 60 in the normal population, equal to or in excess to percentile 70 in the normal population, equal to or in excess to percentile 80 in the normal population, equal to or in excess to percentile 90 in the normal population, and equal to or in excess to percentile 95 in the normal population.

Alternatively, in another particular embodiment, the levels of acid ceramidase can be determined by measuring the levels of the acid ceramidase protein. The determination of the expression levels of the proteins can be carried out by immunological techniques such as ELISA, Western Blot or immunofluorescence. Western blot is based on the detection of proteins previously resolved by gel electrophoreses under denaturing conditions and immobilized on a membrane, generally nitrocellulose by the incubation with an antibody specific and a developing system (e.g. chemoluminiscent). The analysis by immunofluorescence requires the use of an antibody specific for the target protein for the analysis of the expression. ELISA is based on the use of antigens or antibodies labelled with enzymes so that the conjugates formed between the target antigen and the labelled antibody results in the formation of enzymatically-active complexes. Since one of the components (the antigen or the labelled antibody) are immobilised on a support, the antibody-antigen complexes are immobilised on the support and thus, it can be detected by the addition of a substrate which is converted by the enzyme to a product which is detectable by, e.g. spectrophotometry or fluorometry.

Alternatively, the determination of the acid ceramidase protein expression levels can be carried out by constructing a tissue microarray (TMA) containing the patient samples assembled, and determining the expression levels of the protein protein by immunohistochemistry techniques. Immunostaining intensity can be evaluated by two different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, and low expression (1+) versus moderate (2+) and high (3+) expression, taking into account the expression in tumoral cells and the specific cut-off for each marker. As a general criterion, the cut-offs were selected in order to facilitate reproducibility, and when possible, to translate biological events.

When an immunological method is used, any antibody or reagent known to bind with high affinity to the target proteins can be used for detecting the amount of target proteins. It is preferred nevertheless the use of antibody, for example polyclonal sera, hybridoma supernatants or monoclonal antibodies, antibody fragments, Fv, Fab, Fab' y F(ab')2, ScFv, diabodies, triabodies, tetrabodies and humanised antibodies.

Irrespective of whether the acid ceramidase levels are determined by measuring the mRNA expression levels or the protein levels, the values obtained need to be compared with those of a reference sample. A reference sample may correspond to a sample obtained by pooling equal amounts from a number of healthy patients. Once this reference sample value is established, the level of this marker expressed in tumor tissues from patients can be compared with this median value, and thus be assigned a level of "low," "normal" or "high". The collection of samples from which the reference level is derived will preferably be constituted from healthy persons from the same age as the patients. In any case it can contain a different number of samples. In a more preferred embodiment, the samples are biopsy brain samples. Preferably the collection should be sufficient to provide an accurate reference level. Preferably the number of samples used for make the reference values is more than 10, 20, 30, 40, 50, 60, 70, 80, 90, 100, 150, 200, 500 samples.

In a particular embodiment, an increase in expression above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "high" expression. In a particular embodiment, a decrease in expression below the reference value of at least 0.9-fold, 0.75-fold, 0.2-fold, 0.1-fold, 0.05-fold, 0.025-fold, 0.02-fold, 0.01-fold, 0.005-fold or even less compared with the reference value is considered as "low" expression.

On the other hand, the determination of the protein expression levels can be carried out by constructing a tissue microarray (TMA) containing the subject samples assembled, and determining the expression levels of the proteins by immunohistochemistry techniques well known in the state of the art.

In another embodiment, the determination of acid ceramidase levels is carried out by the determination of acid ceramidase activity in the sample under study. Methods for the determination of the enzymatic activity of acid ceramidase are abundantly known to the skilled person and have been described in detail above.

### METHOD FOR SELECTING A PERSONALISED THERAPY FOR A PATIENT SUFFERING FROM CANCER

The results provided by the authors of the present invention allow the identification of those patients suffering from cancer which are going to show resistance to the treatment with ChoK inhibitors based on the expression levels of acid ceramidase in a sample from the patient. Thus, in a further aspect, it is also disclosed a method (hereinafter second method) for selecting a personalised therapy for a patient suffering from cancer comprising determining the levels of acid ceramidase in a sample from said patient wherein if the expression levels of acid ceramidase in said sample are higher than in the reference sample, the patient is candidate for being treated with a a ChoK inhibitor or a with a combination of a ChoK inhibitor and an acid ceramidase inhibitor.

The steps of the second method are essentially as described in the first method and includes the determination of the acid ceramidase levels in a sample from the patient (preferably a tumor sample) wherein said levels can be determined by either determining the mRNA levels, the protein levels or the acid ceramidase activity using any of the method previously described.

Once the levels of acid ceramidase are determined and compared to a reference sample, higher acid ceramidase levels than those found in the reference sample will be indicative that the patient is candidate for being treated with a combination of a ChoK inhibitor and an acid ceramidase inhibitor.

In a preferred embodiment, the cancer is non-small cell lung cancer.

### METHOD FOR THE IDENTIFICATION OF COMPOUNDS THAT INCREASE THE THERAPEUTIC EFFECT OF ChoK INHIBITORS

The authors of the present invention have observed that tumor cells showing increased resistance to ChoK inhibitors show increased levels of acid ceramidase. Thus, by measuring the levels of increase in acid ceramidase in response to a given compound, it will be possible to determine whether this compound is capable of decreasing resistance to ChoK inhibitors, i.e. to increase the therapeutic effect of these compounds.

Thus, in another aspect, it is also disclosed a method (hereinafter third method of the invention) for the identification of compounds capable of increasing the therapeutic effect of a ChoK inhibitor for the treatment of cancer comprising the steps of
(i) contacting a tumor cell showing resistance to ChoK inhibitors with a candidate compound and
(ii) determining in said cell the levels of acid ceramidase
wherein if the levels of acid ceramidase in the cell after having being treated with a candidate compound are lower than before the treatment, then the candidate compound is considered to be able to increase the effect of ChoK inhibitors for the treatment of cancer.

The third method comprises a first step which consists on contacting a tumor cell showing resistance to ChoK inhibitors with a candidate compound. It will be appreciated that said contacting step can be carried out in vivo in a non-human animal which contains a tumor formed by cells resistant to ChoK inhibitors or can be carried out in vitro on a culture of cells showing resistance to ChoK inhibitors.

When the third method is carried out in vitro, a cell culture of tumor cells resistant to ChoK inhibitors is required. The cultures can be obtained from tumor cells which have been previously selected based on their resistance to ChoK inhibitors, from tumor cells previously subjected to one or more rounds of selection with increasing concentrations of a ChoK inhibitor, with cells which overexpress ChoK or with cells that constitutively express siRNA specific for ChoK. If the cells are selected by one or more rounds of selection with increasing concentrations of ChoK inhibitors, any of the ChoK inhibitors mentioned in the previous paragraphs will be adequate for this purpose.

Once a culture of ChoK-inhibitor resistant cells is established, the culture is put into contact with the candidate compound whose effect on increasing ChoK inhibitor therapeutic effect is to be measured. According to the invention, "putting in contact" a cell with the candidate compound includes any possible way of taking the candidate compound inside the cell expressing the DNA construct. Thus, in the event that the candidate compound is a molecule with low molecular weight, it is enough to add said molecule to the culture medium. In the event that the candidate compound is a molecule with a high molecular weight (for example, biological polymers such as a nucleic acid or a protein), it is necessary to provide the means so that this molecule can access the cell interior. In the event that the candidate molecule is a nucleic acid, conventional transfection means can be applied using any of the methods known in the art (calcium phosphate, DEAE-dextran, polybrene, electroporation, microinjection, liposome-mediated fusion, lipofection, infection by retrovirus and biolistic transfection). In case that the candidate compound is a protein, the cell can be put in contact with the protein directly or with the nucleic acid encoding it coupled to elements allowing its transcription / translation once they are in the cell interior. Alternatively, it is possible to put the cell in contact with a variant of the protein to be studied which has been modified with a peptide which can promote the translocation of the protein to the cell interior, such as the Tat peptide derived from the HIV-1 TAT protein, the third helix of the Antennapedia homeodomain protein from D.melanogaster, the VP22 protein of the herpes simplex virus and arginine oligomers (Lindgren, A. et al., 2000, Trends Pharmacol. Sci, 21:99-103, Schwarze, S.R. et al., 2000, Trends Pharmacol. Sci., 21:45-48, Lundberg, M et al., 2003, Mol. Therapy 8:143-150 and Snyder, E.L. and Dowdy, S.F., 2004, Pharm. Res. 21:389-393).

The compound to be assayed is preferably not isolated but forms part of a more or less complex mixture derived from a natural source or forming part of a library of compounds. Examples of libraries of compounds which can be assayed according to the method of the present invention include, but are not limited to, libraries of peptides including both peptides and peptide analogs comprising D-amino acids or peptides comprising non-peptide bonds, libraries of nucleic acids including nucleic acids with phosphothioate type non-phosphodiester bonds or peptide nucleic acids, libraries of antibodies, of carbohydrates, of compounds with a low molecular weight, preferably organic molecules, of peptide mimetics and the like. In the event that a library of organic compounds with a low molecular weight is used, the library can have been preselected so that it contains compounds which can access the cell interior more easily. The compounds can thus be selected based on certain parameters such as size, lipophilicity, hydrophilicity, capacity to form hydrogen bonds.

The compounds to be assayed can alternatively form part of an extract obtained from a natural source. The natural source can be an animal, plant source obtained from any environment, including but not limited to extracts of land, air, marine organisms and the like.

In a second step, the third method comprises the determination of the acid ceramidase levels of the cells treated with the candidate compound. The determination of the acid ceramidase levels can be performed, as previously described, by determining the mRNA levels, the protein levels or the acid ceramidase activity in extracts of the cells using any of the biochemical methods previously described. Those compounds which lead to a decrease in the levels of acid ceramidase will be selected as candidate componds for increasing the response of tumor cells to ChoK inhibitors.

In the event that the candidate compound forms part of a more or less complex mixture, the invention additionally comprises one or several steps (iii) of fractioning said mixture and the repetition of steps (i), (ii) and (iii) of the method of the invention a variable number of times until the compound of the mixture responsible for the transcription promoting activity is isolated. Methods for fractioning the compounds present in a mixture include chromatography (thin layer, gas, gel molecular exclusion, affinity chromatography) crystallization, distillation, filtration, precipitation, sublimation, extraction, evaporation, centrifugation, mass spectroscopy, adsorption and the like.

In another embodiment, the screening method is carried out in vivo in an animal model of cancer obtained by implanting into a non-human animal tumor cells showing a resistance to the ChoK inhibitor. The cells can be obtained using any of the methods previously mentioned. The ChoK inhibitor-resistant cells can be implanted into any non-human animal of any species, preferably mammals and, more preferably, primate (monkey, baboon, chimpanzee and the like), rodent (mouse, rat, rabbit, guinea pig, hamster, and the like) or a pig. In order to facilitate implantation of the tumor cells, the animal may be an immunodeficient animal. Tumor cells that can be implanted into the recipient organism is includes circulating tumour cells, tumour stem cells, cell lines derived from the immortalisation of circulating tumour cells, micrometastatic tumour cells, cell lines derived from the immortalisation of micrometastatic tumour cells, cell lines derived from immortalized tumour cells that had been previously purified from solid tumours, primary tumour cells from solid tumours, a piece of fresh tumour that has been resected from a solid tumour, primary tumour cells, cell lines derived from immortalized cells that had been previously purified from clinical metastasis (i.e. the PC3 cell line) and any combination of any of those.

Once the animal model carrying a tumor formed by ChoK inhibitor-resistant cells is obtained, the first step of the method comprising contacting said tumor cells with a candidate compound. The contacting step is carried out by administering the candidate compound to the animal under conditions adequate for the compound to access the tumor cells. The administration of the test compounds can be performed by any suitable route, including, for example, oral, transdermal, intravenous, infusion, intramuscular, etc. administration.

Once the tumor has been contacted with the candidate compound, the expression levels of acid ceramidase in said tumor cells is determined as previously described, i.e. by determining acid ceramidase mRNA levels, acid ceramidase protein levels or acid ceramidase activity.

The third method of the invention involves comparing the levels of acid ceramidase in the tumor cells after treatment with the candidate compound than the levels observed prior to the treatment. As used herein, it is considered that the acid ceramidase are lower when they show a decrease of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, i.e the acid ceramidase levels are indetectable.

The invention is now described in detail by means of the following methods and examples which are to be construed as merely illustrative and not limitative of the scope of the invention.

### EXAMPLES

### MATERIAL AND METHODS

### Patients

Specimens of lung cancer tissue from 84 randomly selected patients who underwent surgical resection of NSCLC between 2001 and 2004 and that were followed up by The Medical Oncology division at La Paz Hospital in Madrid were used for this analysis. No adjuvant therapy was administrated to these patients. The study was approved by the institutional review board of the hospitals, and written informed consent was obtained from all patients.

### Primary cultures of NSCLC tumours

Resected tissues from NSCLC patients were dissociated (Cell dissociation sieve-tissued grinder Kit CD1, SIGMA), and the obtained cells were seeded in 24 well plates (BD, Falcon, Bioscience, San Jose, CA, USA). Cells were treated with increasing concentrations (0, 0.5, 1, 5, 10 and 20µM) of cDDP, Taxol, Vinolrelbine, Gemcitabine and MN58b for 10 days in DMEN:F12HAM (Ref:D8437, SIGMA) supplemented with 10% Fetal Bovine Serum (FBS, Life Technologies, Grand Island, NY). The final persistent population in each well was quantified by the method of Cristal Violet as previously described (Rodriguez-González, A. et al., Oncogene, 22:8803-8812).

### Compounds

MN58b has been described in WO9805644 and corresponds to 1,4-(4-4'-Bis-((4-(dimethylamine)pyridinium-1-yl) methyl)diphenyl)butane dibromide. RSM-932A has been described in US patent application US2007185170 and corresponds to 1,1'-(biphenyl-4, 4'-diylmethylene) bis [4 - (4 - chloro - N - methylanilino-) quinolinium]dibromide. NOE has been described by Sugita et al (Biochim.Biphys.Acta, 1975, 398:125-131) and corresponds to N-oleoylethanolamine (NOE). D-NMAPPD corresponds to 3-ethoxy-1-(2-methylaminoethyl)-3-phenyl-indol-2-one (CAS 35922-06-6) and has been described by Raisova, M., et al. (FEBS Lett., 2002, 516:47-52) and Selzner, M. et al. (Cancer Res., 2001, 61:1233-1240). TRAIL has been described previously and corresponds to the extracellular domain of human TRAIL (amino acids 95-281).

### RNA isolation and Gene Expression Analysis

The RNAs from the biopsies selected were isolated for microarrays and QT-PCR, using RNeasy Mini Kit (QIAGEN, Hilden, Germany) following the instructions of the manufactures. Samples were prepared and the array hybridized according to the Affymetrix GeneChip Expression Analysis Technical Manual. Hybridization to Affymetrix U133plus2 GeneChips (54,614 probe sets, representing 47,000 transcripts), staining, washing and scanning procedures were carried out at the Genomic Facility in the National Center of Biotechnology (Madrid, Spain) as described in www.affymetrix.com (Affymetrix, Santa Clara, CA). The Signal Log Ratio estimates the magnitude and direction of change of a transcript. The log scale used is base 2, thus a Signal Log Ratio of 1.0 indicates an increase of the transcript level by 2 fold and -1.0 indicates a decrease by 2 fold. A Signal Log Ratio of zero would indicate no change.

Genes were classified according to biological processes by using Ingenuity Pathway Software (IPA, Ingenuity Systems, www.ingenuity.com). Differentially regulated genes between Resistant and Sensitive were overlaid onto a global molecular network developed from information contained in the Ingenuity Pathways Knowledge Base. Networks of the differentially expressed genes were then algorithmically generated based on their connectivity and the final created network is a graphical representation of the molecular relationships between genes. All depicted connections are supported from published references, books or from canonical information stored in the Ingenuity Pathways Knowledge Base (Sorensen G, BMC Genomics, 2008, 9:114; Kim SY et al., Stat. Methods Med. Res., 2006, 15:3-20).

### Quantitative real-time PCR validation of the microarray analysis

1 µg of RNA was used to generate cDNA using High-Capacity cDNA Archive Kit (Applied Biosystems), and quantitative real-time PCR was carried out in triplicate using the ABI PRISM 7700 Sequence Detector (Applied Biosystems). GAPDH and 18S ribosomal mRNA were amplified as internal controls. Probes used for amplification were from Applied Biosystems as Taqman Gene Expression Assays (ASAH1: HS00602774_M1 Taqman Probe, ASAH2: HS00184096_M1 Taqman Probe and ASAH3: HS00370322_M1 Taqman Probe, DUT: HS00798995_S1 Taqman Probe, TYMS: HS00426591_M1 Taqman Probe, UPP1: HS00427695_M1 Taqman Probe, RRM2: HS00357247_G1 Taqman Probe). The 2-^{ΔΔCt} method was used (Livak KJ., Methods. 2001; 25:402-8) to calculate the relative expression of each gene.

### Cell culture and Generation of Resistant Cell Lines to ChoK inhibitors

All cell lines used in this study were maintained under standard conditions of temperature (37°C), humidity (95%), and carbon dioxide (5%). Human primary bronchial epithelial cells, NHBE (BEC) (Cambrex, CC-2541) were grown in BEGM (Bronchial Epithelial cell growth media) BulletKit (Cambrex, CC-3170). Human primary mammary epithelial cells, HMEC (Clonetics, CC-2551) were grown in MEBM medium supplemented with a bullet kit (Clonetics, CC-3150). Epithelial non-small lung cancer cells H460 and H1299, and small cell lung cancer cell lines H510 and H82 were maintained in RPMI supplemented with 10% Fetal Bovine Serum (FBS) (Life Technologies, Grand Island, NY).

Cell lines resistant to MN58b and RSM-932A (identified as MN58R and RSM-932A-R, respectively) were generated by prolonged continuous exposure to increasing concentrations of each drug. A parallel control (H460 stock) of the cell line in the absence of the compounds was kept in culture for the same time.

### Cell proliferation assays

Cells were seeded on 96-well plates (BD, Falcon, Bioscience, San Jose, CA, USA) at a density of 6000 cells/well, and incubated for 24 h under standard conditions. Then, cells were treated with different concentrations of the ChoK inhibitors (quadruplicates of each concentration) and maintained for 72 hours. Quantification of the number of cells remaining in each well was carried out by the MTT (3-(4,5-dimethylthiazol-2-yl)-2,5-diphenyltetrazolium bromide) method. Absorbance is read at 595nm in a VersaMax Microplate Reader (Molecular Devices, Sunnyvale, CA, USA). For sensitization with acid ceramidase inhibitor, cells were pre-treated 4 hours with the correspondent IC50 for NOE before treatment with ChoK inhibitors. NOE (N-Oleoyl ethanolamine) was obtained from Calbiochem (La Jolla, CA, USA).

### Xenografts and in vivo tumor growth inhibition assays

Indicated cells were resuspended in DMEM just before inoculation (10⁶ cells/0.1 ml), and injected subcutaneously in nu/nu immunosuppressed mice. Mice were kept under standard laboratory conditions according to the guidelines of the Spanish Goverment. When tumours reached a mean volume of 0.1 cm³, mice were randomized to control and treated groups. Treatments with the antitumoral agents (and vehicle for control mice) were performed i.p. with following the indicated schedules. Tumors were monitored three times a week by measuring the major (D) and minor (d) diameters, and tumour volume was calculated by V=(D*d²)/2. Stadistical analysis of significant changes in tumour growth was calculated using SPSS software v. 13.0.

### Apoptosis detection using Annexin V

Cell death was analysed by flow cytometry using de Aposcreen Annexin V Apoptosis Kit (Southern Biotech) according to manufacturer procedures. Cells were seeded at 4x10⁵ cells per well in six-well. Twenty-four hours later, cells were treated with the indicated compounds. Cells were rinsed twice in cold PBS and after removing it cells were resuspended in cold 1X binding buffer to a concentration from 1x10⁶ to 1x10⁷ cells/mL. 100 µl of these cells were collected in a flow cytometry tube and 10 µl of Annexin V were added. After gently vortexing, tubes were incubated for 15 minutes on ice, protected from light. Without washing, 380 µl of 1X binding buffer were added to each sample prior to 10 µl of propidium iodine. Cells were immediately analysed by flow cytometry.

### Cell cycle analysis by FACS

Cell cycle analysis was performed on cells (1X 106) fixed in 70% ethanol and stained with 4 µg/mL propidium iodide, using a Beckton Dickinson FACs SCAN. The cytometry data were analyzed using the Cell Quest analysis program (Becton Dickinson) and FlowJo software.

### Determination of intracellular ceramide levels

Cells were seeded at 1x10⁵ cells per well in six-well plates and lipids were labelled with [¹⁴C]-Serine for two days. Cells were treated without replacing the labelled medium for 24 hours. Then lipids were extracted and analysed by TLC using a previously described protocol (van Echten-Deckert, G. (2000) Sphingolipid extraction and analysis by thin-layer chromatography. Methods Enzymol, 312, 64-79). Prior to lipid extraction, cells were kept at -20 °C. Cells were harvested in methanol and lipids were extracted in a final ratio of chloroform-methanol-water of 60:30:6 (v/v/v). The organic phase was dried in a concentrator under N₂ flow at 37 °C. Samples were resuspended in chloroform-methanol (1:1 v/v) and applied on TLC plates. Lipids were separated using a solvent mixture of chloroform-methanol-2M Ammonium (60:45:4, v/v/v). Quantification was carried out by Instantimager (Pakard, Meriden, CT, USA).

### LC-MS ceramide and dihydroceramide analysis:

Cells were seeded at a density of 8x10⁵ cells per p100 plate. Twenty-four hours later, cells were treated with the indicated compounds. Then, cells were washed in PBS, collected by brief trypsinization. An aliquot of cells was taken for protein measurements. Sphingolipid extracts, fortified with internal standards (*N*-dodecanoylsphingosine, *N-*dodecanoylglucosylsphingosine and N-dodecanoyl sphingosylphosphorylcholine, 0.5 nmol each), were prepared and analysed. The liquid chromatography-mass spectrometer consisted of a Waters Aquity UPLC system connected to a Waters LCT Premier orthogonal accelerated time of flight mass spectrometer (Waters, Millford, MA), operated in positive electrospray ionisation mode. Full scan spectra from 50 to 1500 Da were acquired and individual spectra were summed to produce data points each 0.2 s. Mass accuracy and reproducibility were maintained by using an independent reference spray via the LockSpray interference. The analytical column was a 100 mm × 2.1mm id, 1.7 µm C8 Acquity UPLC BEH (Waters). The two mobile phases were phase A: MeOH/H₂O/HCOOH (74:25:1 v/v/v); phase B: MeOH/HCOOH (99/1 v/v), both also contained 5 mM ammonium formate. A gradient was programmed-0.0 min, 80% B; 3 min, 90% B; 6 min, 90% B; 15 min, 99% B; 18 min, 99% B; 20 min, 80% B. The flow rate was 0.3 mL min⁻¹. The column was held at 30 °C. Quantification was carried out using the extracted ion chromatogram of each compound, using 50 mDa windows. The linear dynamic range was determined by injecting standard mixtures. Positive identification of compounds was based on the accurate mass measurement with an error <5 ppm and its LC retention time, compared to that of a standard (±2%).

### Determination of protein expression by Western blotting

Western blot analysis of equal amounts of cell lysates (30 µg) was performed using each correspondent antibody. Proteins were resolved by electrophoresis onto 10% SDS-PAGE gels and transferred to nitro-cellulose. Blots were blocked for 2h in 5% non-fat dried milk in T-TBS. Determination of ASAH1 was carried out using a monoclonal antibody (1:250) obtained from BD Transduction Laboratories (Ref. 6123012). As loading control, blots were assayed against α-tubulin (Sigma T9026). Determination of caspase-3 and PARP was carried out using anti-caspase-3 and anti-PARP antibodies (Santa Cruz Biotechnology, Santa Cruz, CA).

### Combination index assay

MTT growth assays as described above were used to evaluate cisplatin in combination with ChoK inhibitors. Following an overnight incubation, cisplatin was added in varying concentrations and incubated for 3h. ChoK inhibitors in growing concentrations were then added for 40h, and afterwards, cells were cultured for additional 24 hours in fresh culture medium. The results from combination assays in terms of synergy, additivity or antagonism were analyzed using the isobologram combination index method of Chou Talalay (Chou TC. et al., Trends Pharmacol Sci, 1983, 4:450-4). The ranges of CI are established from those already described (Chou TC. et al., *supra*.). Combination indices (CI) < 1 are indicative of synergistic interactions between the two agents, additive interactions are indicated by CI = 1, and CI > 1 indicates antagonism between them. Cooperative effects are considered as interactions with a CI < 1.0. All experiments were performed in quadruplicate.

### Statistical Analysis

The correlation of event rates was determined using the standard Pearson chi-square statistic. All reported P-values are two-sided. Statistical significance was defined as P<0.05. The statistical analyses were performed using SPSS software, version 13.0 (Inc, Chicago, Illinois).

### EXAMPLE 1

### Intrinsic drug resistance to ChoK inhibition by MN58b in patients with NSCLC

In order to identify the putative mechanism of drug resistance to ChoK specific inhibition by MN58b, we performed a preclinical study in 84 patients with non-small cell lung cancer (NSCLC) from La Paz Hospital in Madrid (Spain). To that end, primary cultures from resected tumours of these patients were established and cultivated for 10 days, in which they were treated with increasing concentrations of the ChoK specific inhibitor MN58b up to 20µM, a concentration that represents 7 to 50 times the IC50 for several tumour-derived cells lines generated from human lung tumours (see Table 6). As shown in Figure 1, different responses to this treatment were observed. On one hand, a set of 39 (46,4%) samples were fully resistant to MN58b, since nearly 100% of the cells remained alive at the maximum concentration of the drug at day 10. On the other hand, the other 45 tumours (53,6%) were sensitive to the antiproliferative effect of MN58b. Among them, a group of 15 samples were considered highly sensitive to MN58b (33,3%), since cell viability was totally abolished even at low concentrations of the drug at day 10. Finally, a group of 30 samples (66,7%) were considered partially sensitive to MN58b, remaining around 50% of cells alive at the end of treatment.

In addition, primary cultures of tumours of these patients were also treated with conventional therapies used in NSCLC (Belani CP, Lung Cancer. 2005, 50 Suppl 2: S3-8). Thus, cell viability of 62 samples of these patients were treated with cisplatin, the same 62 samples with taxol, 52 of these samples were also treated with gemcitabine, and 39 with vinorelbine. As shown in Table 3, MN58b was found the most efficient anticancer drug under these conditions, since 55,5% were sensitive to the drug, followed by cisplatin with 50% of the tumors responding.

**Table 3. Incidence of resistance of NSLCL tumours to several chemotherapeutic agents.**

| **DRUG** | **TOTAL SAMPLES** | **SENSITIVES** | **RESISTANT** |
|---|---|---|---|
| MN58b | 63 | 35 (55%) | 28 (44.4%) |
| cDDP | 62 | 31 (50%) | 31 (50%) |
| Taxol | 62 | 27 (43.5%) | 35 (56.5%) |
| Vinolrelbine | 39 | 15 (38.5%) | 24 (61.5%) |
| Gemcitabine | 52 | 18 (34.6%) | 34 (65.4%) |

Furthermore, a statistical analysis for correlation of resistance among the different drugs was performed. Resistance to cisplatin was significantly associated with resistance to taxol, vinorelbine and gemcitabine. Similar results were also observed when analyzing resistance to taxol. In addition, resistance to vinorelbine was associated with resistance to cisplatin and taxol, and resistance to gemcitabine was associated with resistance to taxol. However, the only drug whose response was not associated to resistance to any other chemotherapeutic agent was MN58b (Table 4).

**Table 4. Correlation among resistance to the chemotherapeutic agents in tumours from patients with NSCLC**

| | **Correlations** | **MN58b** | **cDDP** | **Taxol** | **Vinorelbine** | **Gemcitabine** |
|---|---|---|---|---|---|---|
| **MN58b** | Pearson's Correlation | 1 | -0.096 | -0.09 | -0.124 | 0.133 |
| | Sig. (bilateral) | | 0.46 | 0.488 | 0.45 | 0.346 |
| | N | 63 | 62 | 62 | 39 | 52 |
| **cDDP** | Pearson's Correlation | -0.096 | 1 | 0.319(*) | 0.469(*) | 0.074 |
| | Sig. (bilateral) | 0.46 | | 0.012 | 0.003 | 0.603 |
| | N | 62 | 62 | 62 | 39 | 52 |
| **Taxol** | Pearson's Correlation | -0.09 | 0.319(*) | 1 | 0.421(*) | 0.364(*) |
| | Sig. (bilateral) | 0.488 | 0.012 | | 0.008 | 0.008 |
| | N | 62 | 62 | 62 | 39 | 52 |
| **Vinorelbine** | Pearson's Correlation | -0.124 | 0.469(*) | 0.421(**) | 1 | 0.174 |
| | Sig. (bilateral) | 0.45 | 0.003 | 0.008 | | 0.304 |
| | N | 39 | 39 | 39 | 39 | 37 |
| **Gemcitabine** | Pearson's Correlation | 0.133 | 0.074 | 0.364 | 0.174 | 1 |
| | Sig. (bilateral) | 0.346 | 0.603 | 0.008 | 0.304 | |
| | N | 52 | 52 | 52 | 37 | 52 |

| | | | | | | |
|---|---|---|---|---|---|---|
| * Statistical Significance (bilateral) at 0.05 ** Statistical Significance (bilateral) at 0.01 | | | | | | |

These results suggest that patients with NSCLC who do not respond to any of these antitumoral treatments could efficiently respond to a therapy based on ChoK inhibition since its mechanism for resistance is different than for the other four drugs investigated. On the other hand, these results also suggest the existence of a particular chemoresistance system for ChoK inhibition.

### EXAMPLE 2

### Identification of the mechanism of drug resistance to ChoK inhibition in NSCLC

In order to study the genetic difference of tumours that were intrinsically resistant to ChoK inhibition from those that were sensitive, we analyzed the transcriptional profile of tumours from representative patients with NSCLC. We used the Affymetrix Gene Chip Human Genome HG-U133 plus 2 microarrays to compare a group of 5 patients with resistant tumours to MN58b, versus another group of 5 patients with highly sensitive tumours to this treatment. This microarray platform contains 54.614 probe sets, representing 47.000 transcripts. Considering a, -2 ≤ Fold Change ≥ 2 (-1≤ signal ratio ≥ 1), 912 eligible transcripts showed significant differential regulation in resistant tumour samples compared with the responders. To interpret the biological significance of differentially expressed genes, a gene ontology analysis was conducted using Ingenuity Pathways Analysis (IPA, Ingenuity Systems) (Sorensen G., BMC Genomics. 2008, 9:114).

Interactions between those genes found regulated were further investigated and found 32 networks differentially modulated in responders with scores assigned by the software over 20, indicating the relevance of these genes in the input dataset and a good connectivity among them. Top pathways indicate that the main functions of the genes involved in these networks are related to cell cycle, cell death, cancer, immune response, lipid metabolism and drug metabolism.

To avoid false positive rate due to the high number of transcripts present in the microarray, we used a second statistic analysis known as "B", which allows further filtering genes whose differential expression is statistically significant within the whole experiment (Kim SY., Stat Methods Med Res., 2006, 15:3-20). In this experiment, the use of human samples made this analysis highly restrictive, founding 50 genes fulfilling the criteria (B > 0). Eighteen genes were coincident in both analyses (two-fold differential expression, and B > 0), being four of them over expressed in the samples considered resistant to MN58b, and 14 down-modulated. Among these genes, acid ceramidase (ASAH1) was significatively upregulated in resistant samples following these two analyses.

### EXAMPLE 3

### Acid Ceramidase expression in MN58b-resistant tumor NSCLC cell lines

As mentioned above, acid ceramidase (ASAH1), an enzyme involved in the lipid metabolism was found significantly over-expressed according to any selection criteria in those tumours that were resistant to MN58b.

The behaviour of the different ceramidases in MN58b NSCLC-resistant cell lines were studied in detail by microarray. As it is shown in Table 5, the ceramidase that is modulated in resistant NSCLC tumours to MN58b is only acid ceramidase. In addition, we have observed that, though not in a significant manner following B statistic, an identified enzyme called acid ceramidase like that seems to have the same localization and function than acid ceramidase, is also up-regulated in resistant samples (Table 5).

**Table 5. Gene expression profile of the different ceramidases as determined in the microarray analysis.**

| TYPES | LOCATION | Microarrays |
|---|---|---|
| **ASAH1** | Lysosome | 1.94 |
| (Acid ceramidase EC 3.5.1.23) | | (2.42*/1.72*/1.70*) |
| **ASAH2** | Cell membrane (Mitochondria) | NC |
| (Neutral ceramidase EC 3.5.1.23) | | (1.03) |
| **ASAH3** | Endoplasmic Reticulum | NC |
| (Alkaline ceramidase EC 3.5.1.23) | | (1.15) |
| **ASAHL** | Lysosome | 1.94 |
| (Acid ceramidase like EC 3.5.1.-) | | (2.09/1.78) |

| | | |
|---|---|---|
| Medium value of gene expression. Data or the different probes present in the microarray. NC: no differential changes. * Statistical significant. | | |

To validate that changes observed in the microarray analysis correspond to real changes in ASAH1 gene expression, a quantitative real time-PCR using a specific taqman probe on this gene was performed (Applied Biosystems assay Hs00602774_m1), and on the neutral and alkaline ceramidases (Applied Biosystems respective assays Hs00184096_m1 and Hs00370322_m1) as negative controls. To that end, both the 5 samples of resistant and responder tumours used for the microarray analysis, as well as a new batch of 5 additional patient samples in each case were used for this analysis. Real time-PCR revealed that ASAH1, but not ASAH2 or ASAH3, was differentially expressed in resistant tumours confirming the results obtained in the microarrays analysis (Figure 2). These results verify that acid ceramidase is specifically up-regulated in patients with tumours resistant to ChoK inhibition and is the basis for the proposed model for a mechansim of resistance to ChoK inhibition (Figure 3). Thus, inhibition of ChoK activity by specific inhibitors (ChoKI) induces a reduction in the levels of PCho. As a consequence, an alternative pathway for the generation of PCho is activated which consist in the increaesd activity of sphingomyelinase (SMLase). This enzyme generates both PCho and ceramides. This latter metabolite is a potent inducer of cell death. Resistance to the action of ChoKI can be produced if the activity of the enzyme responsible for the cnversion of ceramides into esphingosine is increased. This enzyme is acid ceramidase (ASAH1) (Figure 3).

### EXAMPLE 4

### Inhibition of acid ceramidase sensitizes NSCLC cells to ChoK inhibitors

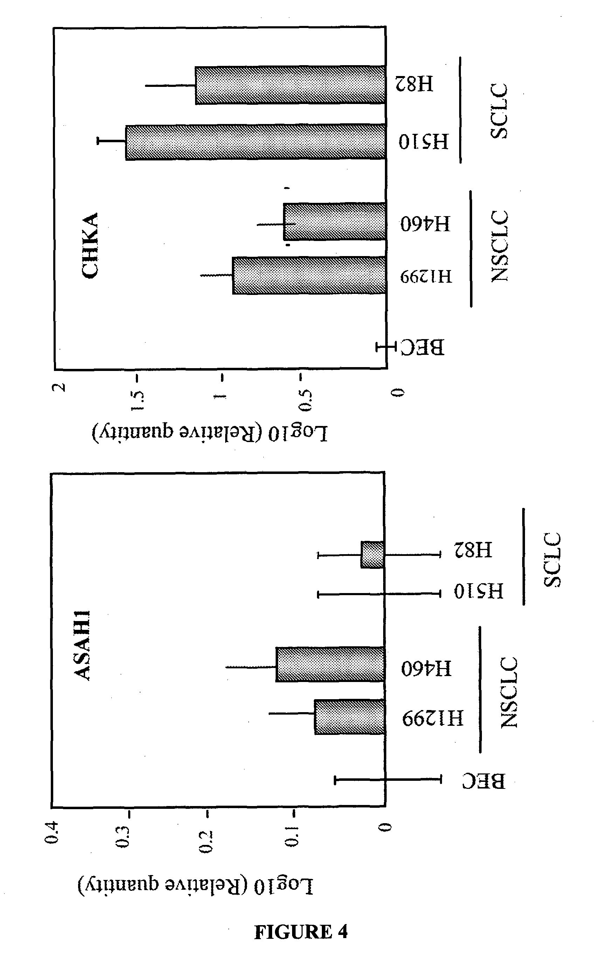

First, the levels of ASAH1 in a set of four human lung tumours cell lines (H460 and H1299 as NSCLC cell lines and H510 and H82 as SCLC) were investigated. As shown in Figure 4, SCLC derived-cell lines displayed levels of acid ceramidase similar to those found in senescent control BEC cells, and much lower than the levels found in NSCLC cells. Interestingly, these SCLC cell lines also displayed the highest levels of choline kinase alpha, and were much more sensitive to ChoK inhibition than the NSCLC cells (Table 6), suggesting that low levels of acid ceramidase could account for at least part of the extremely high response to ChoK inhibitors of the SCLC cells.

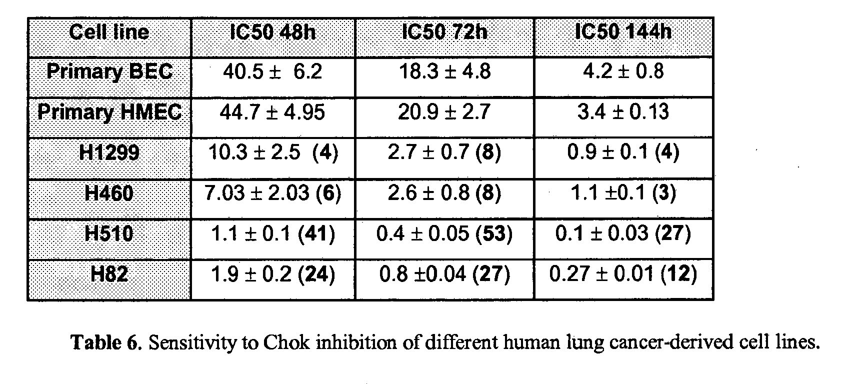

Thus, disruption in the levels of acid ceramidase may allow ChoK inhibitors to have a better effect in these cells. To verify this hypothesis, we used the previously characterized acid ceramidase inhibitor, N-Oleoylethanolamine (NOE) (Grijalvo S., Chem Phys Lipids. 2006; 144:69-84), to investigate if the inhibition of acid ceramidase sensitizes tumour cell lines to ChoK inhibitors. To that end, H460 cells were pre-treated with NOE for three hours to inhibit acid ceramidase. Then, cells were treated with increasing concentrations of ChoKα inhibitors MN58b and RSM-932A and the concentration in which 50% of the total cell population was affected (IC50) was determined. As shown in Table 7, pre-treatment of H460 cells with the acid ceramidase inhibitor NOE, sensitized cells to ChoKα inhibition. These results are consistent with our assumption that increased levels of acid ceramidase may confer a mechanism of drug resistance to ChoK inhibition.

**Table 7.- Inhibition of acid ceramidase with NOE sensitizes human NSCLC cells to ChoK inhibition (4,7 fold induction for MN58b and 3,2 fold for RSM-932A).**

| **H460** | **NOE** | **MN58b** | **NOE + MN58b** | **RSM-932A** | **NOE + RSM-932A** |
|---|---|---|---|---|---|
| **IC₅₀ 72h** | 32,5 µM | 0,28 µM | 0,06 µM | 1,11 µM | 0,35 µM |

With the aim of validating these results, we performed additional experiments using another known acid ceramidase inhibitor, CAY10466 (CAY) (D-NMAPPD (1R,2R)-B13; 10006305 Cayman Chemical) (item VIII in Table 2) and an alkaline ceramidase inhibitor as a negative control D-erythro-MAPP (DMP) (10165 Cayman Chemical). H460 cells were pre-treated with these compounds for three hours to inhibit ceramidases. Then, cells were treated with increasing concentrations of ChoKα inhibitors MN58b and RSM-932A and the concentration in which 50% of the total cell population was affected (IC50) was determined. As shown in Table 8, pre-treatment of H460 cells with the acid ceramidase inhibitor D-NMAPPD, but not D-erytro-MAPP, sensitized cells to ChoKα inhibition. These results are consistent with our assumption that increased levels of acid ceramidase may confer a mechanism of drug resistance to ChoK inhibition. Furthermore, the combined use of ChoK inhibitors and acid ceramidase inhibitors provides a further stronger anticancer activity.

**Table 8 - Inhibition of acid ceramidase with the acid ceramidase specific inhibitor CAY10466 (CAY) sensitizes human NSCLC cells to ChoK inhibition (4,3 fold induction for MN58b and 5 fold for RSM-932A) when compared with inhibition of alkaline ceramidase with the alkaline ceramidase specific inhibitor D-erythro-MAPP (DMP).**

| **Treatment of H460 cells** | | |
|---|---|---|
| Choline kinase inhibitor | Acid ceramidase inhibitor | **IC₅₀ (72h) µM** |
| - | CAY | 28 |
| - | DEM | 31 |
| MN58b | - | 0,28 |
| RSM-932A | - | 1,11 |
| MN58b | CAY | 0,065 |
| RSM-932A | CAY | 0,22 |
| MN58b | DEM | 0,32 |
| RSM-932A | DEM | 1,12 |

### EXAMPLE 5

### Generation of NSLC cells resistant to ChoK inhibitors

To further study the mechanism underlying resistance to ChoK inhibition, as well as the implication of acid ceramidase in this effect, we used the human NSCLC-derived H460 cell line for a set of in vitro experiments. Maintaining this cell line in culture for 9 months with slightly increasing doses cycles of MN58b and a second-generation ChoKα inhibitor, RSM-932A, we established new cell lines with acquired resistance to these ChoKα inhibitors. A control cell line (H460 stock) was also established maintaining H460 in culture for the same time without treatment. Thus, established cells resistant to MN58b (H460 MN58R) and resistant to RSM-932A (H460 RSM-932A-R) were not affected at concentrations where normal H460 cells or control H460stock cells were dramatically affected. Consequently, the necessary concentration to inhibit 50% of cell proliferation (IC50) in these resistant cells with MN58b and RSM-932A is significantly higher to that observed in control cells (Table 9). Furthermore, we found a strong cross-resistance between both ChoK inhibitors as it could be expected due to the similar mechanisms of action of these two antitumoral drugs (Table 9).

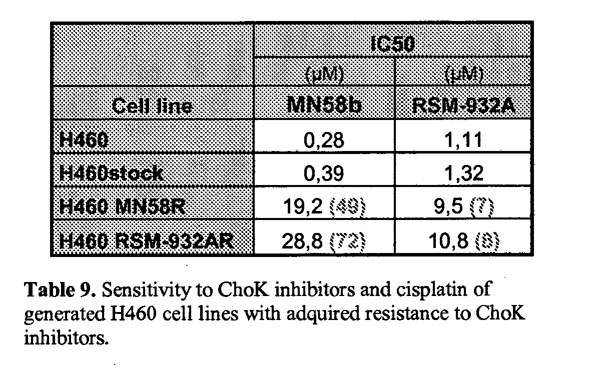

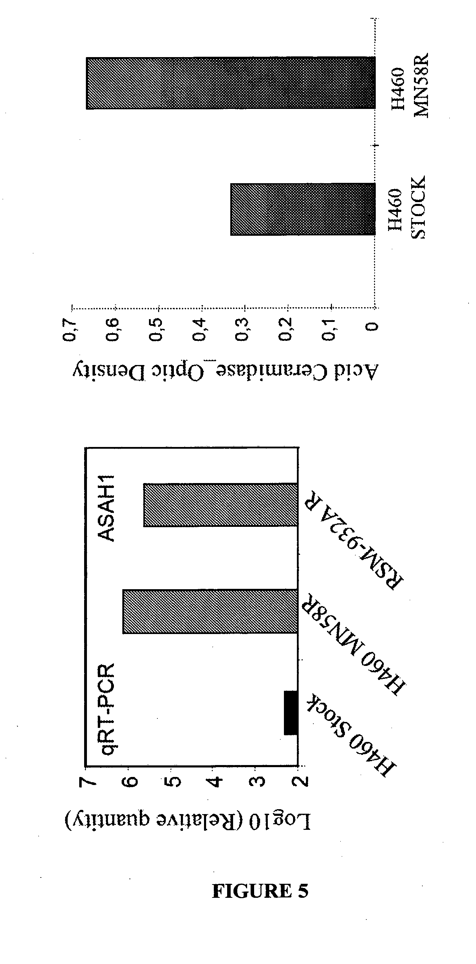

With the aim of testing if this endurance to die in response to ChoKα inhibitors is due to an increase in acid ceramidase levels, we performed both QT-PCR and Western blot analysis to measure the levels of this enzyme in these cells. In keeping with previous results, both gene expression and protein levels were over-expressed in resistant cells to ChoK inhibition with respect to control H460Stock cells (Figure 5).

### EXAMPLE 6

### Non-crossed resistance between ChoK inhibitors and cisplatin

As it has been mentioned before, the statistical analysis of correlation of responders to the different chemotherapeutic agents performed in primary cultures of tumours from patients with NSCLC revealed that resistance to MN58b was not associated to resistance to any other of the anti-oncogenic drugs tested (Table 4). In order to verify the data obtained from patient samples and to establish that the mechanism of resistance to ChoK inhibition was independent to resistance to other antitumoral drug conventionally used in NSCLC treatment, we analyzed the antiproliferative effect of cisplatin in the established cells resistant to ChoK inhibition. As shown in Table 10, H460 MN58R and H460 RSM-932AR were even more sensitive to the antiproliferative effect of cisplatin than parental H460 cells. These results suggest that the acquisition of resistance to ChoK inhibition goes through a specific mechanism likely related to acid ceramidase overexpression, and does not interfere with the mechanism of action of other antioncogenic drugs such as cisplatin.

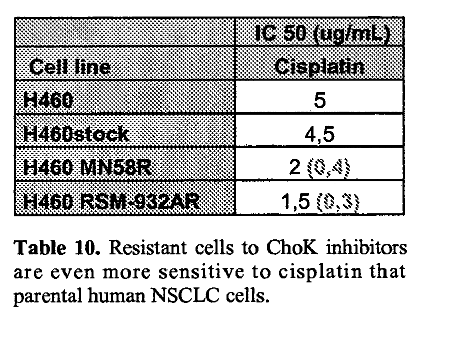

### EXAMPLE 7

### Effectiveness of a combined therapy of cisplatin and ChoK inhibitors in NSCLC

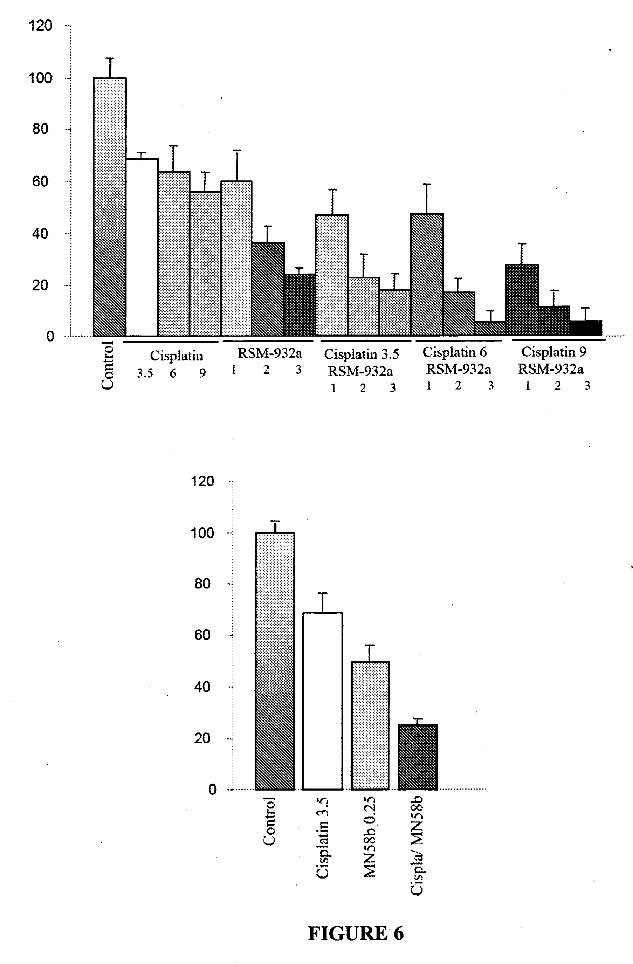

Results shown above suggest that ChoK inhibitors could be used as antitumoral agents for patients with NSCLC that do not respond to cisplatin, and vice versa. The potential effect of a combined therapy of ChoK inhibitors and the conventional platinum based therapy with cisplatin was then analysed. The effects on cell viability of cisplatin plus ChoK inhibitors MN58b and RSM-932A were evaluated in the NSCLC cell line H460 using MTT assays. Sequential treatments were performed in which cisplatin was administrated for 3h followed by 40h of ChoK inhibitors. The results were analyzed using the isobologram combination index method of Chou Talalay (Chou et al., *supra*.). As shown in Figure 6, a strong synergistic (CIs< 0.5) growth inhibition was observed between cisplatin and both MN58b or RSM-932A ChoK inhibitors in H460 cells (CI= 0.1 and CI= 0.4 respectively), indicating that the combination of these two therapies could result in a significant advantage in the control of NSCLC tumour growth.

With the aim of verifying that the combination of these two therapies could result in a significant advantage in the control of NSCLC tumour growth, in vivo experiments using NSCLC xenografts were performed. Following the findings of the in vitro experiments, sequential treatments were performed *in vivo* in which cisplatin was administrated for the first week (twice a week), followed by two weeks of treatment with ChoK inhibitors (three times a week). An additional group of concomitant treatment for the MN58b and cisplatin during the three weeks was included. As shown in Figure 7, a strong synergistic growth inhibition was observed between cisplatin and both MN58b (Figure 7A) or RSM-932A (Figure 7B) ChoK inhibitors in H460 xenografts without increasing toxicity (Figure 7C), indicating that the combination of these two therapies could result in a significant advantage in the management of NSCLC.

It is important to take into account that to obtain the same proportion of antitumoral activity with cisplatin alone that the ones obtained with the combined schedules, concentration of cisplatin has to be quadruplicated, and clinical signs of toxicity such as weigh lost are observed (Figure 7D). Thus, a sequential combined therapy with cisplatin and ChoK inhibitors results in a strong antitumoral effect while decreasing toxicity to imperceptible levels similar to those obtained when using a two-fold decrease in the concentration of the quimiotherapeutic agent.

### EXAMPLE 8 (Comparative example)

### Choline kinase inhibitors and TRAIL cooperates to induce cell death in colon cancer derived cell lines.

In order to investigate if the antitumor activities of RSM-932A and TRAIL follow similar or distinct mechanisms of action, the combination of a choline Kinase Inhibitor (ChoKI) and TRAIL was tested for a cooperatative effect in the cytotoxicity of tumour cells. To that end, five colon cancer derived cell lines were used: DLD-1, HT-29, HCT-116, SW620 and SW480.

For this purpose, cells were seeded in 96 multiwell plates at a density of 1x10⁴ cells/well 24 hours before treatment. Cells were treated with different concentrations of RSM-932A (ChoKI) or TRAIL for different times and the cell proliferation was quantified using the MTT [3-(4,5-Dimethylthiazol-2-yl)-2,5-Diphenyltetrazolium Bromide] colorimetric assay.

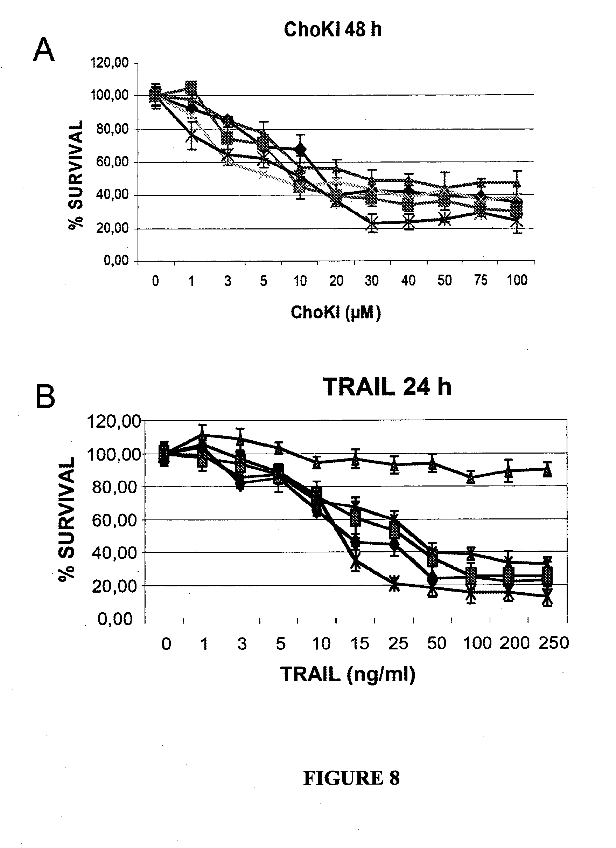

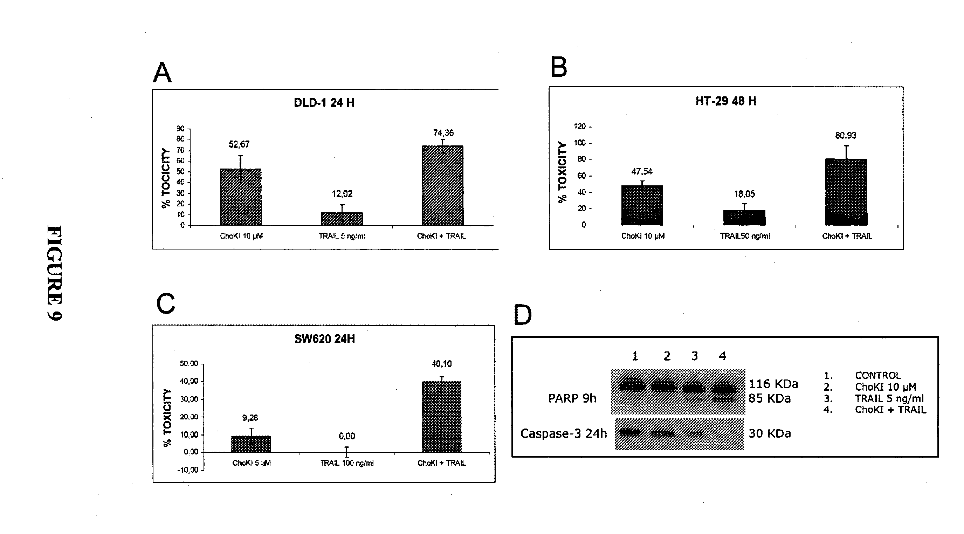

The result show that sensitivity to ChoKI was very similar in all cell lines analysed (Figure 8A). However, sensitivity to TRAIL was also very similar for all cell lines except for SW620, which is nearly resistant to TRAIL treatment (Figure 8B). Next, the same cells lines which had been pre-treated with ChoKI for 2 hours, sufficient to achieve an efficient inhibition of choline kinase, were then treated with TRAIL for an additional 24h. When DLD-1 cells were treated with ChoKI or TRAIL alone, cytotoxicity was 53% and 12% respectively, determined as percentage of cell death observed. When both drugs were combined, the toxicity increased to 75% cell death (Figure 9A). In HT-29 cells, ChoKI-and TRAIL-induced cytotoxicity was 48% and 18% respectively, whereas in combination the cytotoxicity increased to 81% (Figure 9B). In SW620 cells, which are resistant to TRAIL, ChoKI cytotoxicity was 9 %, whereas in combination cytotoxicity increased up to 41% (Figure 9C). This result was confirmed by Western blot analysis, as there is also an increase of PARP degradation or caspase 3 activation when TRAIL and choline kinase inhibitor are combined (Figure 9D).

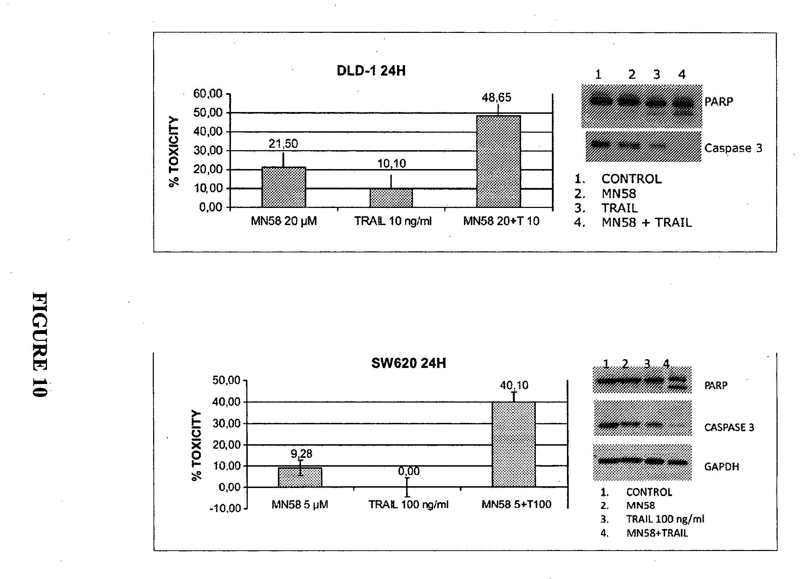

The results of the cooperation between RSM-932A, designed as ChoKI in this example, and TRAIL were analyzed using the isobologram combination index method of Chou Talalay (Chou et al., *supra*.). As shown in Figure 10, a strong synergistic (CIs< 0.5) growth inhibition was observed between these compounds (CI= 0.19 for DLD-1, CI=0.12 for HT-29 and CI= 0.085 for SW620), indicating that the combination of these two therapies could result in a significant advantage in the control of colon tumour growth. These results were verified using the ChoK inhibitor MN58b in DLD-1 and SW620 cells, obtaining similar results (CI=0.10 and CI=1.15 respectively) (Figure 10).

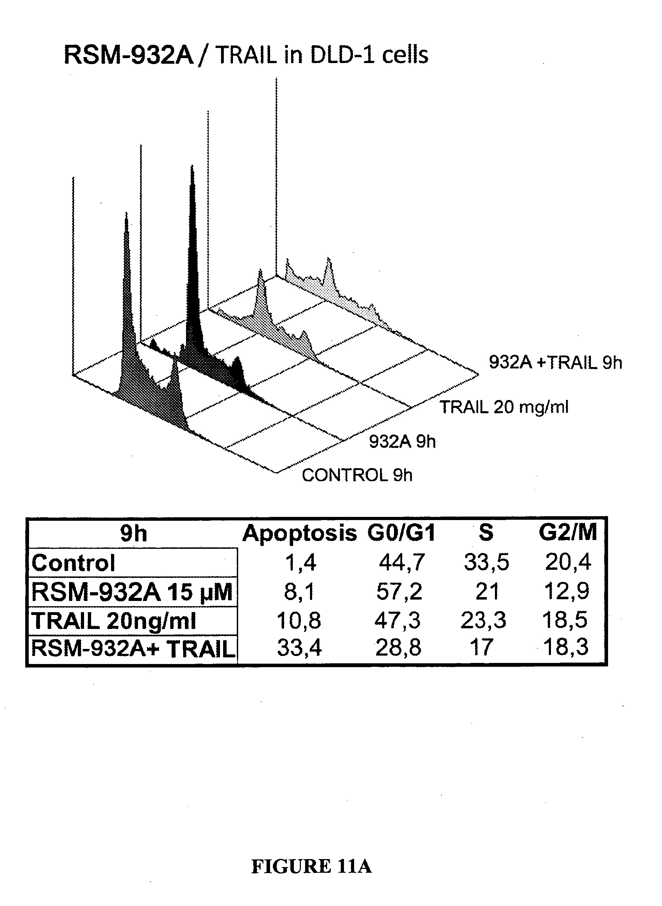

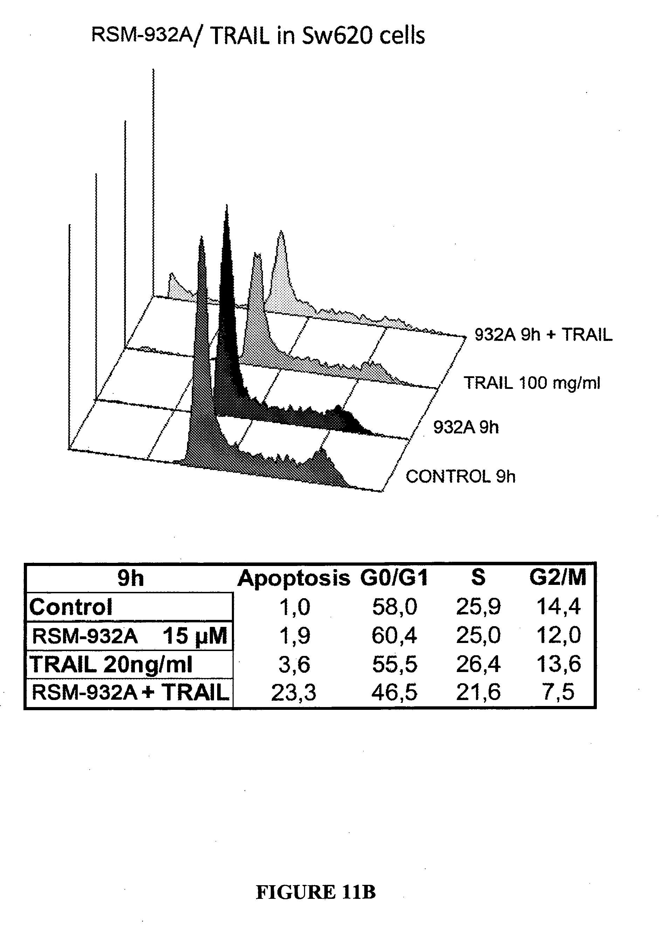

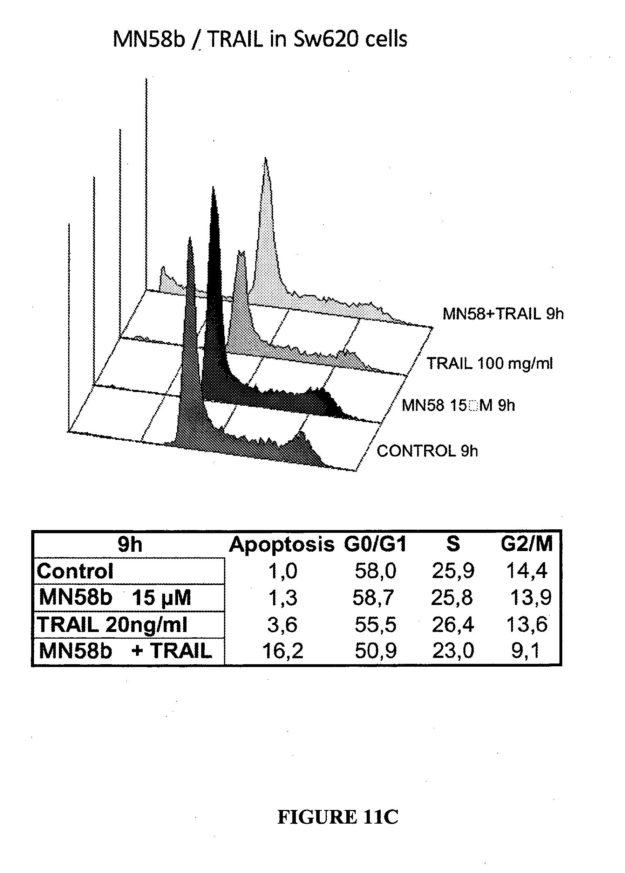

The synergism observed *in vitro* was also analysed by flow cytometry using Annexin V & IP staining to distinguish between apoptosis and necrosis or late apoptosis. After 7 hours treatment, with MN58b there is only a little increase in apoptosis in DLD-1 cells because MN58 needs more than 7 hours to induce tumour cell death. When DLD-1 cells are treated with TRAIL alone there is a 35% of apoptosis. In combination tumour cell death is amplified up to 50%. When DLD-1 are treated with RSM-932A in combination with TRAIL there is a 55% increase in tumour cell death but mainly the necrotic or late apoptosis population because RSM-932A has an effect that is faster than MN58. In SW620, which are resistant to TRAIL, apoptotic population is also increased up to 35% in combination with MN58. When TRAIL is combined with RSM-932A, necrotic or late apoptotic population is increased and there is a total cell death of 59% after 7 hours treatment (Figure 11), verifying the synergistic effect of the combination of TRAIL and ChoK inhibitors in colon cancer cells.

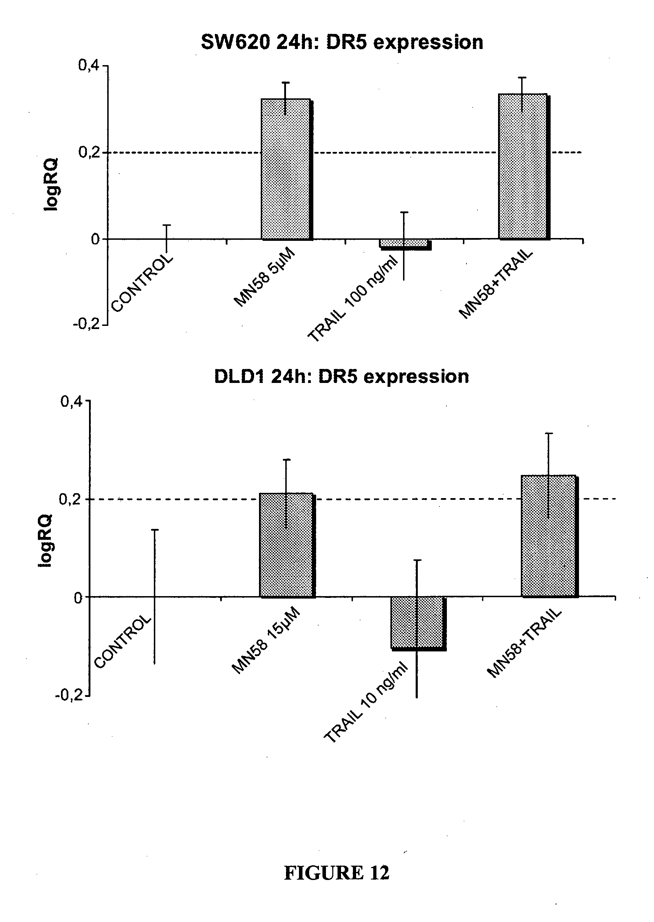

TRAIL is a member of the tumour necrosis factor (TNF) superfamily and can induce apoptosis by the extrinsic pathway associated via stimulation of death receptors DR4 (TNFRSF10A or TRAIL-R1) and DR5 (TNFRSF10B or TRAIL-R2). In order to determine the mechanism of the effectivity of the combined therapy of TRAIL and ChoK inhibitors, DR5 and DR4 levels were analysed by quantitative PCR in DLD-1 and SW620 cell lines after treatments with MN58b, TRAIL or their simultaneous combination. Significant changes were not found for DR4 levels, but MN58b treatment increased DR5 levels resulting in a 2 fold increase in SW620 cell line and in a 1.6 increase in DLD-1 cell line (Figure 12), which could explain the mechanism of action of the synergistic effect observed between ChoK inhibitors and TRAIL.

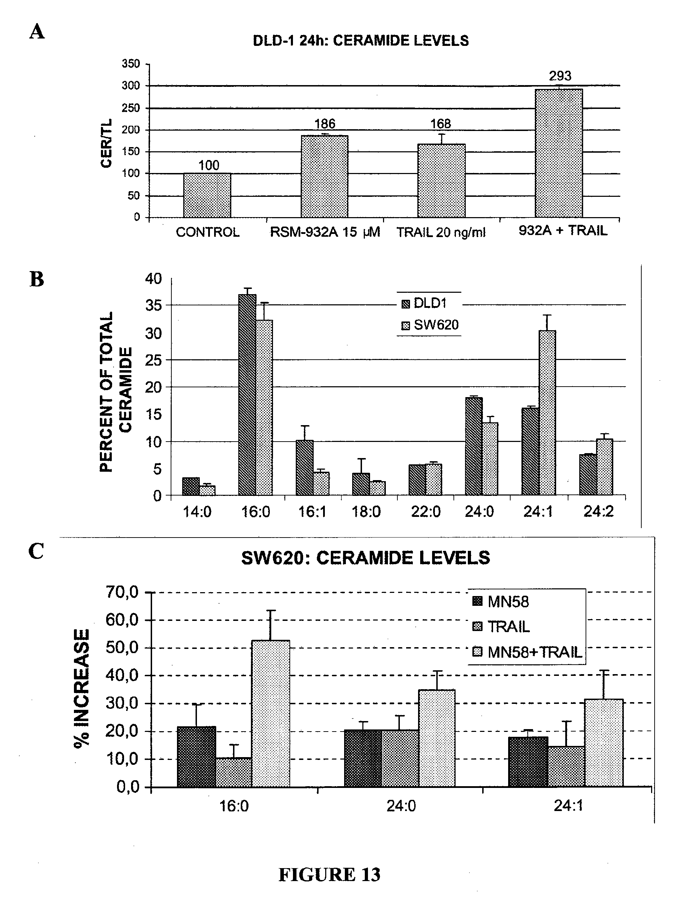

Both MN58b and TRAIL have been previously described to increase intracellular ceramide levels. To study ceramide generation in this system, intracellular lipids were labelled with [¹⁴C]-Serine for 48 hour before treating DLD-1 cells with MN58 or TRAIL. After extraction, lipids were analysed by Thin Layer Chromatography and quantified. As it can be observed in Figure 13, when MN58b and TRAIL are simultaneously combined there is a 3 fold increase in ceramides levels. Furthermore, it has been previously described that SW620 resistance to TRAIL is associated with defects in ceramide signalling and can be overcome by exogenous ceramide. Accordingly, C16-ceramide does not increase in SW620 after treatment. In order to investigate whether ChoK inhibition may contribute to generate C16-ceramide, ceramide species have been analysed by Liquid Chromatography and Mass Spectrometry, obtaining a 50% increase in C16-ceramide when MN58b and TRAIL are combined (Figure 13).

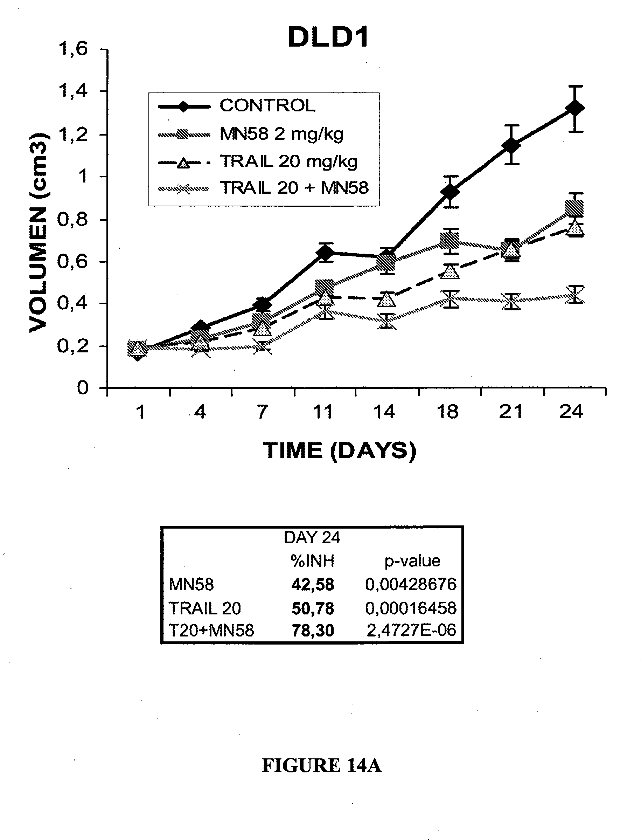

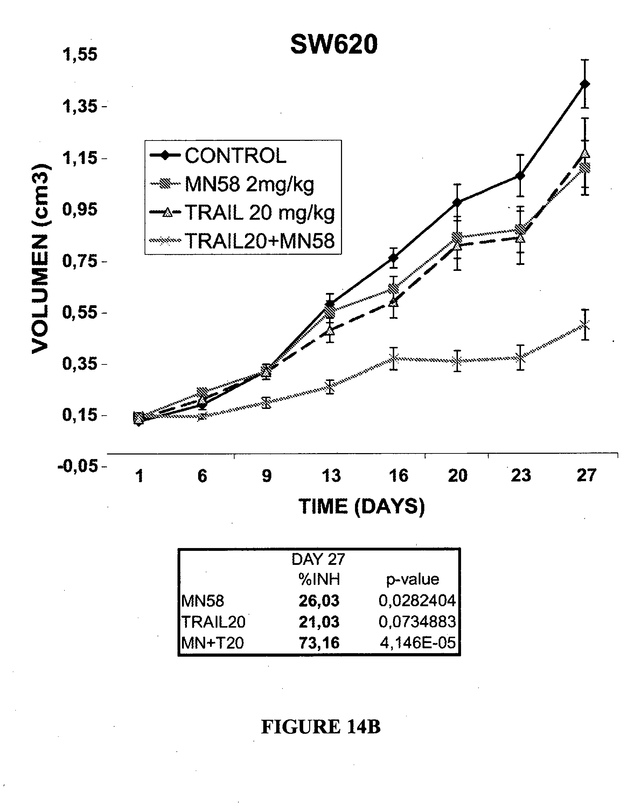

Finally, *in vivo* combinatorial treatments were also performed using a xenograft model in nude mice. Simultaneous combinatorial treatments started when tumours acquired a volume of 0.15 cm³. 10 mice per group were included. Mice were treated Monday, Wednesday and Friday with MN58b (2 mg/kg), and Thuesday and Thursday with 20 mg/kg of TRAIL. As it can be observed in Figure 14, after three weeks treatment there is a 68% and 75% of tumour growth inhibition in DLD-1 and SW620 xenografts were obtained respectively, confirming the strong synergism observed in the *in vitro* experiments.

### EXAMPLE 9

### Choline kinase inhibitors and 5-FU cooperates to induce cell death in colon cancer derived cell lines.

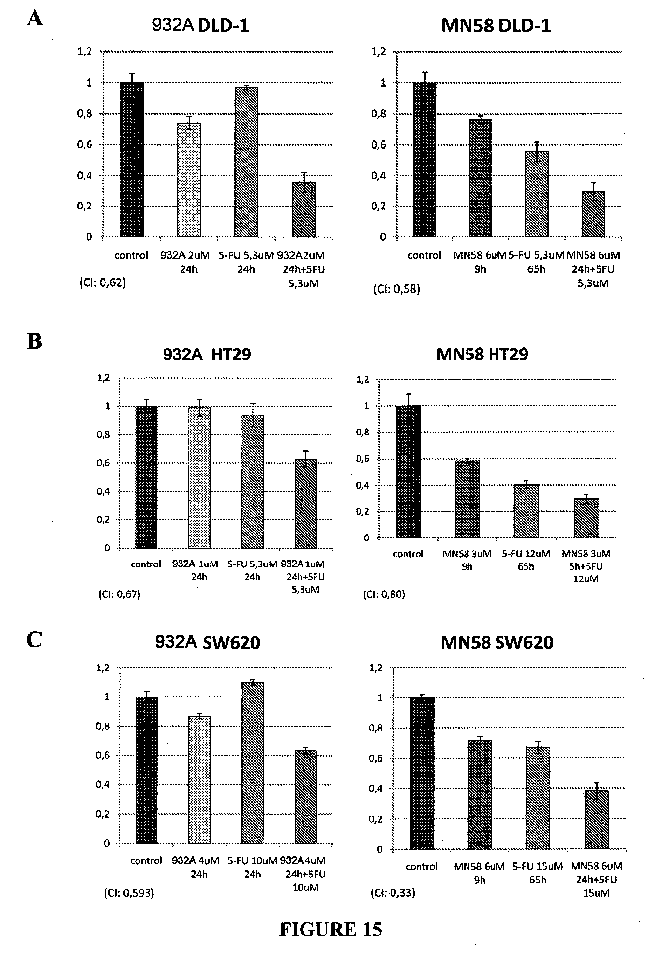

In addition, the potential effect of a combined therapy of ChoK inhibitors and the conventional 5-fluoracile (5-FU) based therapy in colon cancer has been also analysed. The effects on cell viability of 5-FU plus ChoK inhibitors MN58b and RSM-932A were evaluated in DLD-1, SW620 and HT-29 cells using MTT assays. The best options of combinations were identified as concomitant treatments or sequential treatments in which ChoK inhibitors were administrated for short times (9-24h), followed of longer times of 5-FU (48-72h). The results were analyzed using the isobologram combination index method of Chou Talalay (Chou et al., *supra*.). As shown in Figure 15, a synergistic effect on growth inhibition was observed between 5-FU and both MN58b or RSM-932A ChoK inhibitors in all human colon cancer cells analyzed, indicating that the combination of these two therapies could result in a significant advantage in the control of colon tumour growth.

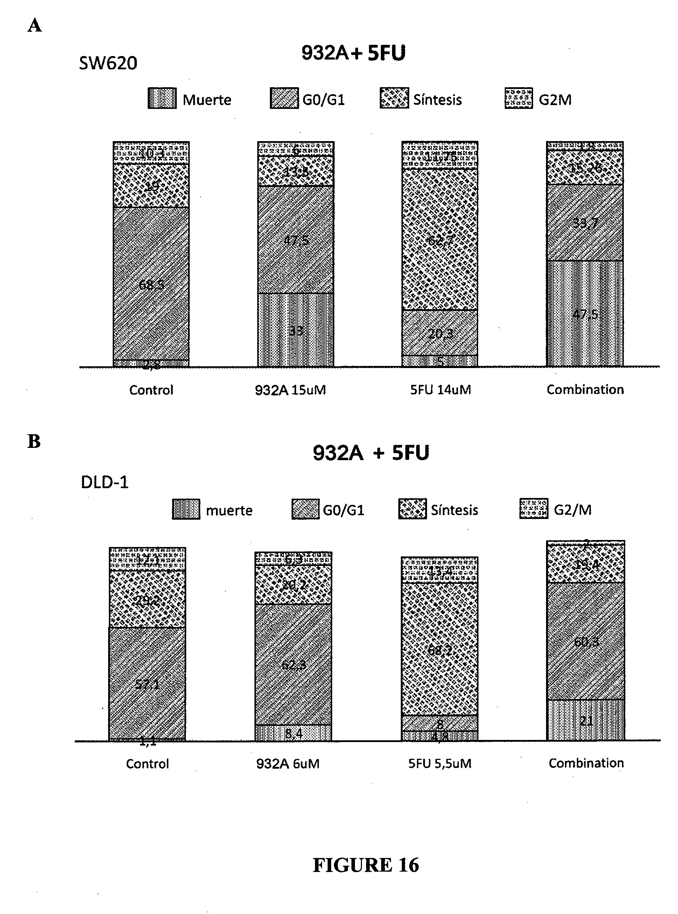

These results were also verified by flow citometry analysis. As it can be observed in Figure 16, cell cycle analysis not only revealed that the combined therapy resulted in a significant increase in cell death even using low concentrations of these agents, but also revealed that whereas ChoK inhibitors display an effect in G0/G1 phase of the cell cycle, 5-FU exerts its effect affecting G2/M phase of the cell cycle, accordingly with previous reports. These results give the evidence of the requirement of the pre-treatment with ChoK inhibitors when following a combined therapy in order to sensitize cells to 5-FU antiproliferative effect.

With the aim of further investigating the mechanism of ChoKIs sensitization to the antitumoral effect of 5-FU, a gene expression analysis of the 5-FU-metabolisation pathway was performed in H460 cells treated at different times with MN58b and RSM-932A using affymetrix technology. As it can be observed in Table 11, several genes of this pathway resulted significantly and commonly affected by ChoK inhibitors, suggesting that pre-treatment with ChoK inhibitors modify the expression of enzymes that potentiate the effect of 5-FU.

**Table 11. Gene expression analysis of 5-FU metabolization pathway in H460 cells treated with ChoK inhibitors determined by affymetrix gene expression assay.**

| **Official symbol** | | **Gene** | **RSM-932A** | | **MN58b** | |
|---|---|---|---|---|---|---|
| | | | **9h** | **24h** | **9h** | **24h** |
| **UPP1** | **UP** | **Uridine phosphorylase 1** | **+1.83** | **+2.48** | **+1.54** | **+1.76** |
| **RRM2** | **RRM2** | **Ribonucleotide Reductase** | **-1.39** | **-3.69** | **-1.48** | **-2.50** |
| **TYMS** | **TS** | **Thymidylate synthetase** | **-1.03** | **-2.73** | **-1.30** | **-2.39** |
| **DUT** | **DUT** | **Deoxyuridine triphosphatase** | **-1.12** | **-2.08** | **-1.17** | **-2.03** |

Since H460 are NSCLC-derived cells, these results were validated using QT-PCR technology in three different colon-derived cancer cells in order to determine whether this mechanism of 5-FU-induced cell death potentiation could be cell-independent and the modification of the expression levels of genes of this pathway could explain the effects of ChoKIs sensitization found in the colon cancer system. As it can be observed in Table 12, this pathway is significantly altered in a similar extent in all cancer cells analyzed, suggesting that the alteration on the metabolization of 5-FU could be a mechanism of action of this combined therapy.

**Table 12. PT-PCR validation of the alteration of the expression of genes of the 5-FU metabolization pathway (DUT:deoxyuridine triphosphate; RRM:Ribonucleotide reductase; TYM: thymidilate synthetase; UPP:uridine phosphorylase 1) after treatment with ChoK inhibitors RSM-932A (932A) and MN58b (MN58b) in DLD-1, HT29 and SW62 colon cancer cells. DR (Down-regulation); UR (Up-regulation)**

| **DLD-1** | DUT | RRM | TYM | UPP |
|---|---|---|---|---|
| 932A | DR | DR | DR | UR |
| MN58b | | | DR | UR |
| | | | | |

| **HT29** | DUT | RRM | TYM | UPP |
|---|---|---|---|---|
| 932A | DR | DR | DR | UR |
| MN58b | DR | DR | DR | |
| | | | | |

| **SW62** | DUT | RRM | TYM | UPP |
|---|---|---|---|---|
| 932A | DR | DR | DR | UR |
| MN58b | | DR | DR | UR |

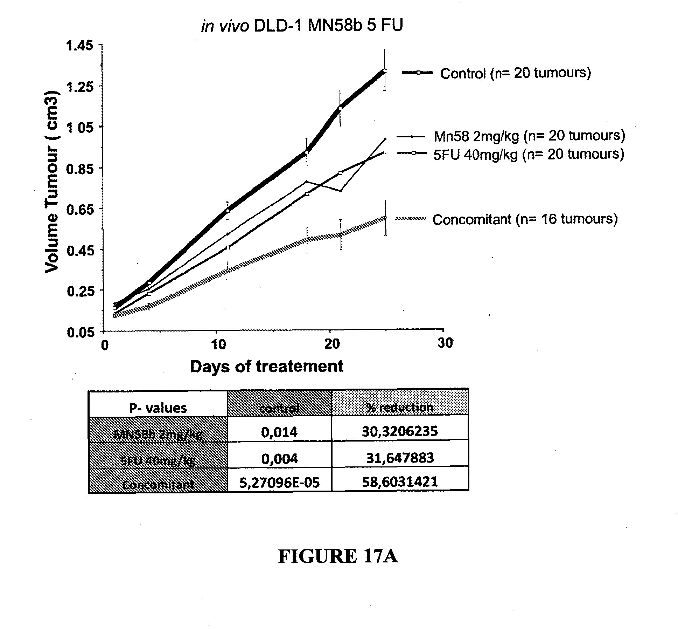

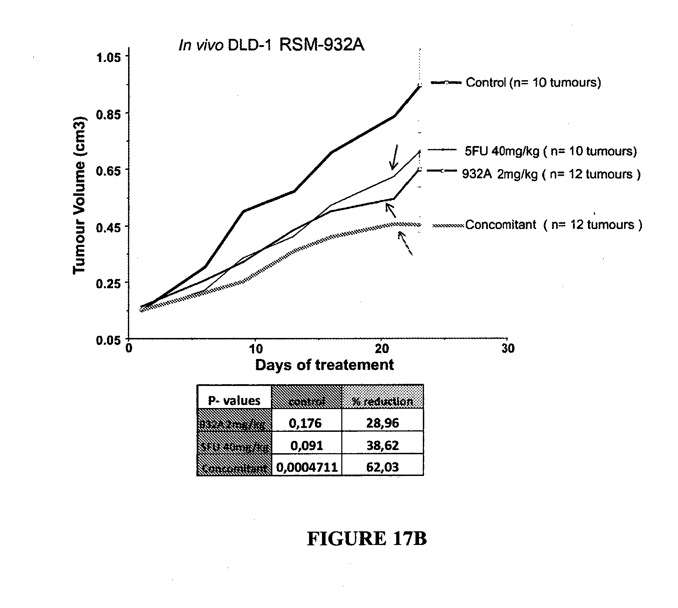

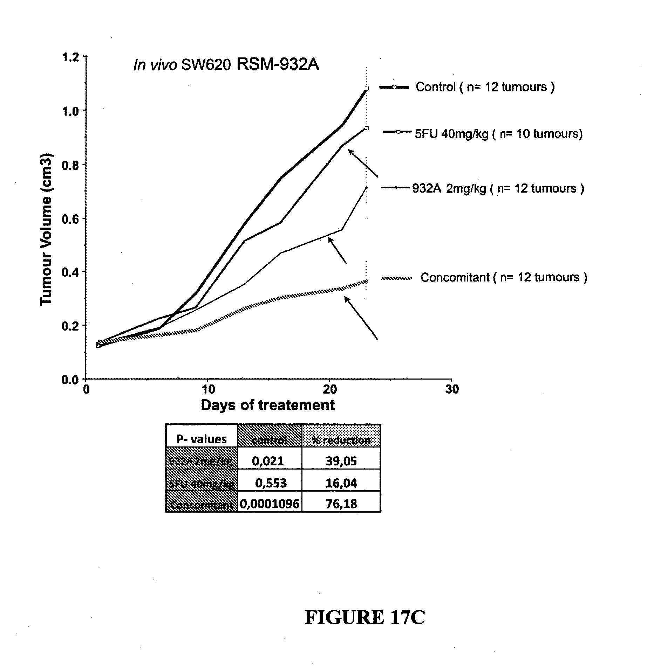

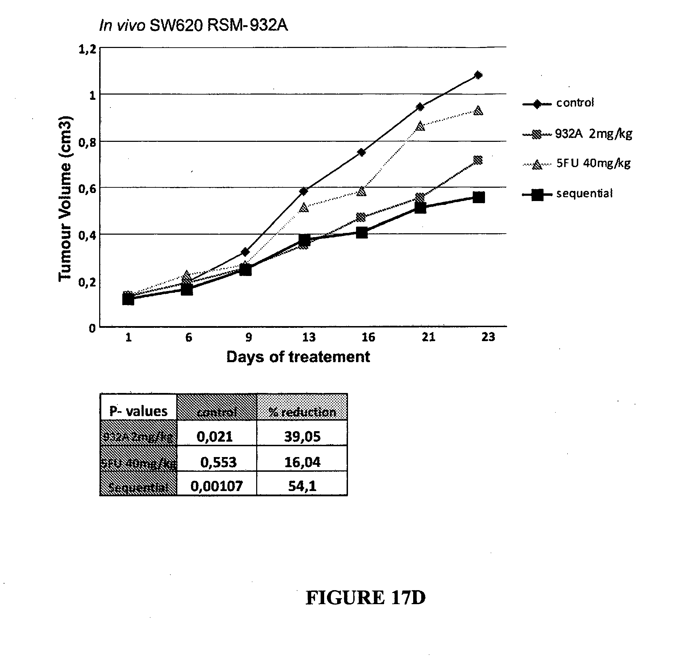

Finally, with the aim of verifying that the combination of these two therapies could result in a significant advantage in the control of NSCLC tumour growth, *in vivo* experiments using two different colon xenografts were performed. Following the findings of the *in vitro* experiments, concomitant treatments, or sequential treatments in which ChoK inhibitors MN58b or RSM-932A were administrated for the first week (three times a week), followed by two weeks of treatment with 5-FU (twice a week) were performed *in vivo.* As it is shown in Figure 17, a strong synergistic growth inhibition was observed between 5-FU and ChoK inhibitor using both DLD-1 and SW620 xenografts, suggesting that the combination of these two therapies could result in a significant advantage for the control growth of colon tumors.

## Claims

1. A composition comprising, separately or together, a first component comprising one or more choline kinase inhibitors specific for the choline kinase alpha isoform and a second component selected from the group of one or more acid ceramidase inhibitors and one or more chemotherapy agents, wherein the chemotherapy agent is selected from the group consisting of a DNA-alkylating agent, an antimetabolite, a mitotic inhibitor, an anthracycline, a topoisomerase I inhibitor, a topoisomerase II inhibitor, cetuximab, gefitinib and imatinib.

2. A composition according to claim 1 wherein the choline kinase inhibitor specific for the choline kinase alpha isoform is a compound as defined in Table 1.

3. A composition according to claims 1 or 2, wherein the acid ceramidase inhibitor is a compound as defined in Table 2.

4. A composition according to claims 1 or 2, wherein the alkylating agent is a platinum-based compound.

5. A composition according to claim 4, wherein the platinum-based compound is cisplatin.

6. A composition according to claims 1 or 2, wherein the antimetabolite is 5-fluorouracile, gemcitabine or pemetrexed.

7. A composition according to claims 1 or 2, wherein the mitotic inhibitor is docetaxel.

8. A composition according to claims 1 or 2, wherein the anthracycline is doxorubicin.

9. A pharmaceutical composition comprising a composition according to any of claims 1 to 8 with a pharmaceutically acceptable carrier or excipient.

10. A composition according to any of claims 1 to 8 for use in medicine.

11. A composition according to any of claims 1 to 8 for use in the treatment of cancer.

12. A composition for use according to claim 11 wherein the cancer is non-small cell lung cancer or colon cancer.

## Patentansprüche

1. Zusammensetzung, die getrennt oder zusammen eine erste Komponente, die einen oder mehrere Cholinkinase-Inhibitoren, die spezifisch sind für die Cholinkinase-Alpha-Isoform, und eine zweite Komponente umfasst, die ausgewählt ist aus der Gruppe aus einem oder mehreren Inhibitoren der sauren Ceramidase und einem oder mehreren chemotherapeutischen Wirkstoffen, wobei der chemotherapeutische Wirkstoff ausgewählt ist aus der Gruppe bestehend aus einem DNA-alkylierenden Wirkstoff, einem Antimetaboliten, einem Mitoseinhibitor, einem Anthracyclin, einem Topoisomerase-I-Inhibitor, einem Topoisomerase-II-Inhibitor, Cetuximab, Gefitinib und Imatinib.

2. Zusammensetzung nach Anspruch 1, wobei der Cholinkinase-Inhibitor, der spezifisch für die Cholinkinase-Alpha-Isoform ist, eine wie in Tabelle 1 definierte Verbindung ist.

3. Zusammensetzung nach Anspruch 1 oder 2, wobei der Inhibitor der sauren Ceramidase eine wie in Tabelle 2 definierte Verbindung ist.

4. Zusammensetzung nach Anspruch 1 oder 2, wobei der alkylierende Wirkstoff eine Platin-basierte Verbindung ist.

5. Zusammensetzung nach Anspruch 4, wobei die Platin-basierte Verbindung Cisplatin ist.

6. Zusammensetzung nach Anspruch 1 oder 2, wobei der Antimetabolit 5-Fluoruracil, Gemcitabin oder Pemetrexed ist.

7. Zusammensetzung nach Anspruch 1 oder 2, wobei der Mitoseinhibitor Docetaxel ist.

8. Zusammensetzung nach Anspruch 1 oder 2, wobei das Anthracyclin Doxorubicin ist.

9. Arzneimittel, das eine Zusammensetzung nach einem der Ansprüche 1 bis 8 mit einem pharmazeutisch verträglichen Trägerstoff oder Excipienten umfasst.

10. Zusammensetzung nach einem der Ansprüche 1 bis 8 zur Verwendung in der Medizin.

11. Zusammensetzung nach einem der Ansprüche 1 bis 8 zur Verwendung in der Behandlung von Krebs.

12. Zusammensetzung zur Verwendung nach Anspruch 11, wobei der Krebs nicht-kleinzelliger Lungenkrebs oder Dickdarmkrebs ist.

## Revendications

1. Composition comprenant, séparément ou conjointement, un premier composant comprenant un ou plusieurs inhibiteurs de la choline kinase spécifiques pour l'isoforme alpha de la choline kinase et un deuxième composant sélectionné dans le groupe d'un ou plusieurs inhibiteurs de la céramidase acide et un ou plusieurs agents chimiothérapeutiques, dans laquelle l'agent chimiothérapeutique est sélectionné dans le groupe constitué par un agent alkylant l'ADN, un antimétabolite, un inhibiteur mitotique, une anthracycline, un inhibiteur de la topoisomérase I, un inhibiteur de la topoisomérase II, le cétuximab, le géfitinib et l'imatinib.

2. Composition selon la revendication 1, dans laquelle l'inhibiteur de la choline kinase spécifique pour l'isoforme alpha de la choline kinase est un composé tel qu'il est défini dans le tableau 1.

3. Composition selon les revendications 1 ou 2, dans laquelle l'inhibiteur de la céramidase acide est un composé tel qu'il est défini dans le tableau 2.

4. Composition selon les revendications 1 ou 2, dans laquelle l'agent alkylant est un composé à base de platine.

5. Composition selon la revendication 4, dans laquelle le composé à base de platine est le cisplatine.

6. Composition selon les revendications 1 ou 2, dans laquelle l'antimétabolite est le 5-fluorouracile, la gemcitabine ou le pémétrexed.

7. Composition selon les revendications 1 ou 2, dans laquelle l'inhibiteur mitotique est le docétaxel.

8. Composition selon les revendications 1 ou 2, dans laquelle l'anthracycline est la doxorubicine.

9. Composition pharmaceutique comprenant une composition selon l'une quelconque des revendications 1 à 8 avec un véhicule ou excipient pharmaceutiquement acceptable.

10. Composition selon l'une quelconque des revendications 1 à 8 destinée à être utilisée dans un médicament.

11. Composition selon l'une quelconque des revendications 1 à 8 destinée à être utilisée dans le traitement du cancer.

12. Composition destinée à être utilisée selon la revendication 11, dans laquelle le cancer est le cancer bronchique non à petites cellules ou le cancer du côlon.


### SEQUENCE LISTING

<110> TRANSLATIONAL CANCER DRUGS PHARMA, S.L.
<120> METHODS AND COMPOSITIONS FOR THE TREATMENT OF CANCER
<130> P4096PC00
<150> EP08380270.2
   <151> 2008-09-17
<160> 7
<170> PatentIn version 3.5
<210> 1
   <211> 1188
   <212> DNA
   <213> Human
<400> 1
   atggcggccg aggcgacagc tgtggccgga agcggggctg ttggcggctg cctggccaaa      60
   gacggcttgc agcagtctaa gtgcccggac actaccccaa aacggcggcg cgcctcgtcg     120
   ctgtcgcgtg acgccgagcg ccgagcctac caatggtgcc gggagtactt gggcggggcc     180
   tggcgccgag tgcagcccga ggagctgagg gtttaccccg tgagcggagg cctcagcaac     240
   ctgctcttcc gctgctcgct cccggaccac ctgcccagcg ttggcgagga gccccgggag     300
   gtgcttctgc ggctgtacgg agccatcttg cagggcgtgg actccctggt gctagaaagc     360
   gtgatgttcg ccatacttgc ggagcggtcg ctggggcccc agctgtacgg agtcttccca     420
   gagggccggc tggaacagta catcccaagt cggccattga aaactcaaga gcttcgagag     480
   ccagtgttgt cagcagccat tgccacgaag atggcgcaat ttcatggcat ggagatgcct     540
   ttcaccaagg agccccactg gctgtttggg accatggagc ggtacctaaa acagatccag     600
   gacctgcccc caactggcct ccctgagatg aacctgctgg agatgtacag cctgaaggat     660
   gagatgggca acctcaggaa gttactagag tctaccccat cgccagtcgt cttctgccac     720
   aatgacatcc aggaagggaa catcttgctg ctctcagagc cagaaaatgc tgacagcctc     780
   atgctggtgg acttcgagta cagcagttat aactataggg gctttgacat tgggaaccat     840
   ttttgtgagt gggtttatga ttatactcac gaggaatggc ctttctacaa agcaaggccc     900
   acagactacc ccactcaaga acagcagttg cattttattc gtcattacct ggcagaggca     960
   aagaaaggtg agaccctctc ccaagaggag cagagaaaac tggaagaaga tttgctggta    1020
   gaagtcagtc ggtatgctct ggcatcccat ttcttctggg gtctgtggtc catcctccag    1080
   gcatccatgt ccaccataga atttggttac ttggactatg cccagtctcg gttccagttc    1140
   tacttccagc agaaggggca gctgaccagt gtccactcct catcctag                 1188
<210> 2
   <211> 395
   <212> PRT
   <213> Human
<400> 2
   Met Ala Ala Glu Ala Thr Ala Val Ala Gly Ser Gly Ala Val Gly Gly
   1               5                   10                  15
   Cys Leu Ala Lys Asp Gly Leu Gln Gln Ser Lys Cys Pro Asp Thr Thr
               20                  25                  30
   Pro Lys Arg Arg Arg Ala Ser Ser Leu Ser Arg Asp Ala Glu Arg Arg
           35                  40                  45
   Ala Tyr Gln Trp Cys Arg Glu Tyr Leu Gly Gly Ala Trp Arg Arg Val
       50                  55                  60
   Gln Pro Glu Glu Leu Arg Val Tyr Pro Val Ser Gly Gly Leu Ser Asn
   65                  70                  75                  80
   Leu Leu Phe Arg Cys Ser Leu Pro Asp His Leu Pro Ser Val Gly Glu
                   85                  90                  95
   Glu Pro Arg Glu Val Leu Leu Arg Leu Tyr Gly Ala Ile Leu Gln Gly
               100                 105                 110
   Val Asp Ser Leu Val Leu Glu Ser Val Met Phe Ala Ile Leu Ala Glu
           115                 120                 125
   Arg Ser Leu Gly Pro Gln Leu Tyr Gly Val Phe Pro Glu Gly Arg Leu
       130                 135                 140
   Glu Gln Tyr Ile Pro Ser Arg Pro Leu Lys Thr Gln Glu Leu Arg Glu
   145                 150                 155                 160
   Pro Val Leu Ser Ala Ala Ile Ala Thr Lys Met Ala Gln Phe His Gly
                   165                 170                 175
   Met Glu Met Pro Phe Thr Lys Glu Pro His Trp Leu Phe Gly Thr Met
               180                 185                 190
   Glu Arg Tyr Leu Lys Gln Ile Gln Asp Leu Pro Pro Thr Gly Leu Pro
           195                 200                 205
   Glu Met Asn Leu Leu Glu Met Tyr Ser Leu Lys Asp Glu Met Gly Asn
       210                 215                 220
   Leu Arg Lys Leu Leu Glu Ser Thr Pro Ser Pro Val Val Phe Cys His
   225                 230                 235                 240
   Asn Asp Ile Gln Glu Gly Asn Ile Leu Leu Leu Ser Glu Pro Glu Asn
                   245                 250                 255
   Ala Asp Ser Leu Met Leu Val Asp Phe Glu Tyr Ser Ser Tyr Asn Tyr
               260                 265                 270
   Arg Gly Phe Asp Ile Gly Asn His Phe Cys Glu Trp Val Tyr Asp Tyr
           275                 280                 285
   Thr His Glu Glu Trp Pro Phe Tyr Lys Ala Arg Pro Thr Asp Tyr Pro
       290                 295                 300
   Thr Gln Glu Gln Gln Leu His Phe Ile Arg His Tyr Leu Ala Glu Ala
   305                 310                 315                 320
   Lys Lys Gly Glu Thr Leu Ser Gln Glu Glu Gln Arg Lys Leu Glu Glu
                   325                 330                 335
   Asp Leu Leu Val Glu Val Ser Arg Tyr Ala Leu Ala Ser His Phe Phe
               340                 345                 350
   Trp Gly Leu Trp Ser Ile Leu Gln Ala Ser Met Ser Thr Ile Glu Phe
           355                 360                 365
   Gly Tyr Leu Asp Tyr Ala Gln Ser Arg Phe Gln Phe Tyr Phe Gln Gln
       370                 375                 380
   Lys Gly Gln Leu Thr Ser Val His Ser Ser Ser
   385                 390                 395
<210> 3
   <211> 21
   <212> DNA
   <213> Artificial
<220>
   <223> Sequence of a shRNA specific for ChoK alpha
<400> 3
   aactgatgct cattgatttc g 21
<210> 4
   <211> 19
   <212> RNA
   <213> Artificial
<220>
   <223> SiRNA specific for choline kinase
<400> 4
   caugcuguuc cagugcucc 19
<210> 5
   <211> 21
   <212> DNA
   <213> Artificial
<220>
   <223> siRNA specific for acid ceramidase as described in Morales et al (Oncogene, 2007, 26:905-916)
<400> 5
   aaaatcaacc tatcctcctt c 21
<210> 6
   <211> 21
   <212> DNA
   <213> Artificial
<220>
   <223> siRNA specific for acid ceramidase as described in WO2007136635
<400> 6
   aatcaaccta tcctccttca g 21
<210> 7
   <211> 281
   <212> PRT
   <213> Human
<400> 7